# EUROPEAN PATENT SPECIFICATION

(11) **EP 4 148 049 B1**
(45) Date of publication and mention of the grant of the patent: **12.11.2025**
(21) Application number: 21803467.6
(22) Date of filing: 12.05.2021
(51) Int. Cl.: C09K 11/06, C07B 59/00, C07D 307/91, C07D 333/76, C07D 409/10, C07F 5/02, C07F 7/08, C07F 9/6571, H10K 85/40, H10K 85/60, H10K 50/11

(54) **ORGANIC LIGHT-EMITTING DEVICE COMPRISING ORGANIC LIGHT-EMITTING COMPOUND**
ORGANISCHE LICHTEMITTIERENDE VORRICHTUNG ENTHALTEND ORGANISCHE LICHTEMITTIERENDE VERBINDUNG
DISPOSITIF ÉLECTROLUMINESCENT ORGANIQUE COMPRENANT COMPOSÉ ÉLECTROLUMINESCENT ORGANIQUE

(30) Priority: 12.05.2020 KR 20200056347
(43) Date of publication of application: 15.03.2023
(73) Proprietor: SFC Co., Ltd., Cheongju-si, Chungcheongbuk-do 28122 (KR)
(72) Inventor: KIM, Ji-yung, Cheongju-si Chungcheongbuk-do 28122 (KR); LEE, Se-jin, Cheongju-si Chungcheongbuk-do 28122 (KR); CHOI, Yeong-tae, Cheongju-si Chungcheongbuk-do 28122 (KR); KIM, Kyung-tae, Cheongju-si Chungcheongbuk-do 28122 (KR); KIM, Myeong-jun, Cheongju-si Chungcheongbuk-do 28122 (KR); KIM, Kyeong-hyeon, Cheongju-si Chungcheongbuk-do 28122 (KR)
(74) Representative: Grosse, Felix Christopher
(86) International application number: PCT/KR2021/005944
(87) International publication number: WO 2021/230653

(56) References cited:
- CN-A- 110 330 472
- KR-A- 20170 130 434
- KR-A- 20190 132 282
- KR-A- 20190 132 282
- KR-A- 20200 004 266
- KR-B1- 102 094 830
- KR-B1- 102 094 830

## Description

The present invention relates to an organic light-emitting compound and an organic light-emitting device including the same. More specifically, the present invention relates to an organic light-emitting device that is capable of exhibiting excellent luminous characteristics such as significantly improved low voltage driving by using both an anthracene derivative having a characteristic structure as a host compound and a polycyclic aromatic derivative compound as a dopant compound in a light-emitting layer.

An organic light-emitting device is a self-luminous device that emits light when energy is released from excitons which are formed by recombination of electrons injected from an electron injection electrode (cathode) and holes injected from a hole injection electrode (anode) in a light-emitting layer. Such an organic light-emitting device attracts a great deal of attention as a next-generation light source due to applicability to full-color flat panel light-emitting displays based on advantages such as low driving voltage, high luminance, wide viewing angle, and rapid response speed thereof.

In order for the organic light-emitting device to exhibit the characteristics, the structure of the organic layer in the organic light-emitting device should be optimized, and the material constituting each organic layer, namely, a hole injection material, a hole transport material, a light-emitting material, an electron transport material, an electron injection material, or an electron blocking material should be based on stable and efficient ingredients. However, there is a continuing need to develop organic layer structures and respective materials thereof for stable and efficient organic light-emitting devices.

In particular, in order to realize the maximum efficiency of the light-emitting layer, the energy bandgap of the host and the dopant must be properly combined so that each of holes and electrons move to the dopant through a stable electrochemical path to form excitons.

Therefore, it is an object of the present invention to provide an organic light-emitting device that is capable of exhibiting excellent luminous characteristics such as improved low voltage driving by using a characteristic host material and a dopant material in a light-emitting layer therein.

Light-emitting devices similar to the invention are disclosed in, for example, KR102094830 and KR20190132282.

In accordance with the present invention, the above and other objects can be accomplished by the provision of an organic light-emitting device including a first electrode, a second electrode facing the first electrode, and a light-emitting layer interposed between the first electrode and the second electrode. The light-emitting layer includes a compound represented by the [Formula I] and a compound represented by the [Formula B-1] as defined in the appended claims.

The organic light-emitting device according to the present invention is capable of realizing improved low voltage driving using an anthracene derivative having a characteristic structure as a host compound and a polycyclic aromatic derivative compound as a dopant compound in a light-emitting layer, thus being useful for various display devices such as flat panel, flexible, and wearable displays as well as lighting devices.

Hereinafter, the present invention will be described in detail with reference to the annexed drawings.

A compound that is comprised by the organic light-emitting device of the present invention is represented by the following [Formula I], has a structure including at least one dibenzofuran or dibenzothiophene and, based on this structure, is used as a host compound in a light-emitting layer of the organic light-emitting device to impart improved low-voltage driving characteristics to the organic light-emitting device. 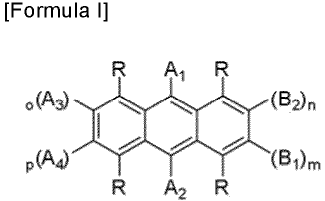 wherein
A₁ to A₄, B₁ and B₂ are identical to or different from each other and are each independently substituted or unsubstituted dibenzofuran, substituted or unsubstituted dibenzothiophene, or substituted or unsubstituted C6-C30 aromatic hydrocarbon,
m, n, o, and p are each independently an integer from 0 to 1, with the proviso that m+n+o+p is an integer from 1 to 3, and
R's are identical to or different from each other and are each independently selected from hydrogen, deuterium, a substituted or unsubstituted C1-C30 alkyl group, a substituted or unsubstituted C3-C30 cycloalkyl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylsilyl group, and a substituted or unsubstituted C6-C30 arylsilyl group.

According to an embodiment of the present invention, the compound of [Formula I] may be a compound represented by the following [Formula I-1] and may have a structure including at least one dibenzofuran or dibenzothiophene. 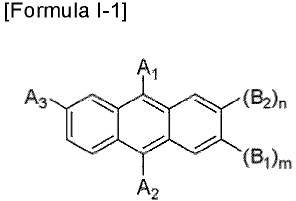 wherein
A₁ to A₃, B₁ and B₂ are identical to or different from each other and are each independently substituted or unsubstituted dibenzofuran, substituted or unsubstituted dibenzothiophene, or substituted or unsubstituted C6-C30 aromatic hydrocarbon, and
m and n are each independently an integer from 0 to 1, and m+n is preferably an integer of 1.

Further described herein but not according to the present invention is an organic light-emitting device including the anthracene derivative compound represented by [Formula I] in a light-emitting layer and which may further include a dopant compound represented by the following [Formula A-1], [Formula A-2], [Formula B], or [Formula C] in the light-emitting layer. 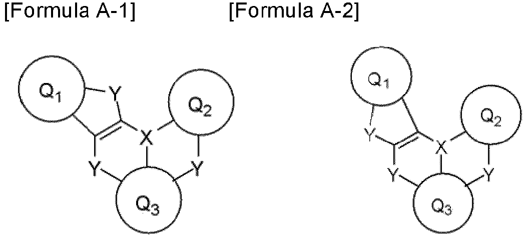 wherein
Q₁ to Q₃ are identical to or different from each other and are each independently a substituted or unsubstituted C6-C50 aromatic hydrocarbon ring, or a substituted or unsubstituted C2-C50 aromatic heterocyclic ring, and
X is selected from B, P and P=O, Y is selected from N-R₁, CR₂R₃, O, S, Se, and SiR₄R₅, and Y's are identical to or different from each other,
wherein R₁ to R₅ are identical to or different from each other and are each independently selected from hydrogen, deuterium, a substituted or unsubstituted C1-C30 alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthio group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C5-C30 arylsilyl group, a nitro group, a cyano group, and a halogen group,
wherein each of R₁ to R₅ is bonded to the ring Q₁ to Q₃ to further form an alicyclic or aromatic monocyclic or polycyclic ring, and R₂ and R₃, and R₄ and R₅ are bonded to each other to further form an alicyclic or aromatic monocyclic or polycyclic ring.

Further described herein but not according to the present invention is that [Formula A-1] or [Formula A-2] may be represented by any one of the following [Formula A-3] to [Formula A-6]. 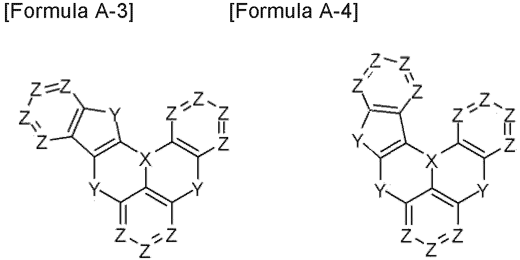 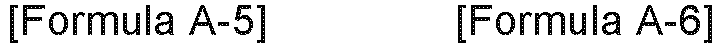 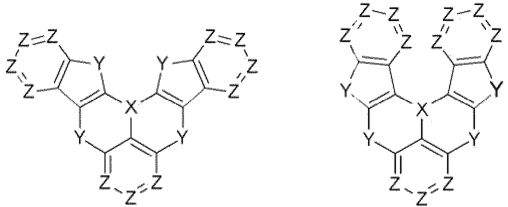 wherein
Z is CR or N, with the proviso that R is selected from hydrogen, deuterium, a substituted or unsubstituted C1-C30 alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C5-C30 arylsilyl group, a nitro group, a cyano group, a halogen group, and Z's and R's are identical to or different from each other,
wherein R's are bonded to each other or each thereof is bonded to an adjacent substituent to form at least one alicyclic or aromatic monocyclic or polycyclic ring, and the carbon atom of the formed alicyclic or aromatic monocyclic or polycyclic ring is substituted with at least one heteroatom selected from (N), a sulfur atom (S), and an oxygen atom (O),
X and Y are each the same as defined in [Formula A-1] and [Formula A-2]. 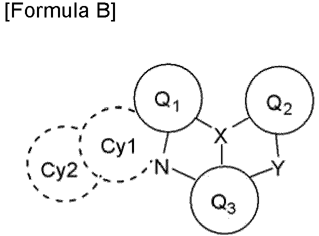 wherein
   Q₁ to Q₃ are identical to or different from each other and are each independently a substituted or unsubstituted C6-C50 aromatic hydrocarbon ring, or a substituted or unsubstituted C2-C50 aromatic heterocyclic ring, and
   X is selected from B, P and P=O, and Y is selected from N-R₆, CR₇R₈, O, S, and Se,
   wherein R₆ to R₈ are identical to or different from each other and are each independently selected from hydrogen, deuterium, a substituted or unsubstituted C1-C30 alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C5-C30 arylsilyl group, a nitro group, a cyano group, and a halogen group,
   wherein each of R₆ to R₈ is bonded to the ring Q₂ to Q₃ to further form an alicyclic or aromatic monocyclic or polycyclic ring, and R₇ and R₈ are bonded to each other to further form an alicyclic or aromatic monocyclic or polycyclic ring,
   Cy1 is linked to the nitrogen (N) atom and an aromatic carbon atom in the Q₁ ring to form a fused ring and the formed Cy1 ring is a substituted or unsubstituted C1-C10 alkylene group, preferably a substituted or unsubstituted C2-C5 alkylene group, provided that the nitrogen (N) atom, the aromatic carbon atom in the Q₁ ring to which the nitrogen (N) atom is bonded, and the aromatic carbon atom in the Q₁ ring to which the Cy1 ring is bonded are excluded,
   Cy2 is added to Cy1 to form a saturated hydrocarbon ring and the formed Cy2 ring is a substituted or unsubstituted C1-C10 alkylene group, provided that carbon atoms included in Cy1 are excluded.

At least one of Q₁ to Q₃ in [Formula B] is bound to an arylamino group represented by the following [Structural Formula 1] in the ring. 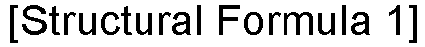 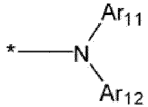 wherein
"*" refers to a site bonded to at least one ring of Q₁ to Q₃, Ar₁₁ and Ar₁₂ are identical to or different from each other and are each independently a substituted or unsubstituted C6-C50 aryl group, or a substituted or unsubstituted C2-C50 heteroaryl group, preferably a substituted or unsubstituted C6-C18 aryl group.
In addition, the Q₂ ring in [Formula B] may include the amine group substituted with naphthyl, that is, the Q₂ ring may include [Structural Formula 1] and at least one of Ar₁₁ and Ar₁₂ includes a naphthyl group.

According to the present invention, a further compound that is comprised by the organic light-emitting device of the present invention is represented by the following [Formula B-1]. 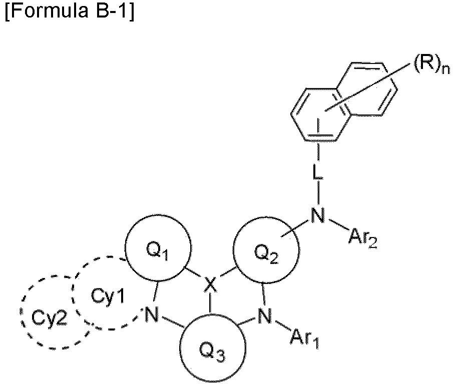 wherein
Q₁ to Q₃, Cy1 and Cy2 are as defined in [Formula B],
Ar₁ and R are each independently selected from hydrogen, deuterium, a substituted or unsubstituted C1-C30 linear, branched or cyclic alkyl group, a substituted or unsubstituted C1-C30 halogenated linear, branched or cyclic alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C6-C30 arylsilyl group, a nitro group, a cyano group, and a halogen group, and
Ar₂'s are identical to or different from each other and are each independently a substituted or unsubstituted C6-C50 aryl group, or a substituted or unsubstituted C2-C50 heteroaryl group,
wherein each of Ar₁ is optionally bonded to Q₂ or Q₃ to further form an alicyclic or aromatic monocyclic or polycyclic ring,
n is an integer from 1 to 7, and R's are identical to or different from each other provided that n is 2 or more, and
L is a single bond or is selected from a substituted or unsubstituted C6-C30 arylene group, and a substituted or unsubstituted C1-C30 heteroarylene group. 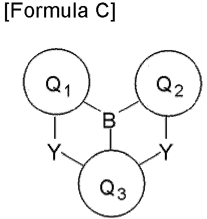 wherein
   Q₁ to Q₃ are identical to or different from each other and are each independently a substituted or unsubstituted C6-C50 aromatic hydrocarbon ring, or a substituted or unsubstituted C2-C50 aromatic heterocyclic ring, and
   Y is selected from N-R₉, CR₁₀R₁₁, O, S, and Se, wherein Y's are identical to or different from each other,
   wherein R₉ to R₁₁ are identical to or different from each other and are each independently selected from hydrogen, deuterium, a substituted or unsubstituted C1-C30 alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C5-C30 arylsilyl group, a nitro group, a cyano group, and a halogen group,
   wherein each of R₉ to R₁₁ is bonded to the ring Q₂ to Q₃ to further form an alicyclic or aromatic monocyclic or polycyclic ring, and
   R₁₀ and R₁₁ are bonded to each other to further form an alicyclic or aromatic monocyclic or polycyclic ring.

Meanwhile, as used herein, the term "substituted" indicates substitution of various substituents defined in [Formula I], [Formula A-1] or [Formula A-2], [Formula B], and [Formula C] with one or more substituents selected from deuterium, a cyano group, a halogen group, a hydroxyl group, a nitro group, a C1-C24 linear, branched or cyclic alkyl group, a C3-C24 cycloalkyl group, a C1-C24 halogenated linear, branched or cyclic alkyl group, a C1-C24 alkenyl group, a C1-C24 alkynyl group, a C1-C24 heteroalkyl group, a C1-C24 heterocycloalkyl group, a C6-C24 aryl group, a C6-C24 arylalkyl group, a C2-C24 heteroaryl group, a C2-C24 heteroarylalkyl group, a C1-C24 alkoxy group, a C1-C24 alkylamino group, a C1-C24 arylamino group, a C1-C24 heteroarylamino group, a C1-C24 alkylsilyl group, a C1-C24 arylsilyl group, and a C1-C24 aryloxy group, or substitution with a substituent including two or more of the substituents linked to each other. The term "unsubstituted" in the same definition indicates having no substituent.

In addition, the range of the number of the carbon atoms of the alkyl group or aryl group in the term "substituted or unsubstituted C1-C30 alkyl group", "substituted or unsubstituted C6-C50 aryl group" or the like refers to the total number of carbon atoms constituting the alkyl or aryl moiety when the corresponding group is not substituted without considering the number of carbon atoms in the substituent(s). For example, a phenyl group substituted at the para position with a butyl group corresponds to an aryl group having 6 carbon atoms substituted with a butyl group having 4 carbon atoms.

In addition, as used herein, the expression "a substituent is bonded to an adjacent substituent to form a ring" means that the corresponding substituent is bonded to the adjacent substituent to form a substituted or unsubstituted alicyclic or aromatic ring, and the term "adjacent substituent" may mean a substituent substituted for an atom which is directly attached to an atom substituted with the corresponding substituent, a substituent sterically disposed at the nearest position to the corresponding substituent, or another substituent substituted for an atom which is substituted with the corresponding substituent. For example, two substituents substituted at the ortho position in a benzene ring and two substituents substituted at the same carbon in the aliphatic ring may be considered "adjacent" to each other.

As used herein, the alkyl group may be a linear or branched alkyl group. The carbon number thereof is not specifically limited and is preferably 1 to 20. Examples of the alkyl group include, but are not limited to, a methyl group, an ethyl group, a propyl group, an n-propyl group, an isopropyl group, a butyl group, an n-butyl group, an isobutyl group, a tert-butyl group, a sec-butyl group, a 1-methylbutyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like.

As used herein, the alkenyl group may include a linear or branched alkenyl group and may be further substituted with another substituent. Specifically, examples of the alkenyl group include, but are not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like.

As used herein, the alkynyl group may also include a linear or branched alkynyl group, and may be further substituted with another substituent, and examples of the substituent may include, but are not limited to, ethynyl, 2-propynyl, and the like.

As used herein, the cycloalkyl group may include a monocyclic or polycyclic group and may be further substituted with another substituent. The polycyclic group means a cycloalkyl group directly bonded to or fused with another cyclic group and the other cyclic group may be a cycloalkyl group or other types of cyclic group such as a heterocycloalkyl, aryl, or heteroaryl group. Specifically, examples thereof include, but are not limited to, a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, and a cyclooctyl group.

As used herein, the term "heterocycloalkyl group" refers to a cyclic radical containing at least one heteroatom such as O, S, Se, N or Si, includes a monocyclic or polycyclic group and may be further substituted with another substituent. The polycyclic group means a heterocycloalkyl group directly bonded to or fused with another cyclic group and the other cyclic group may be a heterocycloalkyl group or other types of cyclic group such as cycloalkyl, aryl, or heteroaryl group.

As used herein, the aryl group may be monocyclic or polycyclic, and examples of the monocyclic aryl group include a phenyl group, a biphenyl group, a terphenyl group, a stilbene group, and the like, and examples of the polycyclic aryl group include, but are not limited to, a naphthyl group, an anthracenyl group, a phenanthrenyl group, a pyrenyl group, a perylenyl group, a tetracenyl group, a chrysenyl group, a fluorenyl group, an acenaphthenyl group, a triphenylene group, a fluoranthene group, and the like, but the scope of the present invention is not limited thereto.

As used herein, the heteroaryl group is a heterocyclic ring containing at least one heteroatom and examples thereof include, but are not limited to, thiophene, furan, pyrrole, imidazole, triazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, and phenothiazinyl groups and the like.

As used herein, specifically, the alkoxy group may be methoxy, ethoxy, propoxy, isobutyloxy, sec-butyloxy, pentyloxy, iso-amyloxy, hexyloxy, or the like, but is not limited thereto.

As used herein, the silyl group means an alkyl-substituted silyl group or aryl-substituted silyl group. Specific examples of the silyl group include trimethylsilyl, triethylsilyl, triphenylsilyl, trimethoxysilyl, dimethoxyphenylsilyl, diphenylmethylsilyl, diphenylvinylsilyl, methylcyclobutylsilyl, dimethylfurylsilyl, and the like.

As used herein, the amine group may be -NH₂, an alkylamine group, an arylamine group, or the like. The arylamine group refers to amine substituted with aryl, and the alkylamine group refers to amine substituted with alkyl. For example, the arylamine group includes a substituted or unsubstituted monoarylamine group, a substituted or unsubstituted diarylamine group, or a substituted or unsubstituted triarylamine group. The aryl group in the arylamine group may be a monocyclic aryl group or a polycyclic aryl group. The arylamine group that contains two or more aryl groups may include a monocyclic aryl group, a polycyclic aryl group, or both the monocyclic aryl group and the polycyclic aryl group. In addition, the aryl group in the arylamine group may be selected from examples of aryl groups described above.

As used herein, examples of the aryl group in the aryloxy group and the arylthioxy group are identical to examples of the aryl group described above and specifically, examples of the aryloxy group include a phenoxy group, a p-tolyloxy group, an m-tolyloxy group, a 3,5-dimethylphenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, and examples of the arylthioxy group include, but are not limited to, a phenylthioxy group, a 2-methylphenylthioxy group, a 4-tert-butylphenylthioxy group, and the like.

In the present invention, examples of the halogen group include fluorine, chlorine, bromine, and iodine.

In an embodiment of the present invention, the compound represented by [Formula I] is selected from the compounds represented by the following [Formula 1] to [Formula 123], but these compounds should not be construed as limiting the scope of [Formula I].

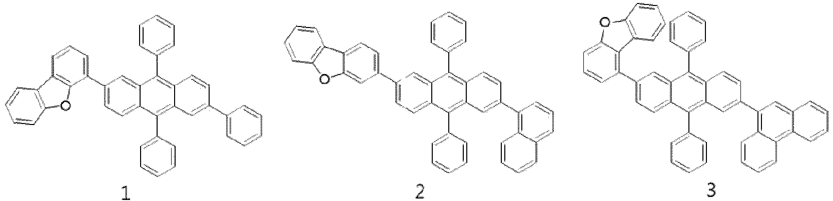

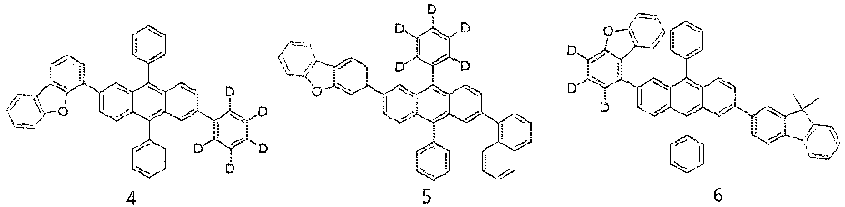

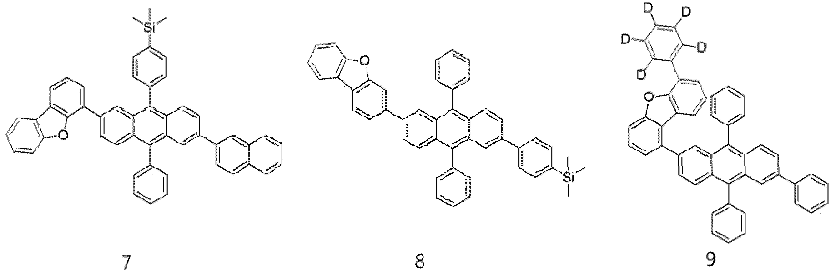

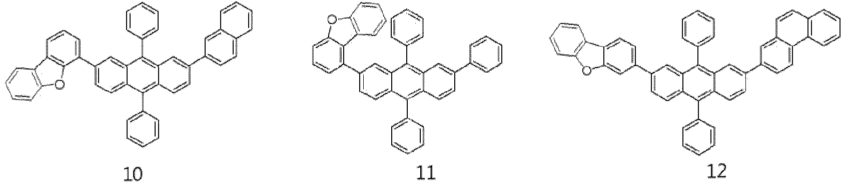

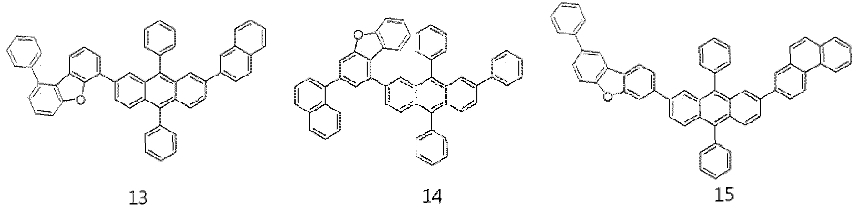

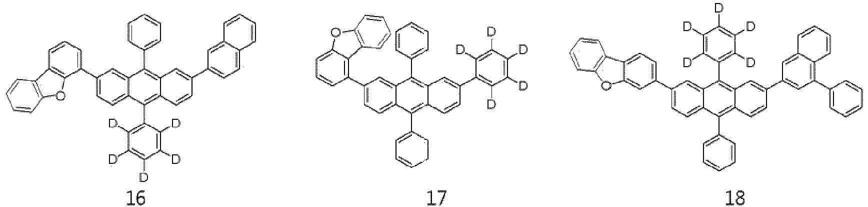

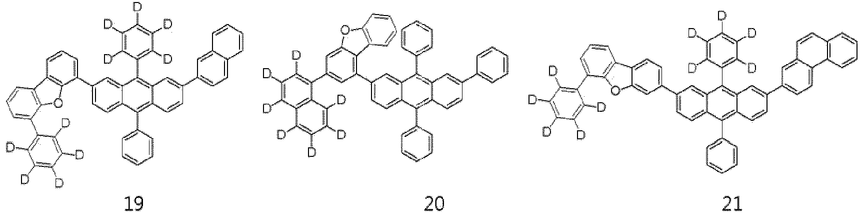

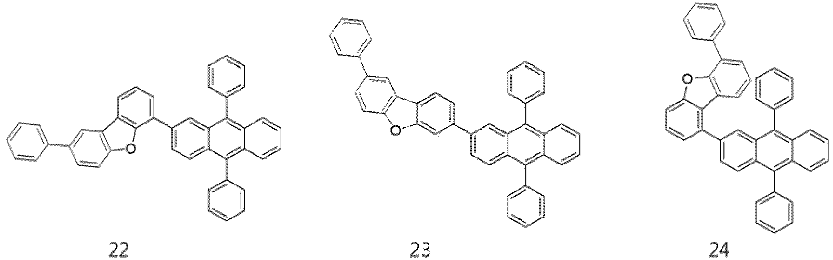

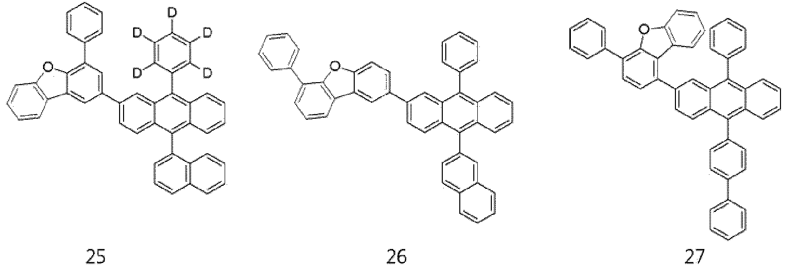

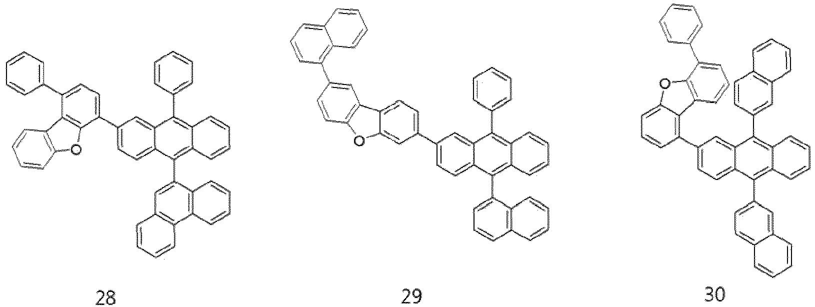

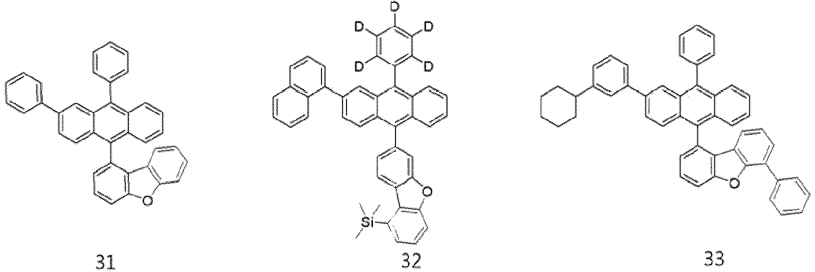

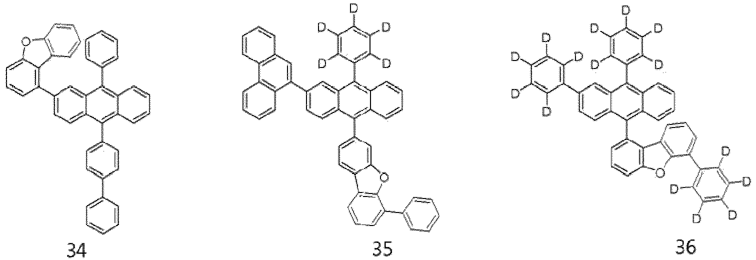

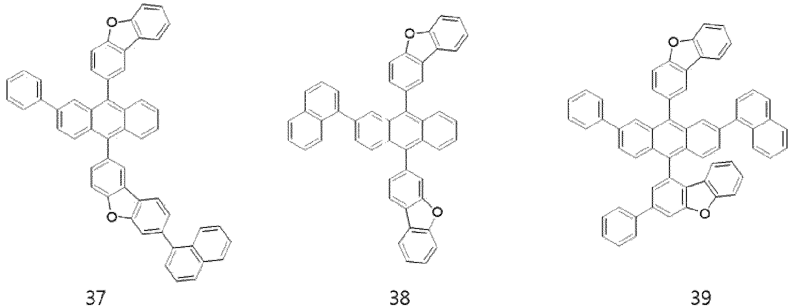

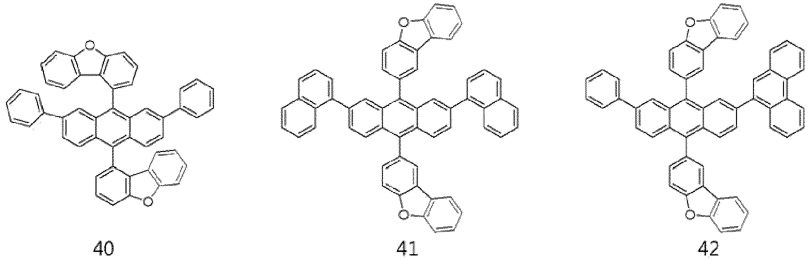

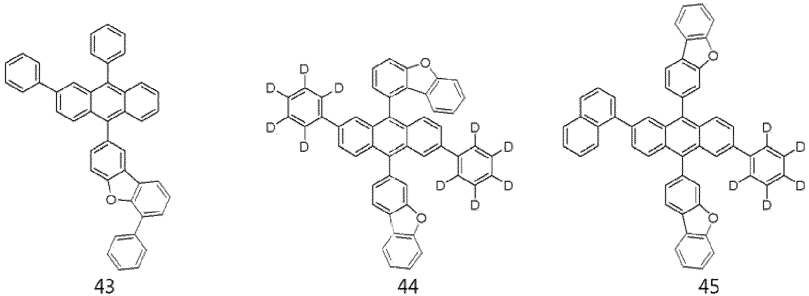

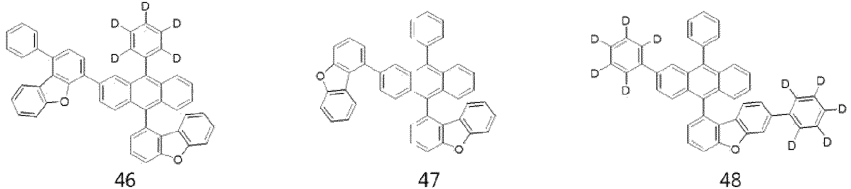

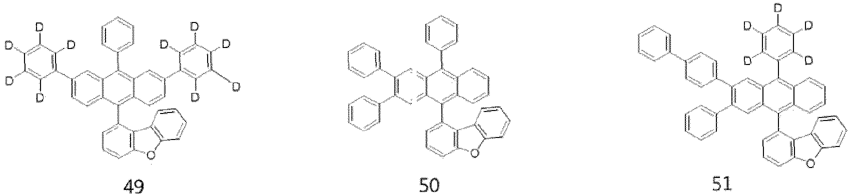

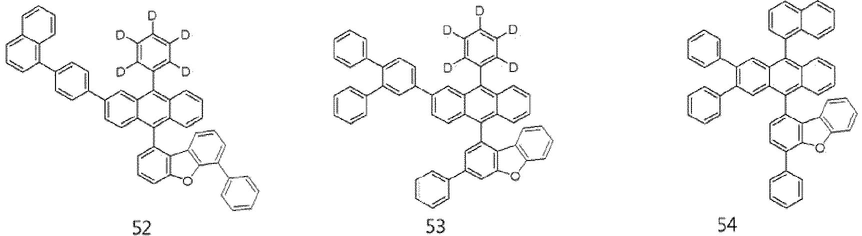

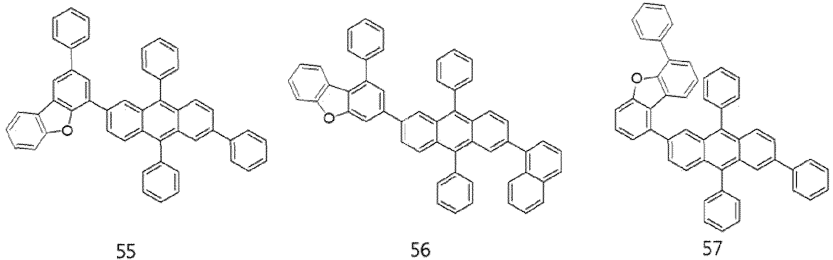

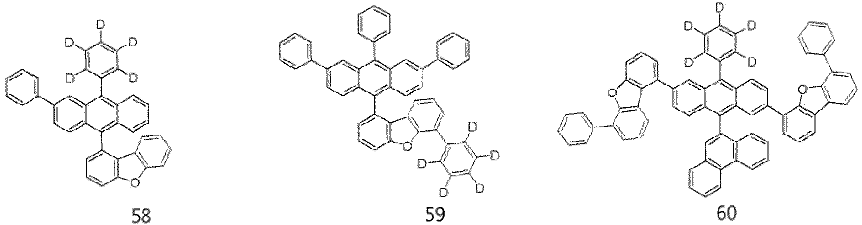

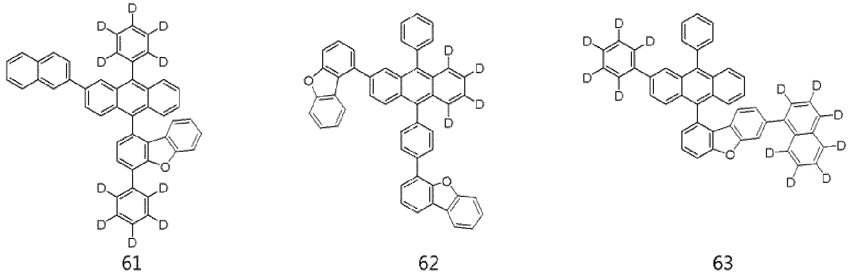

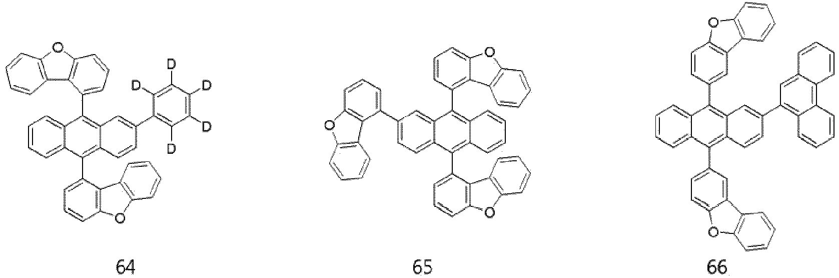

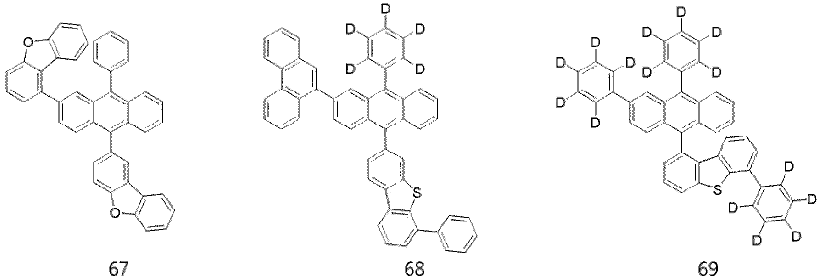

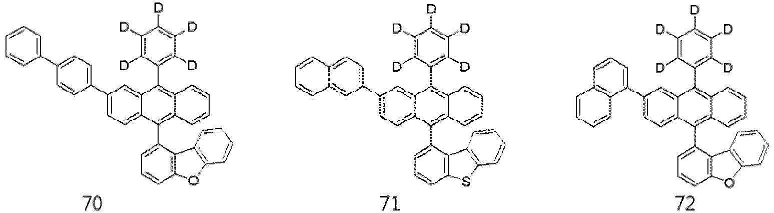

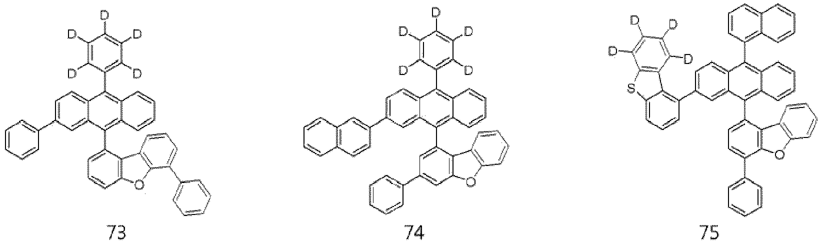

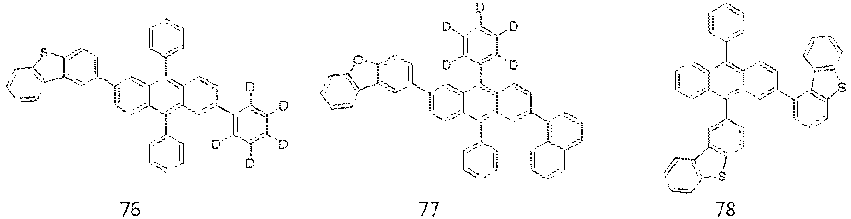

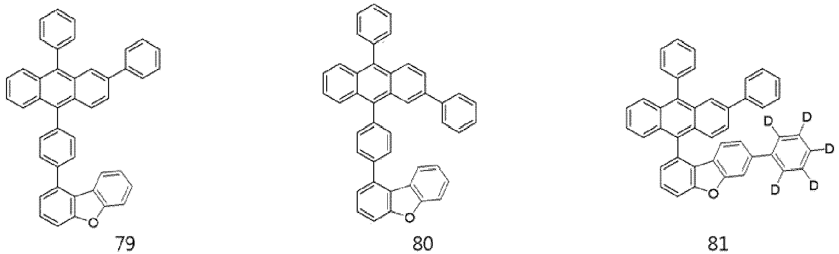

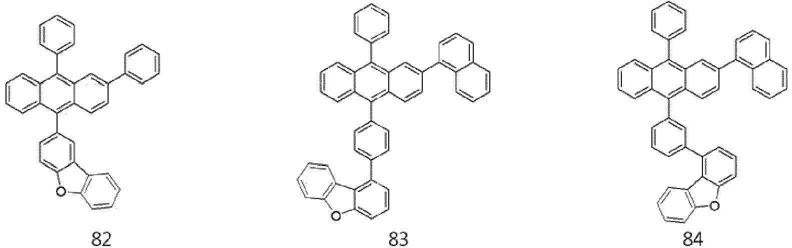

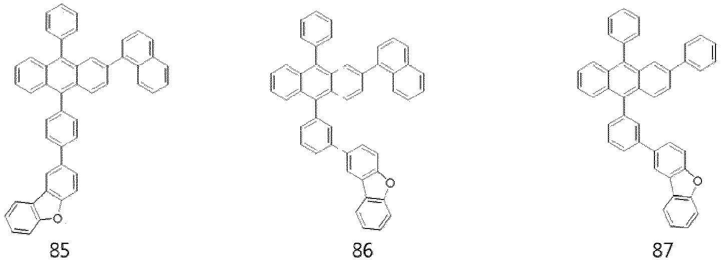

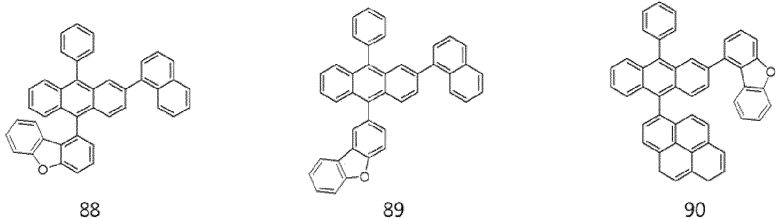

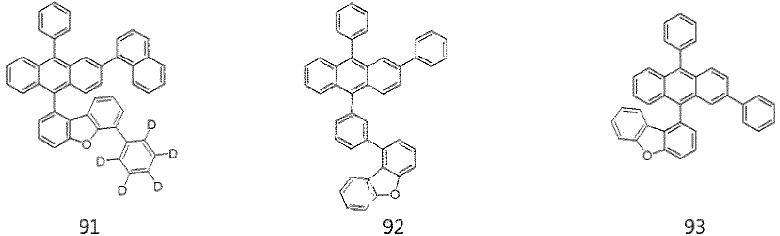

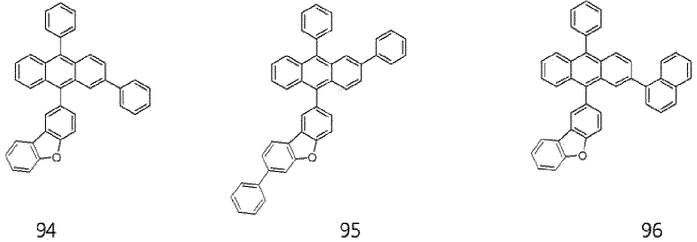

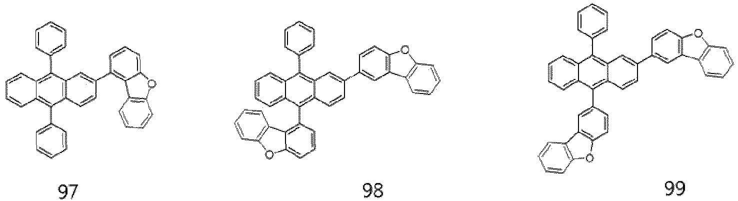

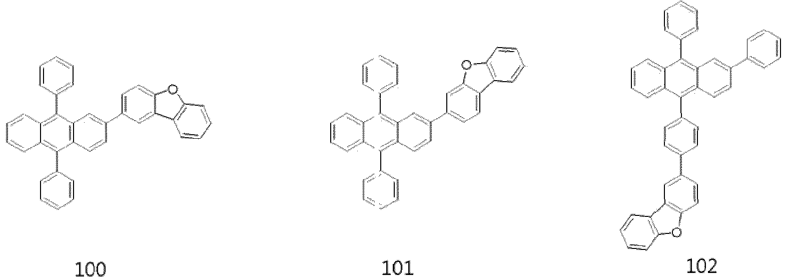

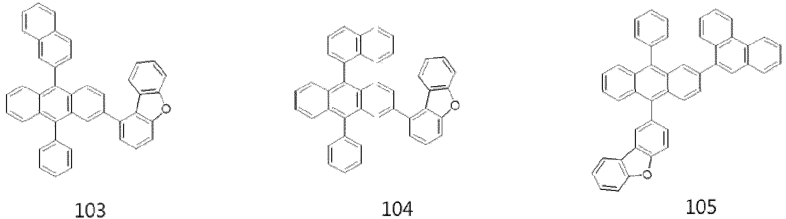

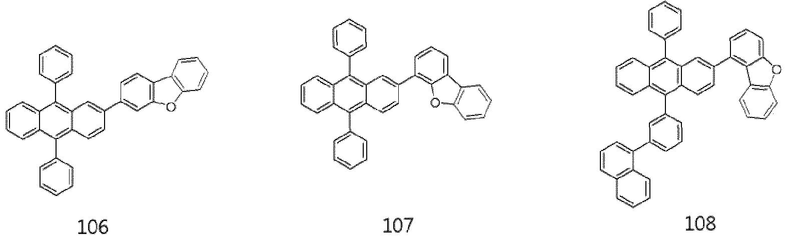

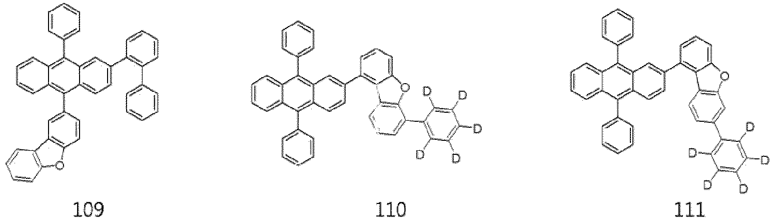

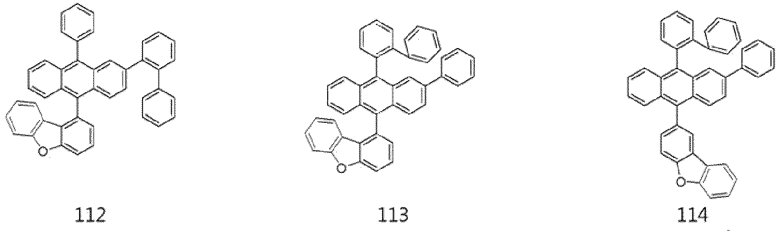

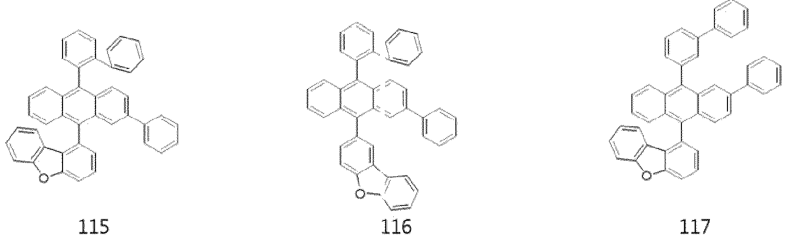

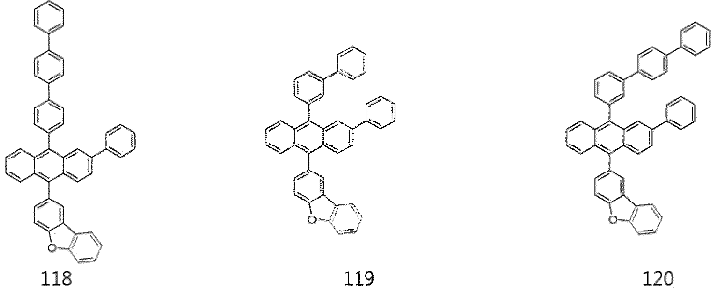

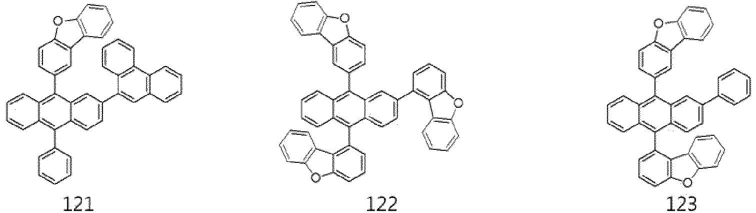

The compound represented by [Formula A-1] or [Formula A-2] (not according to the invention) is selected from the compounds represented by the following [Formula A1] to [Formula A212], but these compounds should not be construed as limiting the scope of [Formula A-1] or [Formula A-2].

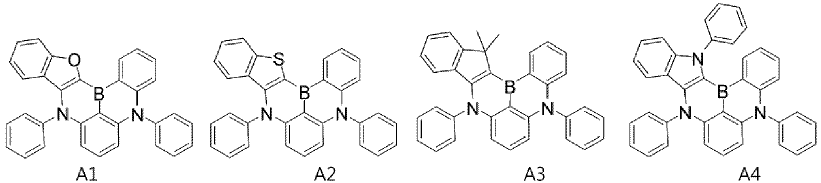

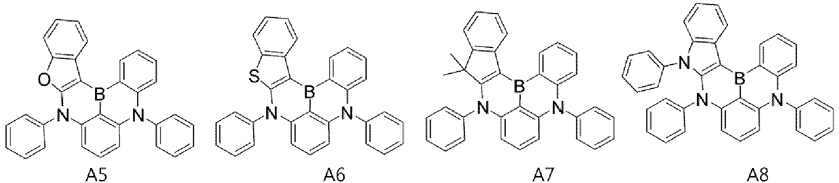

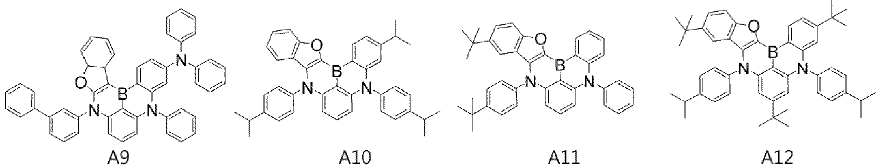

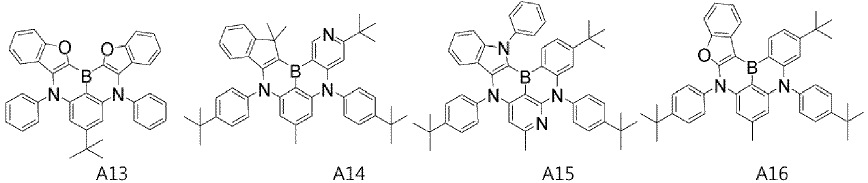

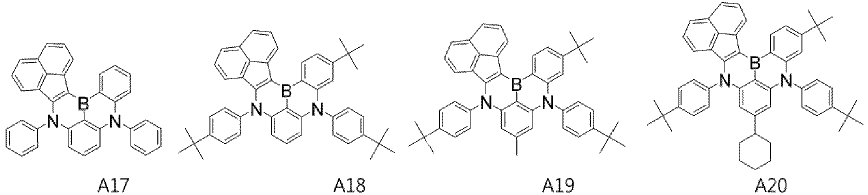

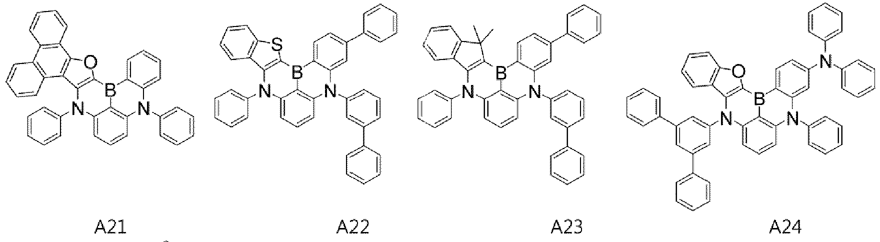

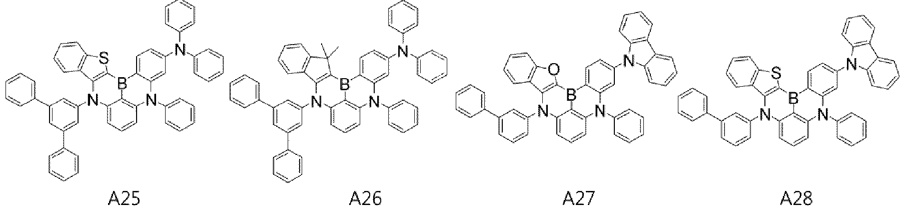

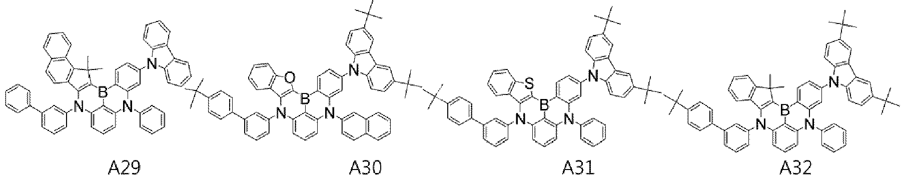

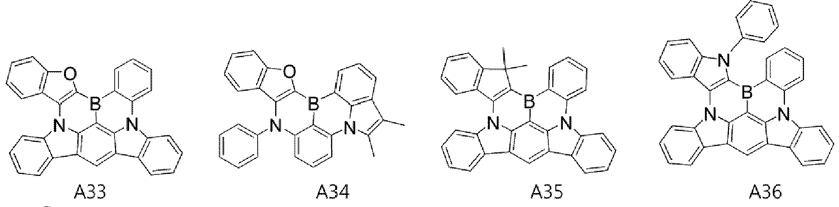

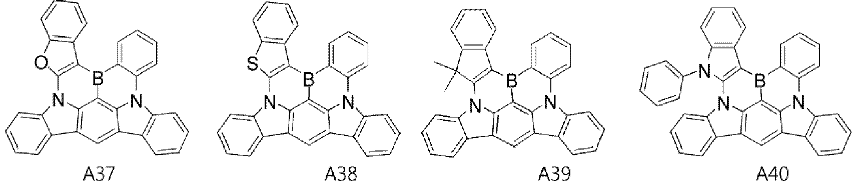

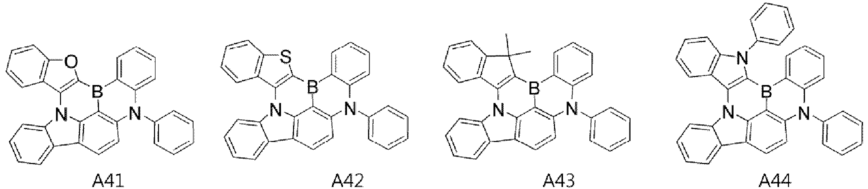

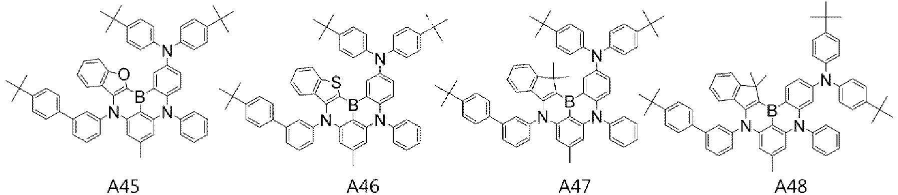

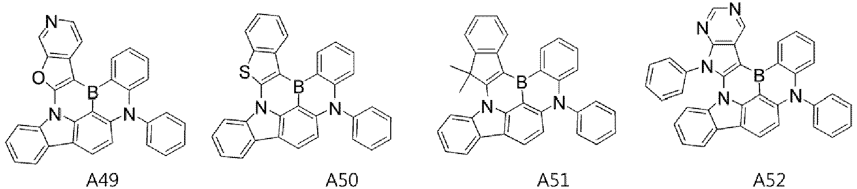

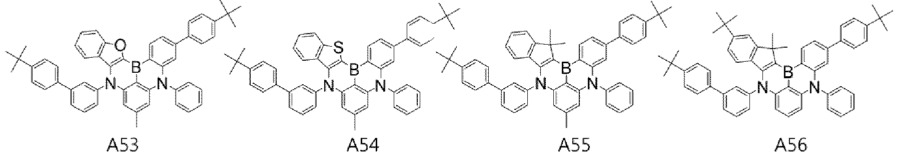

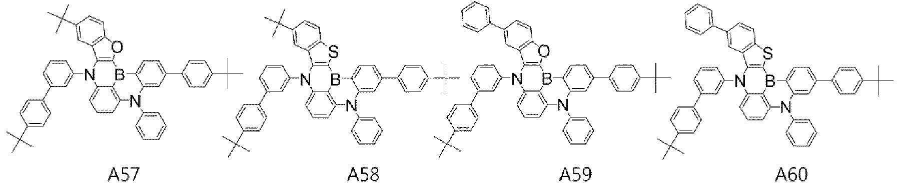

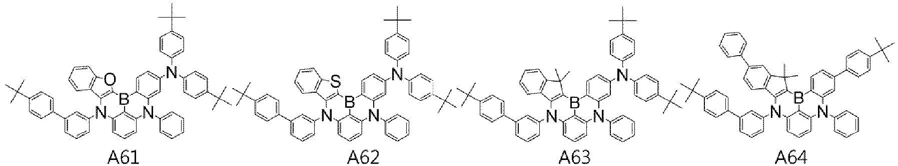

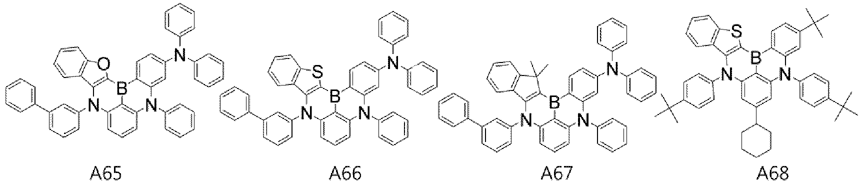

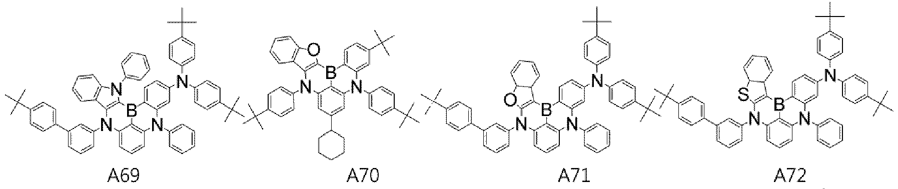

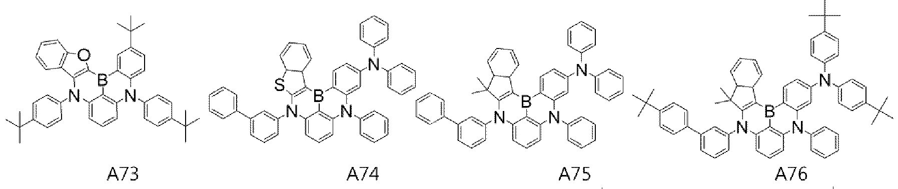

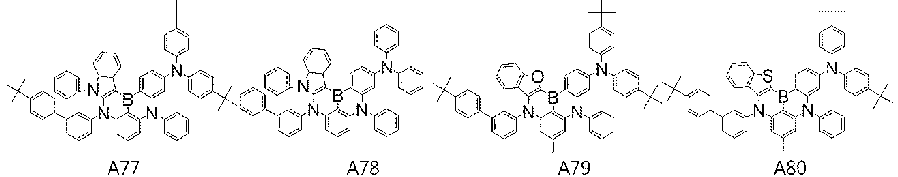

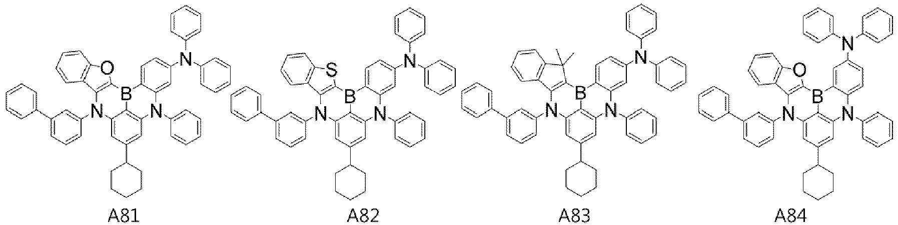

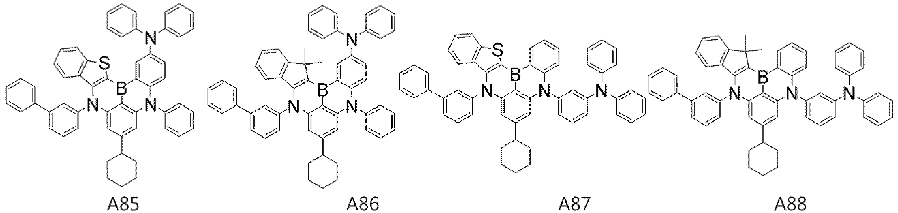

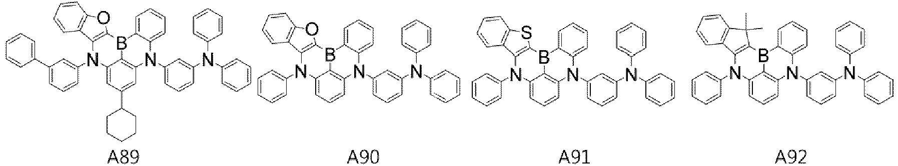

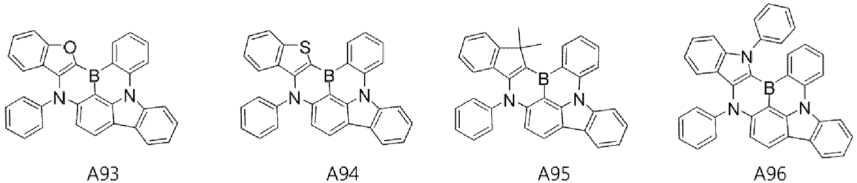

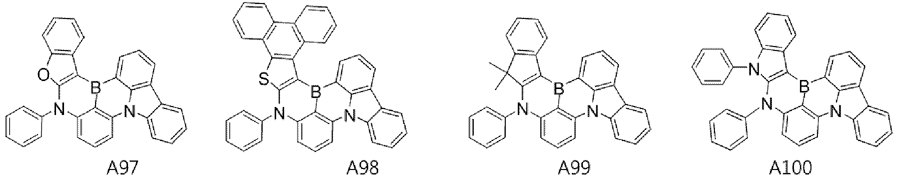

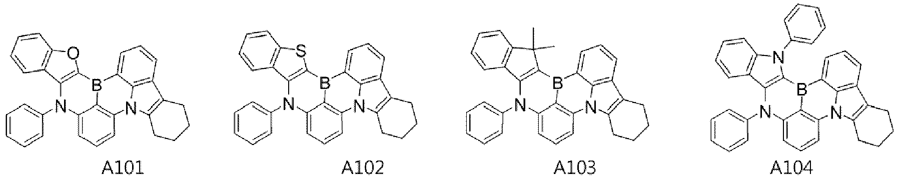

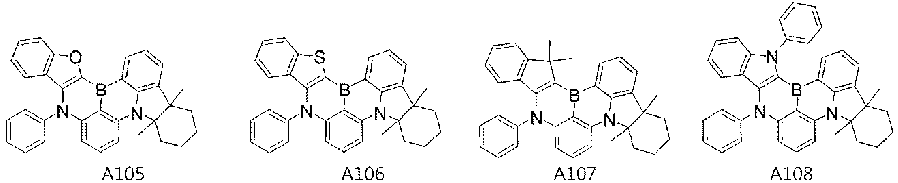

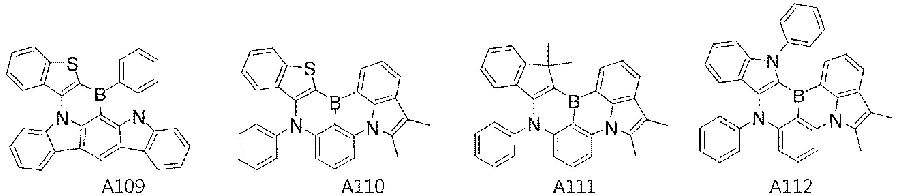

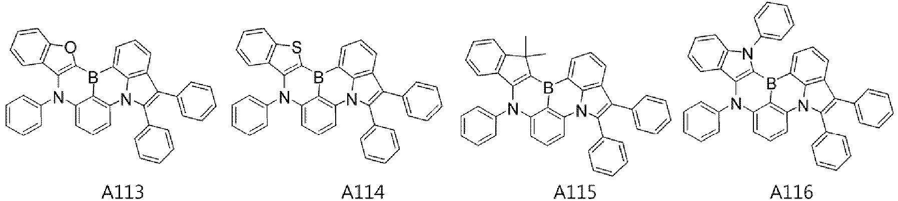

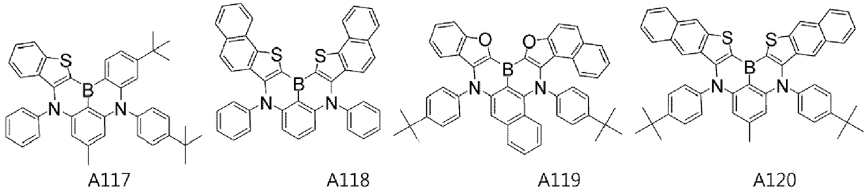

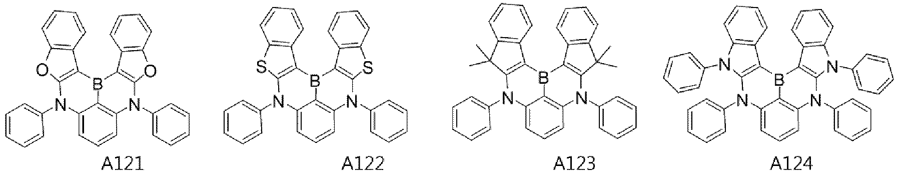

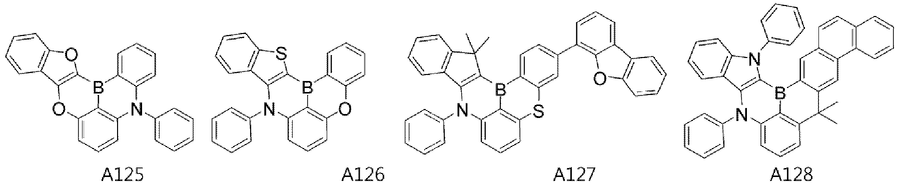

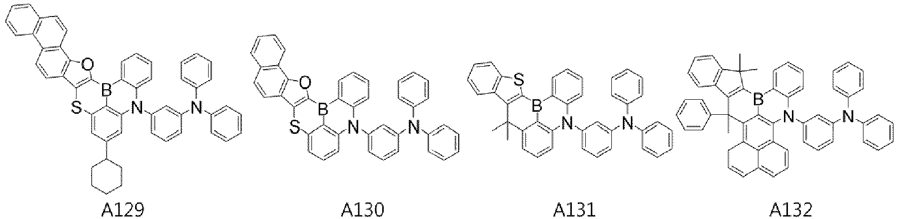

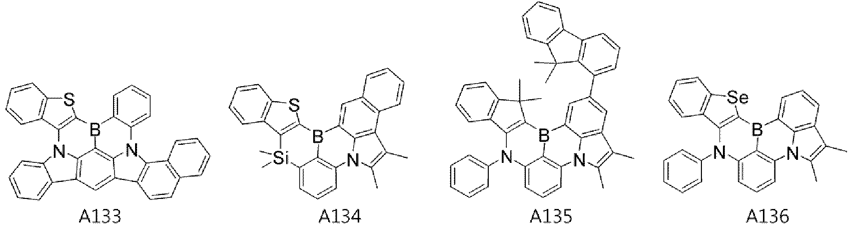

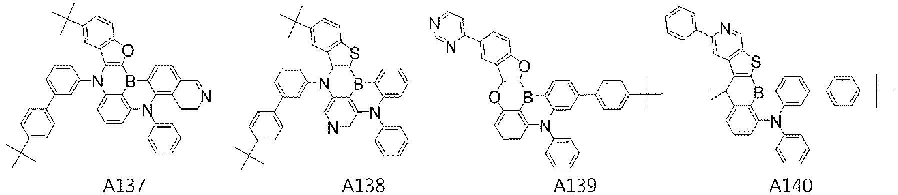

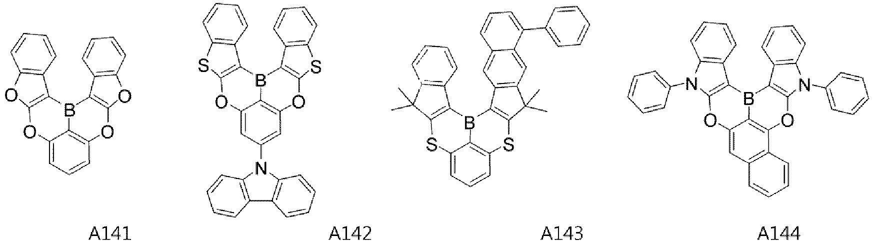

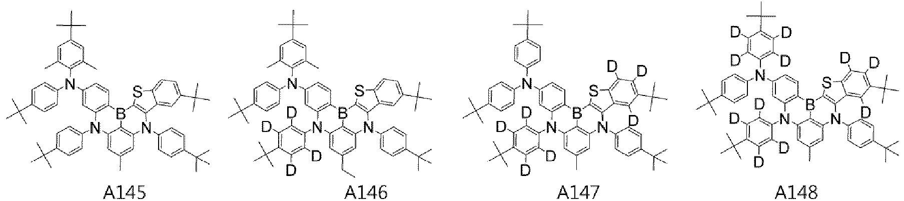

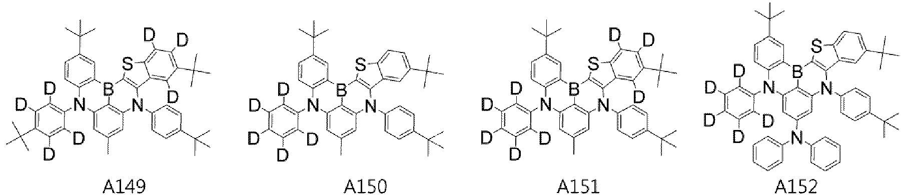

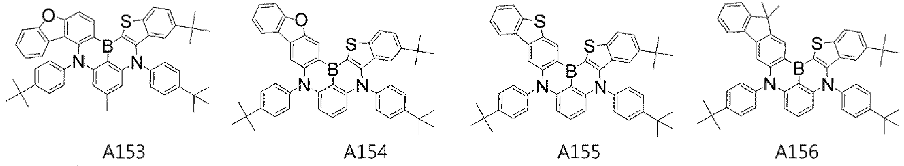

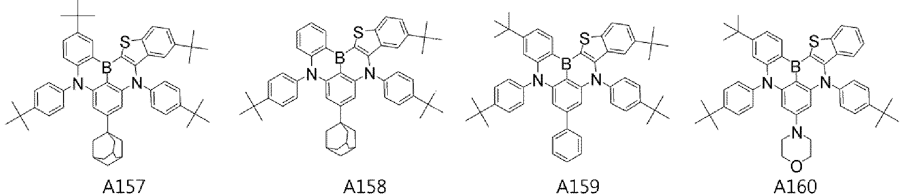

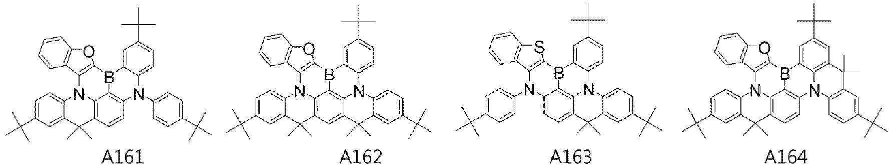

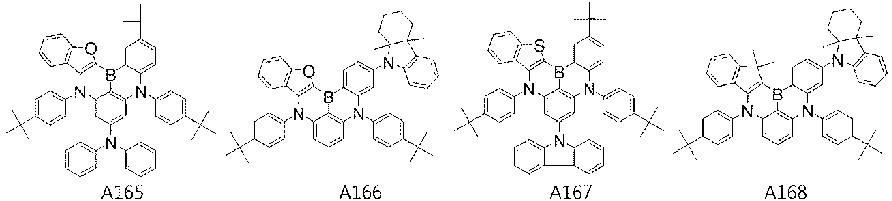

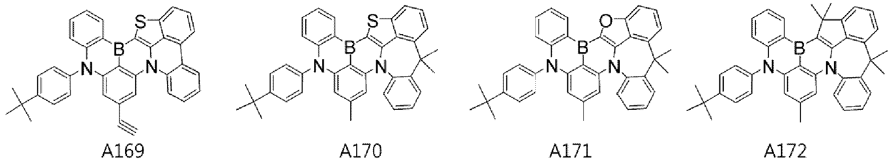

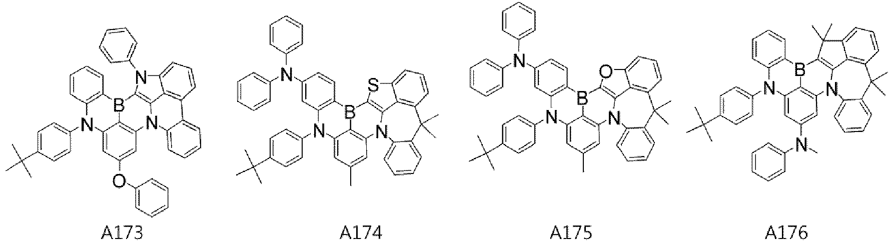

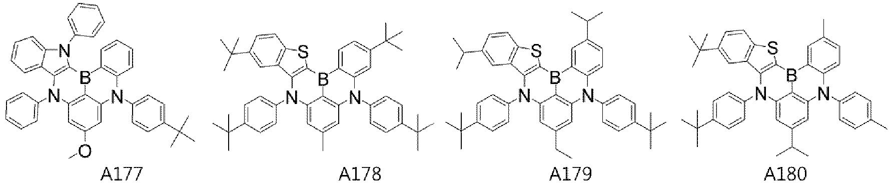

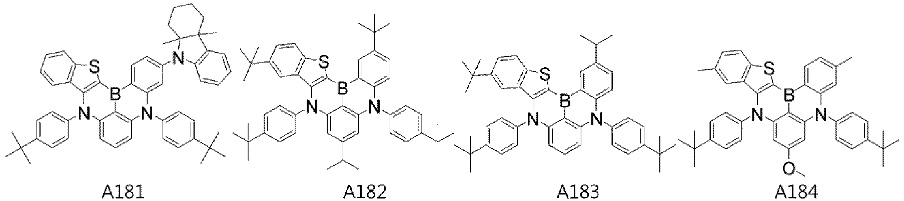

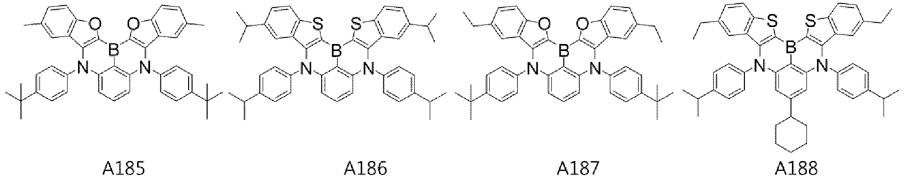

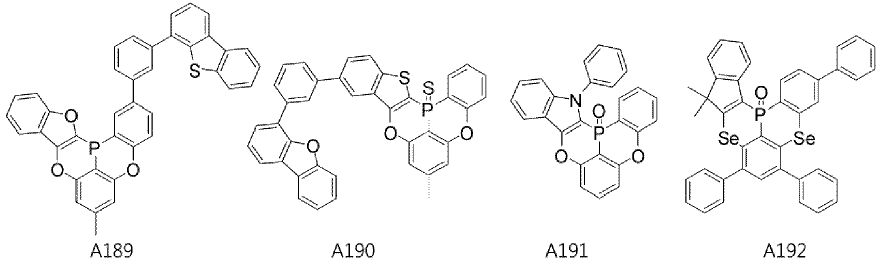

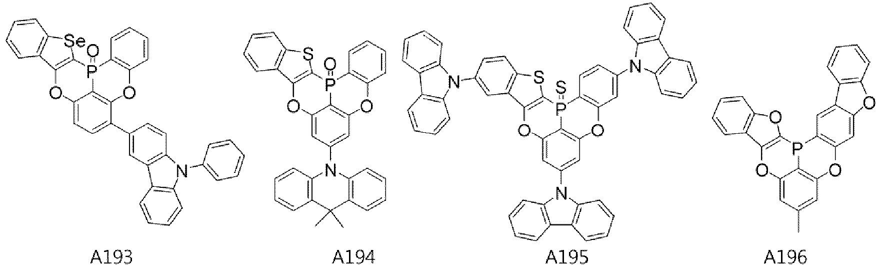

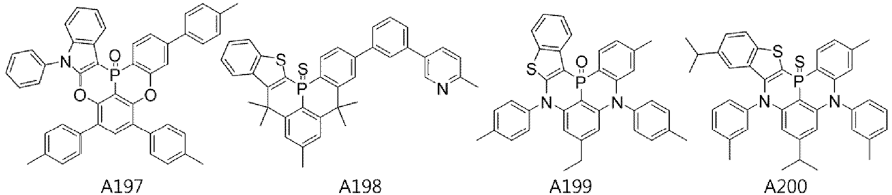

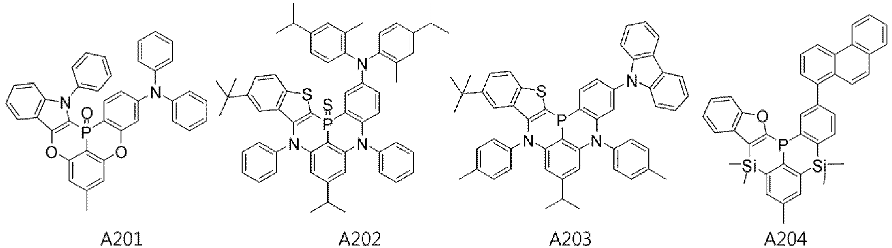

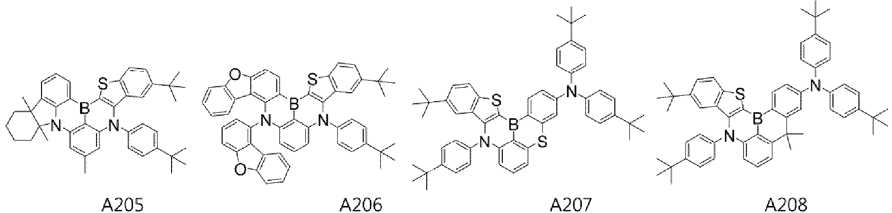

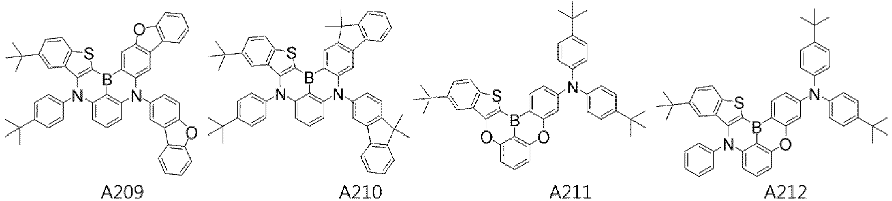

In an embodiment of the present invention, the compound represented by [Formula B-1] is selected from the compounds represented by the following [Formula B91] to [Formula B105], and [Formula B112] to [Formula B135] but these compounds should not be construed as limiting the scope of [Formula B].

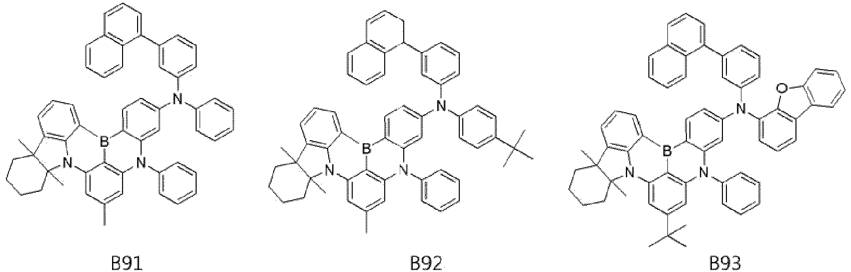

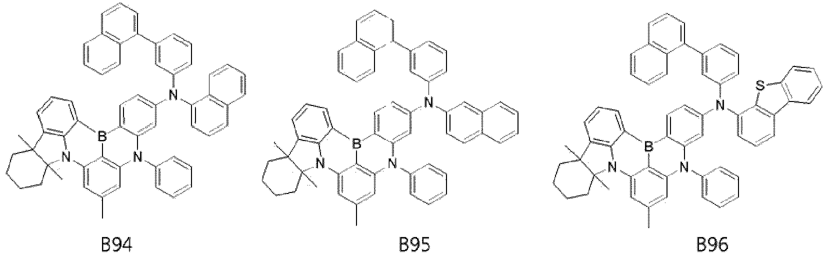

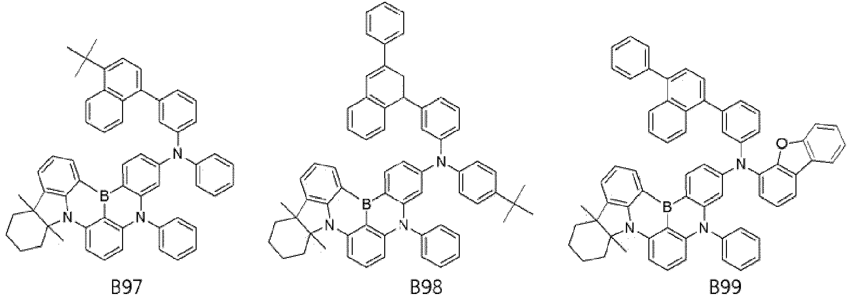

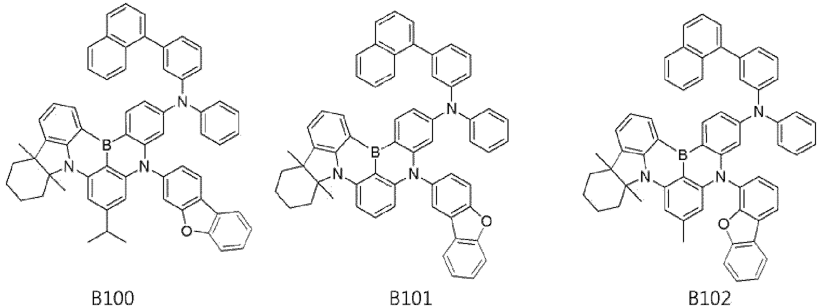

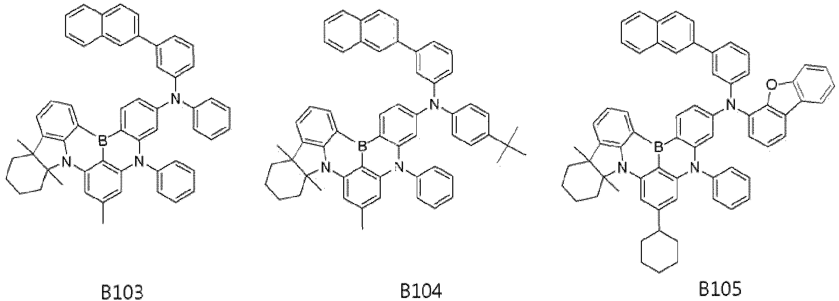

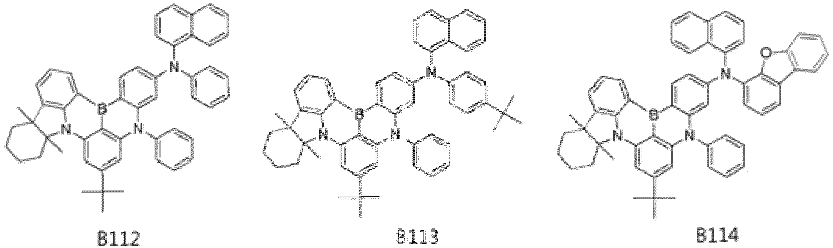

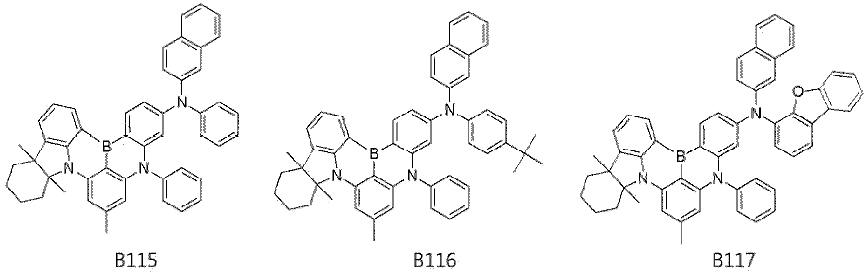

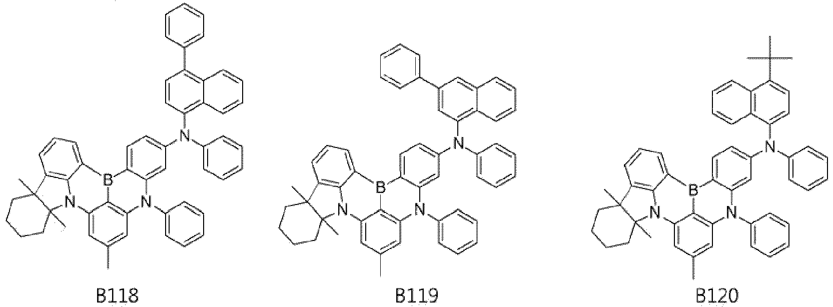

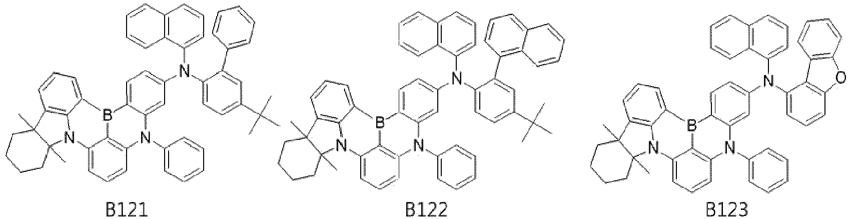

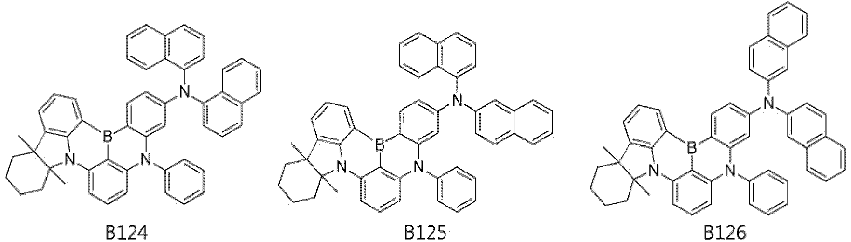

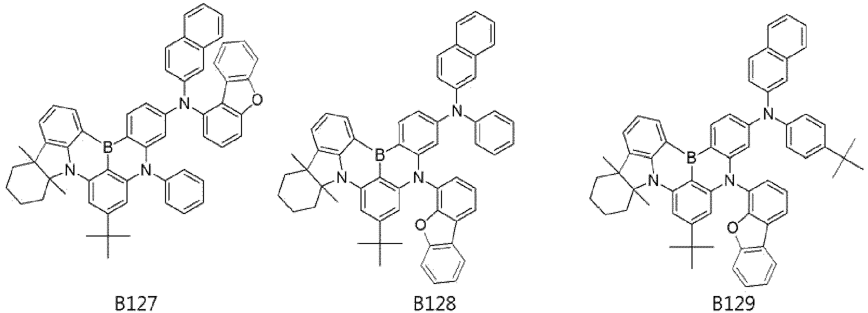

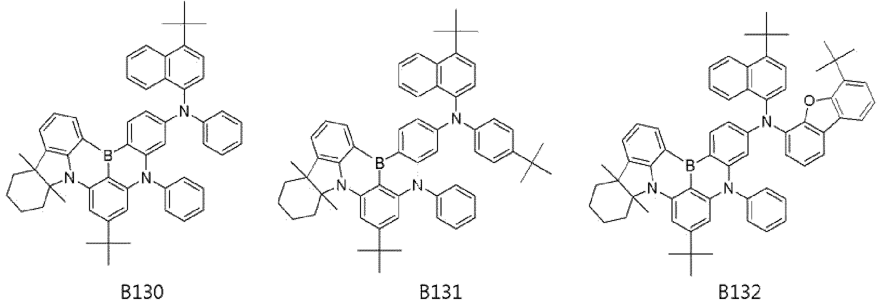

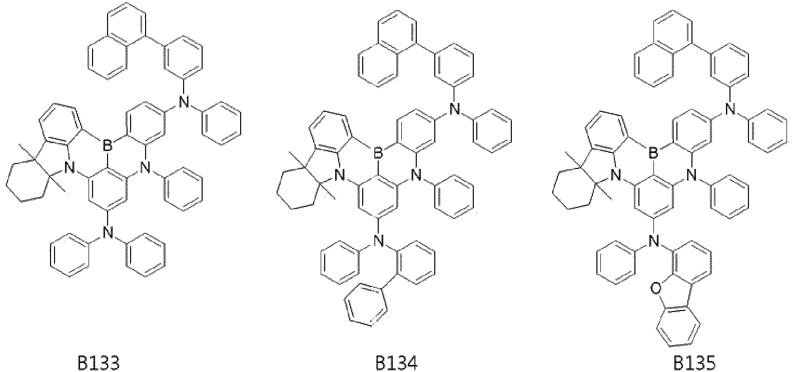

In an embodiment which is not according to the present invention, the compound represented by [Formula C] is selected from the compounds represented by the following [Formula C1] to [Formula C30], but these compounds should not be construed as limiting the scope of [Formula C].

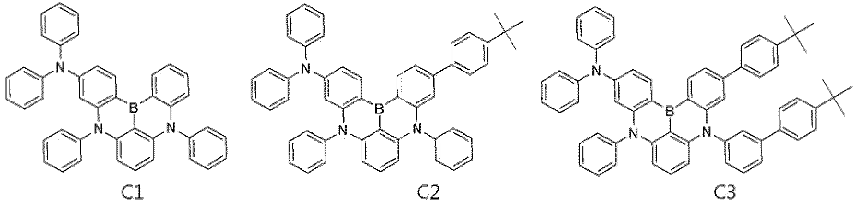

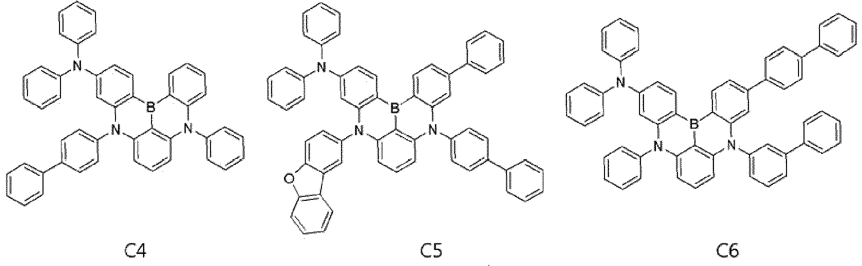

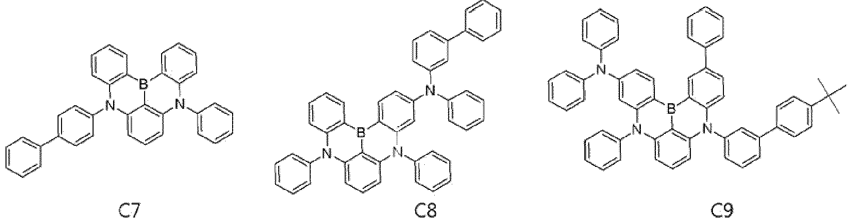

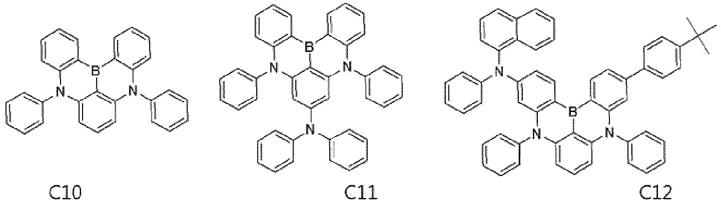

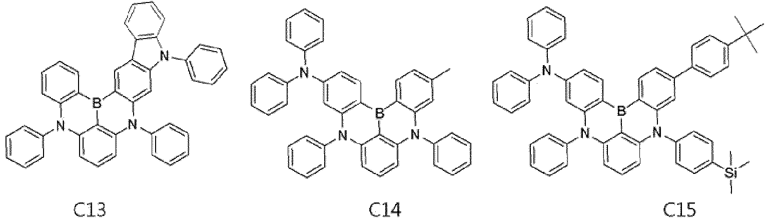

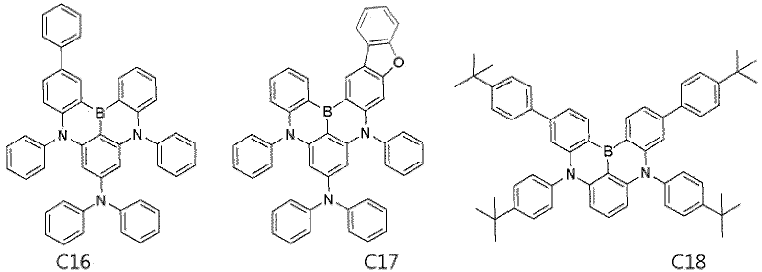

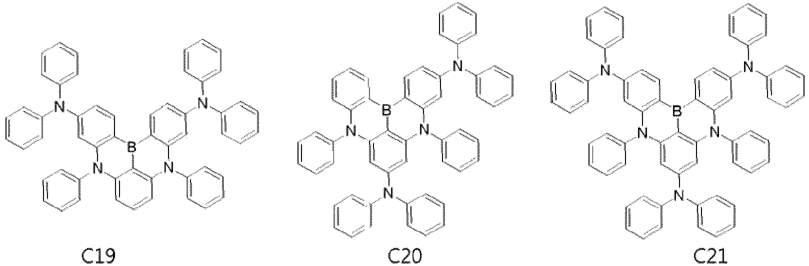

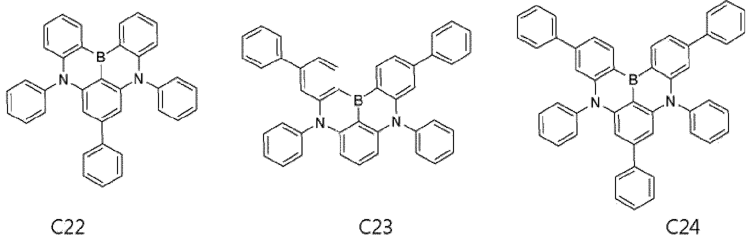

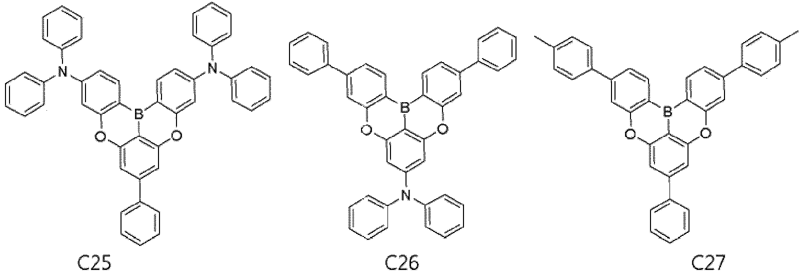

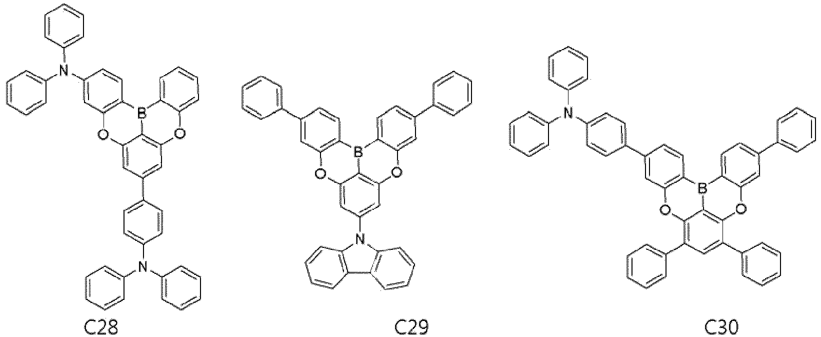

In addition, described herein but not according to the present invention is an organic light-emitting device including a first electrode, a second electrode, and at least one organic layer interposed between the first electrode and the second electrode, wherein the organic layer, preferably, a light-emitting layer, includes the anthracene derivative compound represented by [Formula I].

In addition, the organic light-emitting device not according to the present invention further includes a dopant compound represented by [Formula A-1], [Formula A-2], [Formula B], or [Formula C] in the light-emitting layer in the device.

The organic layer of the organic light-emitting device according to the present invention may have a single layer structure or a multilayer structure in which two or more organic layers are stacked. For example, the organic layer may have a structure including a hole injection layer, a hole transport layer, a hole blocking layer, a light-emitting layer, an electron blocking layer, an electron transport layer, an electron injection layer, and the like. However, the structure of the organic layer is not limited thereto and may include a smaller or larger number of organic layers, and the preferred organic material layer structure of the organic light-emitting device according to the present invention will be described in more detail with reference to Example which will be described later.

In addition, the organic light-emitting device according to an embodiment of the present invention includes a substrate, a first electrode (anode), an organic material layer, a second electrode (cathode), and a capping layer, wherein the capping layer is formed under the first electrode (bottom emission) or on the second electrode (top emission).

In the configuration in which the capping layer is formed on the second electrode (top emission), light formed in the light-emitting layer is emitted toward the cathode, and the light emitted toward the cathode passes through the capping layer (CPL) including the compound having a relatively high refractive index according to the present invention. As a result, the wavelength of light is amplified and thus luminous efficacy is increased. In addition, the configuration in which the capping layer is formed under the first electrode (bottom emission) also improves the light efficacy of the organic light-emitting device using the compound according to the present invention in the capping layer in the same mechanism as the top emission.

Hereinafter, an embodiment of the organic light-emitting device according to the present invention will be described in more detail.

The organic light-emitting device according to the present invention includes an anode, a hole transport layer, a light-emitting layer, an electron transport layer, and a cathode, and if necessary, may further include a hole injection layer between the anode and the hole transport layer, may further include an electron injection layer between the electron transport layer and the cathode, may further include one or two intermediate layers, and may further include a hole blocking layer or an electron blocking layer. As described above, the organic light-emitting device may further include an organic layer, such as a capping layer, having various functions depending on characteristics thereof.

Meanwhile, the specific structure of the organic light-emitting device according to an embodiment of the present invention, the method of manufacturing the same, and respective organic layer materials will be described as follows.

First, a substrate is coated with a material for an anode to form the anode. The substrate used herein is a substrate generally used for organic light-emitting devices and is preferably an organic substrate or a transparent plastic substrate that has excellent transparency, surface evenness, handleability and waterproofness. In addition, a material for the anode is indium tin oxide (ITO), indium zinc oxide (IZO), tin oxide (SnO₂), zinc oxide (ZnO), or the like, which is transparent and has excellent conductivity.

A hole injection layer is formed on the anode by vacuum thermal evaporation or spin coating using a material for the hole injection layer, and then a hole transport layer is formed on the hole injection layer by vacuum thermal evaporation or spin coating using a material for the hole transport layer.

The material for the hole injection layer may be used without particular limitation as long as it is commonly used in the art and specific examples thereof include 2-TNATA [4,4',4"-tris(2-naphthylphenyl-phenylamino)-triphenylamine], NPD [N,N'-di(1-naphthyl)-N,N'-diphenylbenzidine)], TPD [N,N'-diphenyl-N,N'-bis(3-methylphenyl)-1,1'-biphenyl-4,4'-diamine], DNTPD [N,N'-diphenyl-N,N'-bis-[4-(phenyl-m-tolyl-amino)-phenyl]-biphenyl-4,4'-diamine], and the like.

In addition, the material for the hole transport layer is also used without particular limitation as long as it is commonly used in the art and is, for example, N,N'-bis(3-methylphenyl)-N,N'-diphenyl-[1,1-biphenyl]-4,4'-diamine (TPD) or N,N'-di(naphthalen-1-yl)-N,N'-diphenylbenzidine (α-NPD).

Subsequently, a hole auxiliary layer and a light-emitting layer are sequentially stacked on the hole transport layer, and a hole blocking layer is selectively deposited on the light-emitting layer by vacuum deposition or spin coating to form a thin film. Because the lifespan and efficiency of the device are reduced when holes are introduced into the cathode through the organic light-emitting layer, the hole blocking layer is formed using a material having a very low HOMO (highest occupied molecular orbital) level so as to prevent this problem. The hole blocking material used herein is not particularly limited and is typically BAlq, BCP or TPBI that has an electron transport ability and has an ionization potential higher than that of a light-emitting compound.

The material used for the hole blocking layer may be BAIq, BCP, Bphen, TPBI, NTAZ, BeBq₂, OXD-7, Liq, or the like, but is not limited thereto.

An electron transport layer is deposited on the hole blocking layer through vacuum deposition or spin coating and a metal for forming a cathode is formed on the electron injection layer through vacuum thermal evaporation to form a cathode. As a result, an organic light-emitting device according to an embodiment is completed.

Here, the metal for forming the cathode may be lithium (Li), magnesium (Mg), aluminum (Al), aluminum-lithium (Al-Li), calcium (Ca), magnesium-indium (Mg-In), magnesium-silver (Mg-Ag) or the like. A transmissive cathode using ITO or IZO may be used in order to obtain a top-emission type light-emitting device.

The material for the electron transport layer functions to stably transport electrons injected from the cathode and may be a well-known electron transport material. Examples of the well-known electron transport material include quinoline derivatives, especially, tris(8-quinolinolate)aluminum (Alq3), TAZ, BAlq, beryllium bis(benzoquinolin-10-olate: Bebq2) and oxadiazole derivatives (PBD, BMD, BND, etc.).

In addition, each of the organic layers may be formed by a monomolecular deposition or solution process. The deposition is a method of forming a thin film by evaporating a material for forming each layer through heating in the presence of a vacuum or low pressure and the solution process is a method of forming a thin film by mixing a material for forming each layer with a solvent and forming the thin film from the mixture through a method such as inkjet printing, roll-to-roll coating, screen printing, spray coating, dip coating, or spin coating.

In addition, the organic light-emitting device is used for a display or lighting system selected from flat panel displays, flexible displays, monochromatic or white flat panel lighting systems, and monochromatic or white flexible lighting systems.

Hereinafter, the present invention will be described in more detail with reference to preferred examples. However, it will be obvious to those skilled in the art that these examples are merely provided for illustration of the present invention, and should not be construed as limiting the scope of the present invention.

### Synthesis Example 1 : Synthesis of Formula 1

### Synthesis Example 1-(1) : Synthesis of 1-a

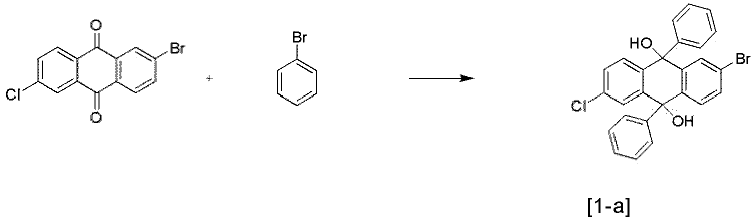

15.6 g (0.1 mol) of bromobenzene was added to a round bottom flask containing 150 mL of tetrahydrofuran under a nitrogen atmosphere, the mixture was cooled to -78°C, and then 58.3 mL (93 mmol) of 1.6 M N-butyllithium was slowly added thereto dropwise. The mixture was stirred at -78 ° C for 2 hours, and a dilution of 10 g (31 mmol) of 2-bromo-6-chloroanthraquinone in 100 mL of tetrahydrofuran was slowly added thereto dropwise. After the dropwise addition was completed, the mixture was allowed to warm to room temperature, followed by stirring for 3 hours. After completion of the reaction, the reaction product was subjected to acid treatment with a 2N HCl aqueous solution, extracted with water and ethyl acetate, and then concentrated to obtain [1-a] (yield 105%).

### Synthesis Example 1-(2) : Synthesis of 1-b

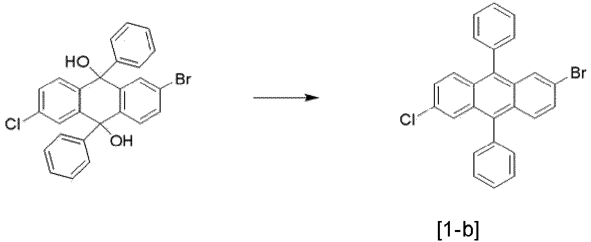

18.3 g of [1-a] was dissolved in acetic acid under a nitrogen atmosphere, and 5.49 g (93 mmol) of potassium iodide and 19.78 g (0.18 mol) of sodium hypophosphite were added thereto, followed by reflux for 2 hours. After completion of the reaction, the reaction product was filtered, thoroughly washed with water and methanol, extracted with methylene chloride and water, and concentrated. After concentration, the residue was separated by column chromatography to obtain [1-b] (yield 50%).

### Synthesis Example 1-(3): Synthesis of 1-c

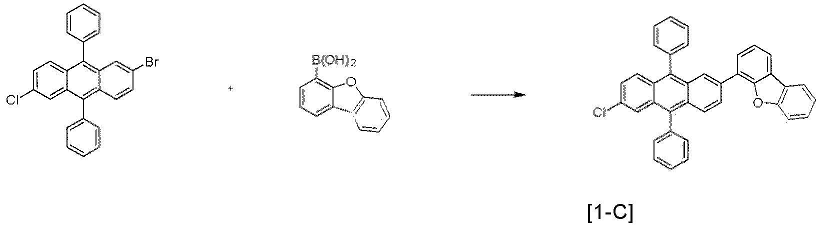

6.9 g (116 mmol) of [1-b], 3.1 g (15 mmol) of 4-dibenzofuran boronic acid, 2.6 g (19 mmol) of potassium carbonate, and 0.36 g (0 mmol) of tetrakis(triphenylphosphine) palladium were added to a round bottom flask, and 50 mL of toluene and 15 mL of water were added thereto, followed by reflux for 5 hours. After completion of the reaction, the reaction product was cooled to room temperature and 50 mL of methanol was added thereto, followed by stirring and filtering under reduced pressure. The solid was recrystallized from toluene to obtain [1-c] (yield 65%).

### Synthesis Example 1-(4): Synthesis of Formula 1

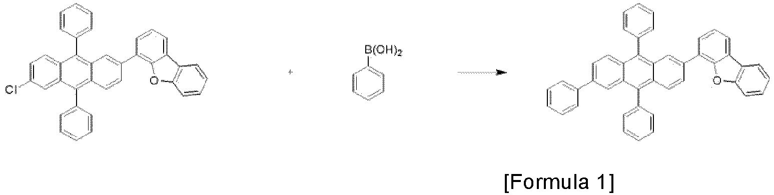

5.8 g (11 mmol) of [1-c], 1.6 g (13 mmol) of phenylboronic acid, 3.01 g (22 mmol) of potassium carbonate, 0.5 g of tetrakis(triphenylphosphine) palladium, and 50 mL of toluene were added to a round bottom flask, and 50 mL of 1,4-dioxane, and 15 mL of water were added thereto, followed by reflux for 12 hours. After completion of the reaction, the reaction product was cooled to room temperature, and 50 mL of methanol was added thereto, followed by stirring and filtering under reduced pressure. The solid was recrystallized from toluene and acetone to obtain [Formula 1] (yield 30%).
MS (MALDI-TOF) : m/z 572.21 [M+]

### Synthesis Example 2: Synthesis of Formula 3

### Synthesis Example 2-(1): Synthesis of 2-a

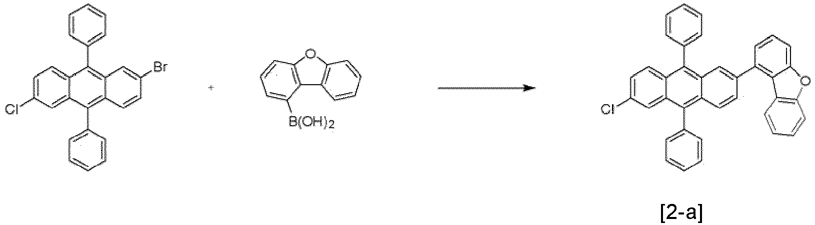

[2-a] was obtained by synthesis in the same manner as in Synthesis Example 1-3 except that 1-dibenzofuran boronic acid was used instead of 4-dibenzofuran boronic acid (yield 60%).

### Synthesis Example 2-(2): Synthesis of Formula 3

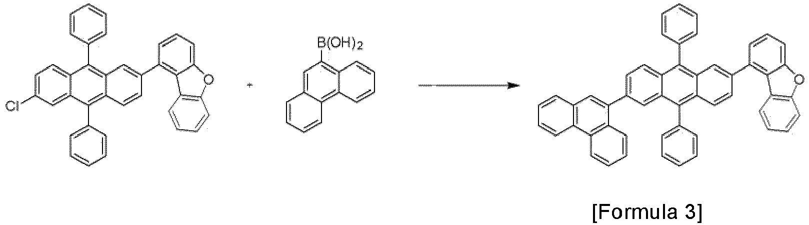

[Formula 3] was obtained by synthesis in the same manner as in Synthesis Example 1-4 except that 9-phenanthrene boronic acid was used instead of phenyl boronic acid and [2-a] was used instead of [1-c] (yield 38%).
MS (MALDI-TOF) : m/z 672.25 [M+]

### Synthesis Example 3: Synthesis of Formula 4

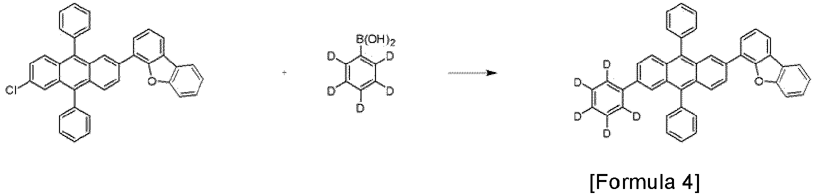

[Formula 4] was obtained by synthesis in the same manner as in Synthesis Example 1-4 except that phenyl-(d5)-boronic acid was used instead of phenyl boronic acid (yield 27%).
MS (MALDI-TOF) : m/z 577.25 [M+]

### Synthesis Example 4: Synthesis of Formula 9

### Synthesis Example 4-(1): Synthesis of 4-a

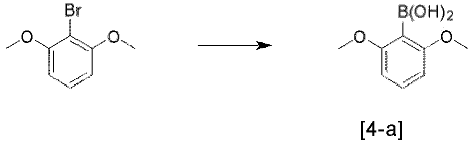

50 g (230 mmol) of 2-bromo-1,3-dimethoxybenzene was dissolved in 400 mL of tetrahydrofuran under a nitrogen atmosphere. The solution was cooled to -78°C and then 167 mL (280 mmol)

of 1.6 M N-butyllithium was slowly added thereto dropwise. The mixture was stirred at the same temperature for 2 hours, 36 mL (320 mmol) of trimethyl borate was added thereto, and the mixture was stirred at room temperature for 5 hours. After completion of the reaction, 2 N hydrochloric acid was slowly added dropwise to acidify the reaction product. After extraction with water and ethyl acetate, the extract was concentrated and recrystallized from heptane and toluene to obtain [4-a] (yield 50%).

### Synthesis Example 4-(2): Synthesis of 4-b

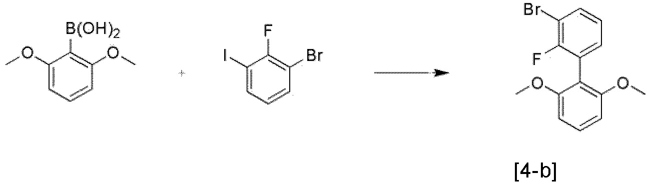

[4-b] was obtained by synthesis in the same manner as in Synthesis Example 1-4 except that [4-a] was used instead of phenyl boronic acid and 1-bromo-2-fluoro-3-iodobenzene was used instead of [1-c] (yield 63%).

### Synthesis Example 4-(3): Synthesis of 4-c

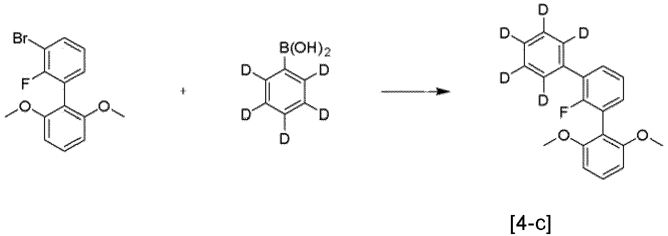

[4-c] was obtained by synthesis in the same manner as in Synthesis Example 1-4 except that phenyl-d5-boronic acid was used instead of phenyl boronic acid and [4-b] was used instead of [1-c] (yield 72%).

### Synthesis Example 4-(4): Synthesis of 4-d

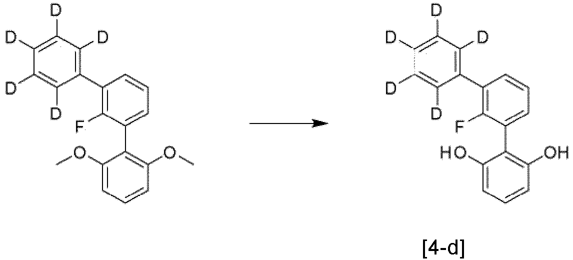

16.6 g (53 mmol) of [4-d], 48 mL (280 mmol) of hydrobromic acid, and 100 mL of acetic acid were added to a round-bottom flask, followed by stirring for 12 hours. After completion of the reaction, the reaction product was cooled to room temperature and water was poured thereto, followed by stirring. The reaction product was extracted with water and ethyl acetate, and the organic layer was separated. The organic layer was concentrated under reduced pressure and recrystallized from heptane to obtain [4-d] (yield 95%).

### Synthesis Example 4-(5): Synthesis of 4-e

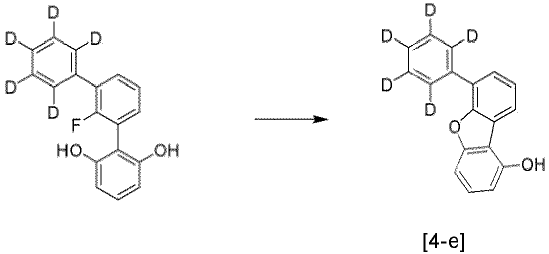

14.3 g (50 mmol) of [4-d], 20.7 g (150 mmol) of calcium carbonate, and 112 mL of N-methyl-2-pyrrolidone were added to a round-bottom flask, followed by stirring for 12 hours. After completion of the reaction, the reaction product was cooled to room temperature, and the organic layer was separated by extraction with water and ethyl acetate. The organic layer was concentrated under reduced pressure and then recrystallized from heptane to obtain [4-e] (yield 80%).

### Synthesis Example 4-(6): Synthesis of 4-f

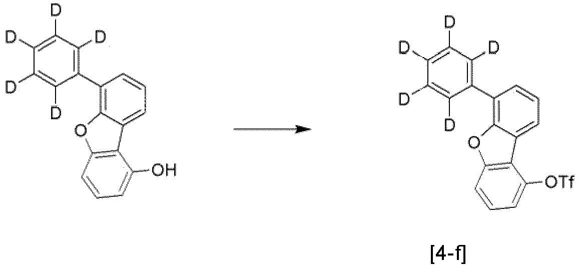

10 g (38 mmol) of [4-e], 3.9 mL (49 mmol) of pyridine, and 300 mL of methylene chloride were added under a nitrogen atmosphere, and the resulting solution was cooled to 0°C. Then, 11.7 g (41 mmol) of trifluoromethanesulfonic anhydride was slowly added thereto dropwise, followed by stirring for 1 hour. After completion of the reaction, the reaction product was extracted with 5°C distilled water and then recrystallized from methylene chloride and hexane to obtain [4-f] (yield 70%).

### Synthesis Example 4-(7): Synthesis of 4-q

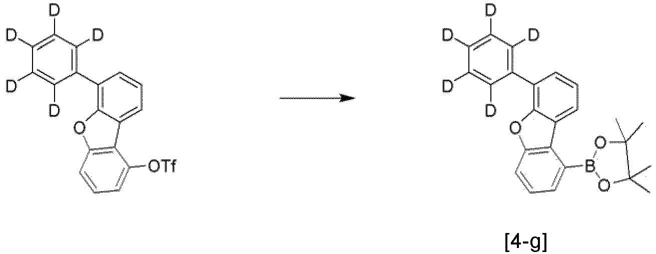

10.5 g (27 mmol) of [4-f], 10.1 g (40 mmol) of bis(pinacolato)diboron, 0.7 g (1 mmol) of palladium (II) chloride-1,1'-bis(diphenylphosphino)ferrocene, 7.8 g (80 mmol) of potassium acetate and 100 mL of toluene were stirred under reflux for 10 hours. After completion of the reaction, the reaction product was filtered under reduced pressure and the filtrate was concentrated and purified by column chromatography to obtain [4-f] (yield 64%).

### Synthesis Example 4-(8): Synthesis of 4-h

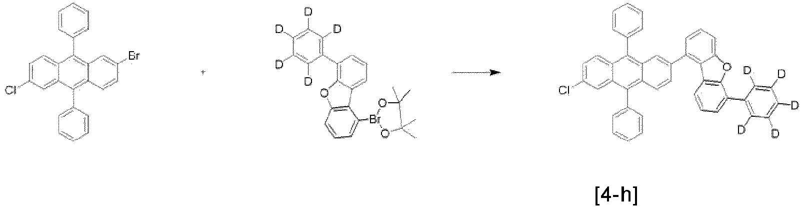

[4-h] was obtained by synthesis in the same manner as in Synthesis Example 1-3 except that [4-g] was used instead of 4-dibenzofuran boronic acid (yield 61%).
MS (MALDI-TOF) : m/z 611.21 [M+]

### Synthesis Example 4-(9): Synthesis of Formula 9

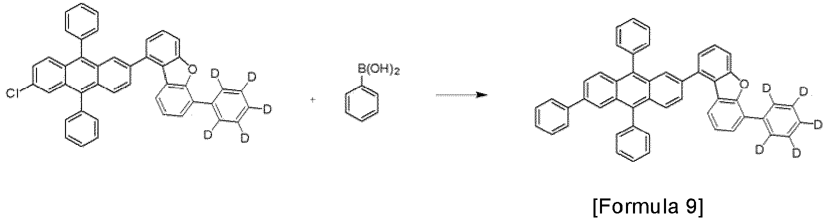

[Formula 9] was obtained by synthesis in the same manner as in Synthesis Example 1-4 except that [4-h] was used instead of [1-c] (yield 33%).
MS (MALDI-TOF): m/z 653.28 [M+]

### Synthesis Example 5: Synthesis of Formula 10

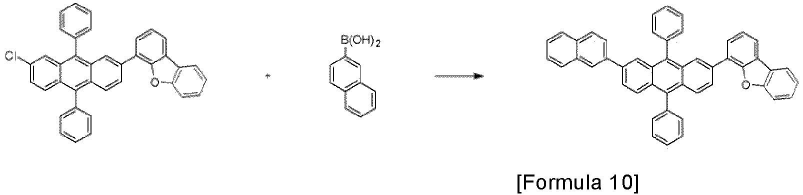

[Formula 10] was obtained by synthesis in the same manner as in Synthesis Examples 1-1 to 1-4 except that 2-bromo-7-chloro-anthraquinone was used instead of 2-bromo-6-chloroanthraquinone in Synthesis Example 1-1 and 2-naphthyl boronic acid was used instead of phenylboronic acid in Synthesis Example 1-4 (yield 36%).
MS (MALDI-TOF) : m/z 622.23 [M+]

### Synthesis Example 6: Synthesis of Formula 12

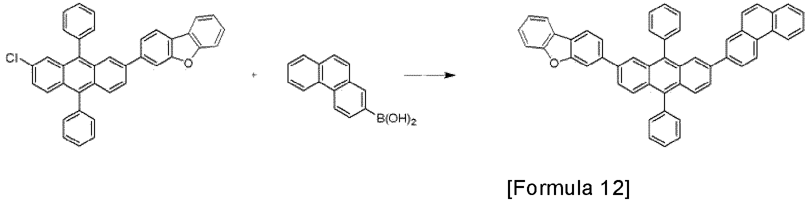

[Formula 12] was obtained by synthesis in the same manner as in Synthesis Examples 1-1 to 1-4 except that 2-bromo-7-chloro-anthraquinone was used instead of 2-bromo-6-chloroanthraquinone in Synthesis Example 1-1, 3-dibenzofuran boronic acid was used instead of 4-dibenzofuran boronic acid in Synthesis Example 1-3, and 2-phenanthrene boronic acid was used instead of phenylboronic acid in Synthesis Example 1-4 (yield 34%).
MS (MALDI-TOF): m/z 672.25 [M+]

### Synthesis Example 7: Synthesis of Formula 24

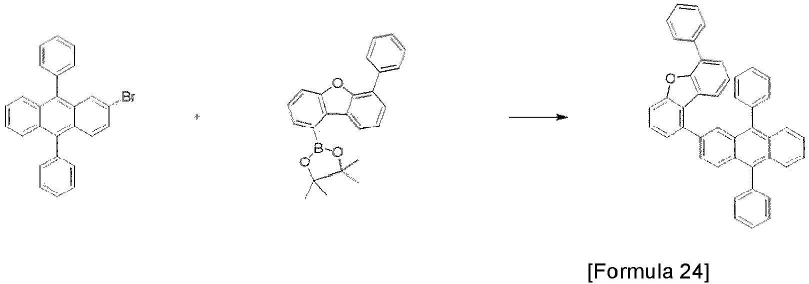

[Formula 24] was obtained by synthesis in the same manner as in Synthesis Examples 1-1 to 1-4 and 4-1 to 4-7 except that 2-bromo-anthraquinone was used instead of 2-bromo-6-chloroanthraquinone in Synthesis Example 1-1, and phenyl boronic acid was used instead of phenyl-(d5)-boronic acid in Synthesis Example 4-3 (yield 43%).
MS (MALDI-TOF): m/z 572.21 [M+]

### Synthesis Example 8: Synthesis of Formula 30

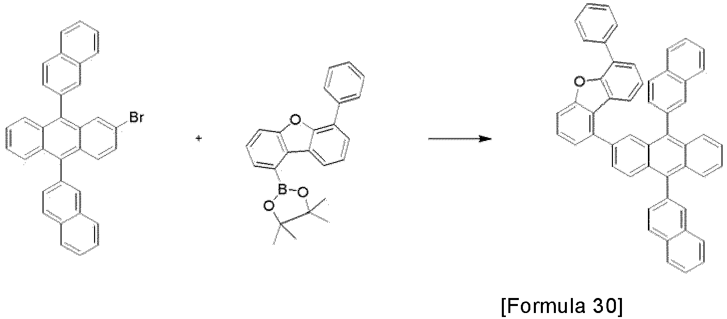

[Formula 30] was obtained by synthesis in the same manner as in Synthesis Examples 1-1 to 1-4, and 4-1 to 4-7, except that 2-bromoanthraquinone was used instead of 2-bromo-6-chloroanthraquinone and 1-bromonaphthalene was used instead of bromobenzene in Synthesis Example 1-1, and phenyl boronic acid was used instead of phenyl-(d5)-boronic acid in Synthesis Example 4-3 (yield 41%).
MS (MALDI-TOF) : m/z 672.25 [M+]

### Synthesis Example 9: Synthesis of Formula 31

### Synthesis Example 9-(1): Synthesis of 9-a

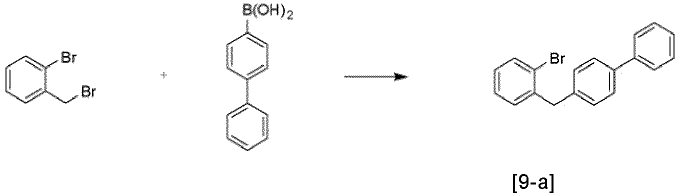

[9-a] was obtained by synthesis in the same manner except that bromobenzyl bromide was used instead of [1-c] and 4-biphenyl boronic acid was used instead of phenyl boronic acid in Synthesis Example 1-4 (yield 81%).

### Synthesis Example 9-(2): Synthesis of 9-b

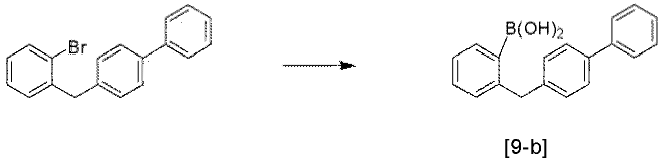

[9-b] was obtained by synthesis in the same manner except that [9-a] was used instead of 2-bromo-1,3-dimethoxybenzene used in Synthesis Example 4-1 (yield 75%).

### Synthesis Example 9-(3): Synthesis of 9-c

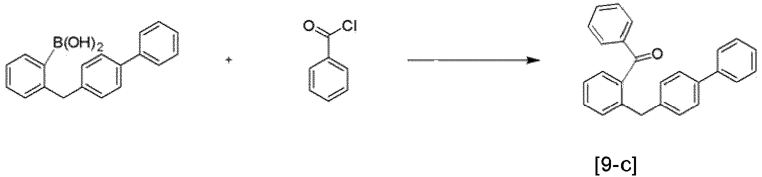

[9-c] was obtained by synthesis in the same manner as in Synthesis Example 1-4 except that benzoyl chloride was used instead of [1-c] and [9-b] was used instead of phenyl boronic acid (yield 52%).

### Synthesis Example 9-(4): Synthesis of 9-d

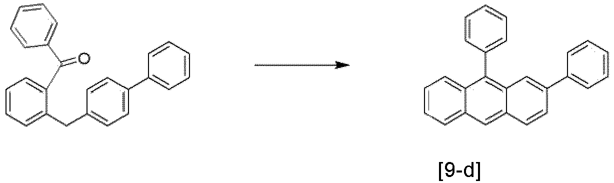

38 g (0.109 mol) of [9-d], 6 g (11 mmol) of indium (III) trifluoromethanesulfonate, and 190 mL of 1,2-dichloroethane were refluxed in a round-bottom flask for 12 hours. The reaction solution was extracted with methylene chloride and water, and the organic layer was concentrated and purified by column chromatography to obtain [9-d] (yield 82%).

### Synthesis Example 9-(5): Synthesis of 9-e

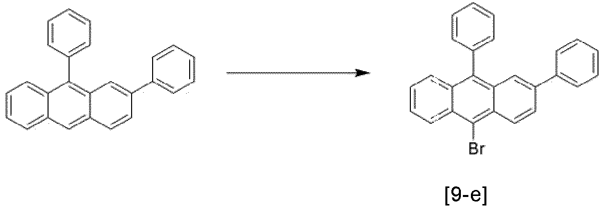

30 g (91 mmol) of [9-d] and 300 mL of methylene chloride were cooled to 0°C and stirred in a round-bottom flask. A dilution of 15 mL of bromine in 60 mL of methylene chloride was added slowly thereto. After completion of the reaction, the reaction solution was reprecipitated in an aqueous sodium thiosulfate solution and extracted with methylene chloride and water. The organic layer was concentrated and recrystallized from toluene to obtain [9-e] (yield 86%).

### Synthesis Example 9-(6): Synthesis of Formula 31

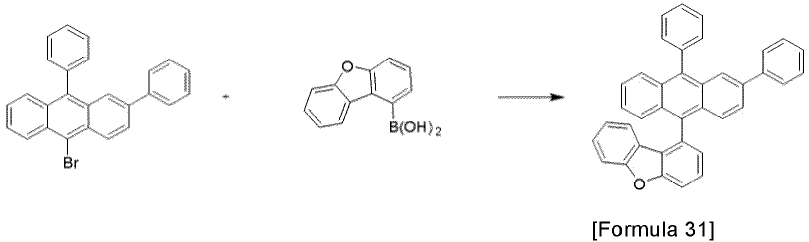

[Formula 31] was obtained by synthesis in the same manner as in Synthesis Example 1-4 except that [9-e] was used instead of [1-c] and 1-dibenzofuran boronic acid was used instead of phenyl boronic acid (yield 55%).
MS (MALDI-TOF) : m/z 496.18 [M+]

### Synthesis Example 10: Synthesis of Formula 34

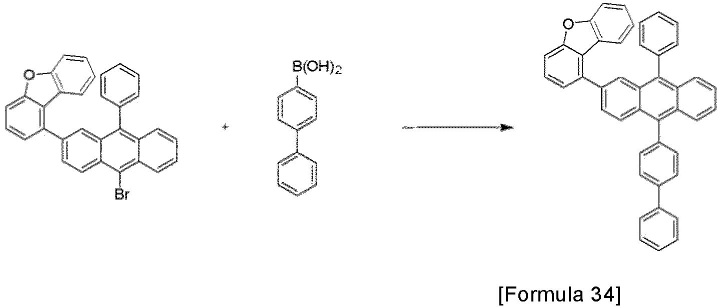

[Formula 34] was obtained by synthesis in the same manner as in Synthesis Examples 9-1 to 9-6, except that 1-phenyl-4-dibenzofuran boronic acid was used instead of 4-biphenyl boronic acid in Synthesis Example 9-1, and 4-biphenyl boronic acid was used instead of 1-dibenzofuran boronic acid in Synthesis Example 9-6 (yield 53%).
MS (MALDI-TOF) : m/z 572.21 [M+]

### Synthesis Example 11: Synthesis of Formula 40

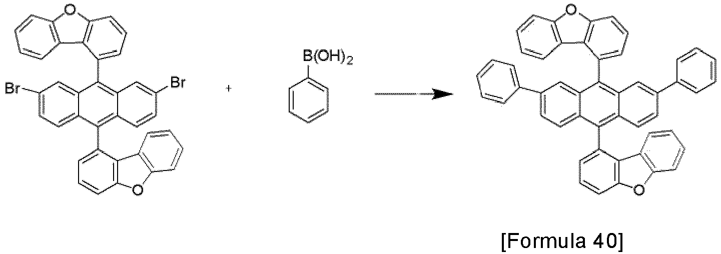

[Formula 40] was obtained by synthesis in the same manner as in Synthesis Examples 1-1 to 1-4 except that 2,7-dibromo-anthraquinone was used instead of 2-bromo-6-chloroanthraquinone and 1-bromodibenzofuran was used instead of bromobenzene in Synthesis Example 1-1 (yield 48%).
MS (MALDI-TOF): m/z 662.22 [M+]

### Synthesis Example 12: Synthesis of Formula 41

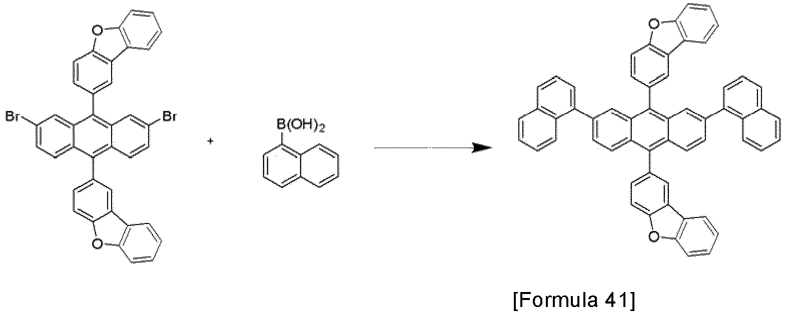

[Formula 41] was obtained by synthesis in the same manner as in Synthesis Examples 1-1 to 1-4, except that 2,7-dibromo-anthraquinone was used instead of 2-bromo-6-chloroanthraquinone and 2-bromodibenzofuran was used instead of bromobenzene in Synthesis Example 1-1 and 1-naphthyl boronic acid was used instead of phenyl boronic acid in Synthesis Example 1-4 (yield 42%).
MS (MALDI-TOF): m/z 762.26 [M+]

### Synthesis Example 13: Synthesis of Formula 45

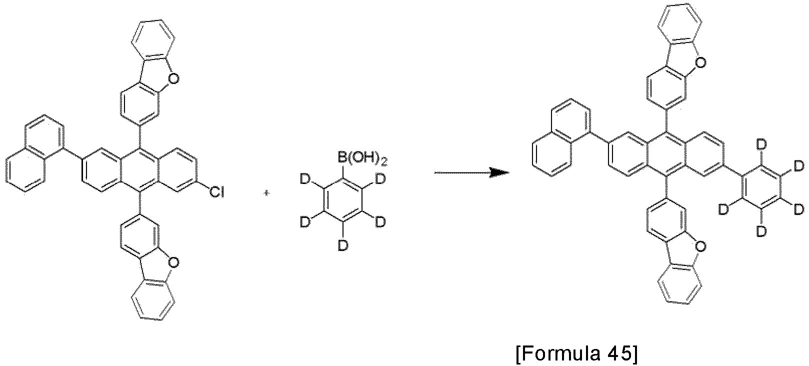

[Formula 45] was obtained by synthesis in the same manner as in Synthesis Examples 1-1 to 1-4 except that 3-bromodibenzofuran was used instead of bromobenzene in Synthesis Example 1-1, 1-naphthyl boronic acid was used instead of 4-dibenzofuran boronic acid in Synthesis Example 1-3, and phenyl-(d5)-boronic acid was used instead of phenyl boronic acid in Synthesis Example 1-4 (yield 33%).
MS (MALDI-TOF): m/z 717.27 [M+]

### Synthesis Example 14: Synthesis of Formula 47

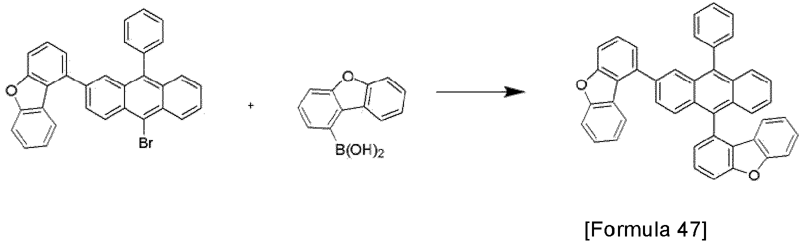

[Formula 47] was obtained by synthesis in the same manner as in Synthesis Examples 9-1 to 9-6, except that 4-phenyl-1-dibenzofuran boronic acid was used instead of 4-biphenyl boronic acid in Synthesis Example 9-1 (yield 41%).
MS (MALDI-TOF): m/z 586.19 [M+]

### Synthesis Example 15: Synthesis of Formula 50

### Synthesis Example 15-(1): Synthesis of 15-a

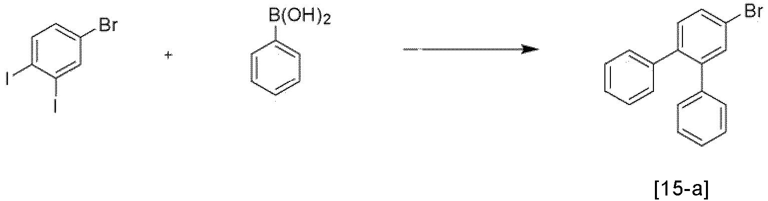

[15-a] was obtained by synthesis in the same manner except that 4-bromo-1,2-diiodobenzene was used instead of [1-c] in Synthesis Example 1-4 (yield 73%).

### Synthesis Example 15-(2): Synthesis of 15-b

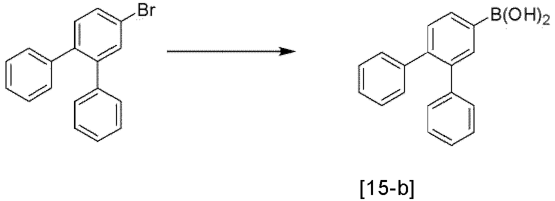

[15-b] was obtained by synthesis in the same manner as in Synthesis Example 4-1 except that [15-a] was used instead of 2-bromo-1,3-dimethoxybenzene (yield 70%).

### Synthesis Example 15-(3): Synthesis of Formula 50

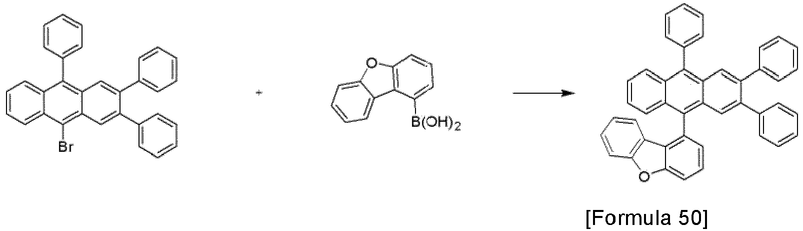

[Formula 50] was obtained by synthesis in the same manner as in Synthesis Examples 9-1 to 9-6, except that [15-b] was used instead of 4-biphenyl boronic acid in Synthesis Example 9-1 (yield 47%).
MS (MALDI-TOF): m/z 572.21 [M+]

### Synthesis Example 16: Synthesis of Formula 58

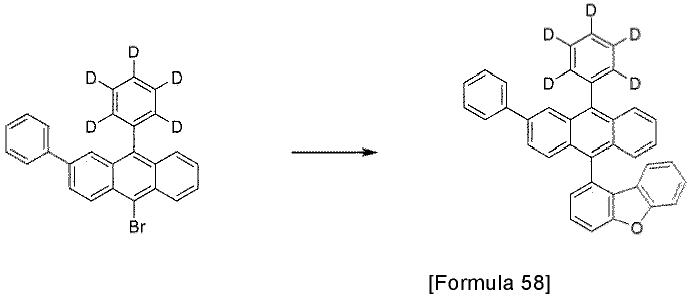

[Formula 58] was obtained by synthesis in the same manner as in Synthesis Examples 9-1 to 9-6 except that benzoyl (d5) chloride was used instead of benzoyl chloride in Synthesis Example 9-3 (yield 42%).
MS (MALDI-TOF): m/z 501.21 [M+]

### Synthesis Example 17: Synthesis of Formula 64

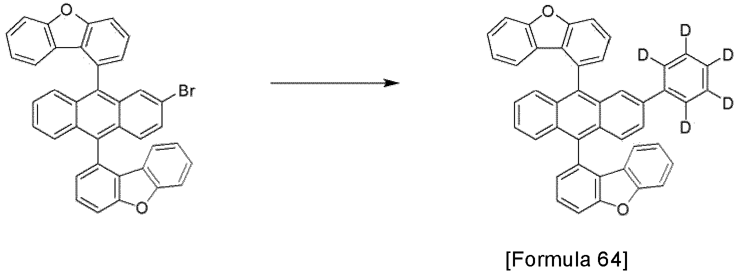

[Formula 64] was obtained by synthesis in the same manner as in Synthesis Examples 1-1 to 1-4 except that 2-bromo-anthraquinone was used instead of 2-bromo-6-chloro-anthraquinone and 1-dibenzofuran boronic acid was used instead of bromobenzene in Synthesis Example 1-1, and phenyl-(d5)-boronic acid was used instead of phenyl boronic acid in Synthesis Example 1-4 (yield 38%).
MS (MALDI-TOF): m/z 591.22 [M+]

### Synthesis Example 18: Synthesis of Formula 70

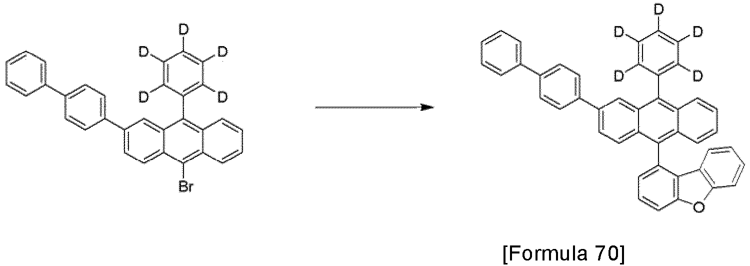

[Formula 70] was synthesized in the same manner as in Synthesis Examples 9-1 to 9-6, except that 4-triphenyl boronic acid was used instead of 4-biphenyl boronic acid in Synthesis Example 9-1, and benzoyl (d5) chloride was used instead of benzoyl chloride in Synthesis Example 9-3 (yield 41%).
MS (MALDI-TOF): m/z 577.25 [M+]

### Synthesis Example 19: Synthesis of Formula 73

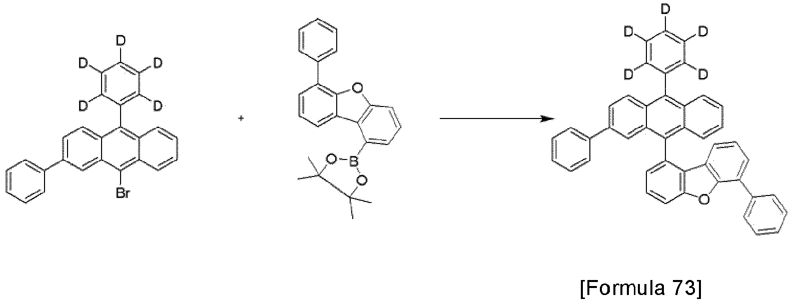

[Formula 73] was synthesized in the same manner as in Synthesis Examples 9-1 to 9-6 and 4-1 to 4-6, except that 3-biphenyl boronic acid was used instead of 4-biphenyl boronic acid in Synthesis Example 9-1, benzoyl (d5) chloride was used instead of benzoyl chloride in Synthesis Example 9-3, and phenyl boronic acid was used instead of phenyl-(d5)-boronic acid in Synthesis Example 4-3 (yield 38%).
MS (MALDI-TOF): m/z 577.25 [M+]

### Synthesis Example 20: Synthesis of Formula 79

### Synthesis Example 20-(1): Synthesis of 79-a

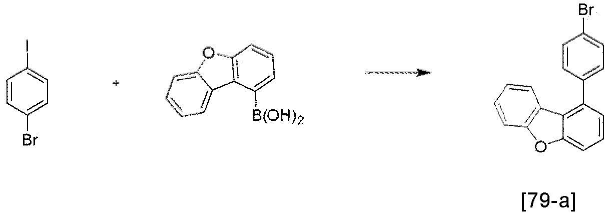

[79-a] was obtained by synthesis in the same manner except that 1,4-iodobromobenzene was used instead of [1-c] and 1-dibenzofuran boronic acid was used instead of phenyl boronic acid in Synthesis Example 1-4 (yield 65%).

### Synthesis Example 20-(2): Synthesis of 79-b

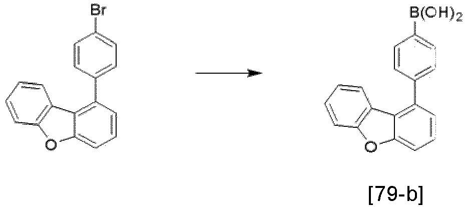

[79-b] was obtained by synthesis in the same manner as in Synthesis Example 4-1 except that [79-a] was used instead of 2-bromo-1,3-dimethoxybenzene (yield 59%).

### Synthesis Example 20-(3): Synthesis of Compound 79

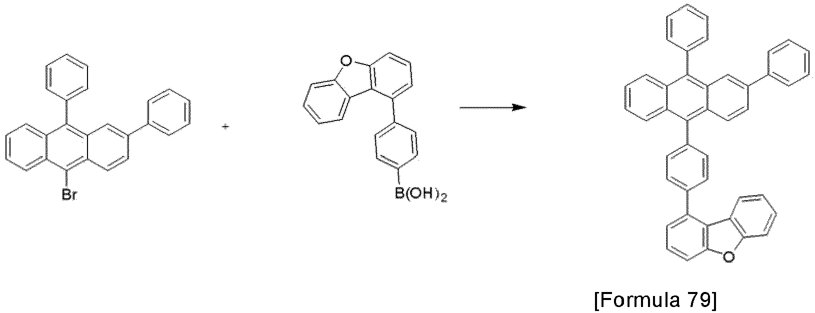

[Formula 79] was obtained by synthesis in the same manner except that [9-e] was used instead of [1-c] and 2-dibenzofuran boronic acid was used instead of phenyl boronic acid in Synthesis Example 1-4 (yield 49%).
MS (MALDI-TOF): m/z 572.21 [M+]

### Synthesis Example 21: Synthesis of compound 81

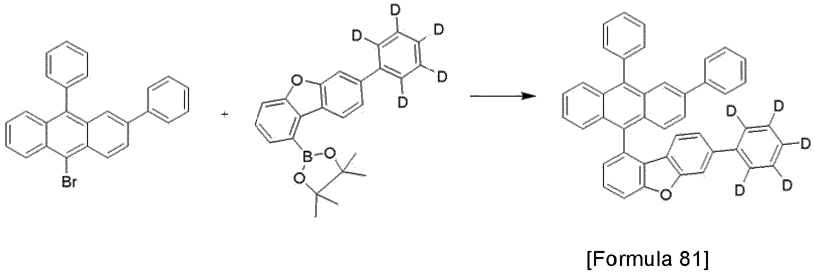

[Formula 81] was obtained by synthesis in the same manner as in Synthesis Examples 9-1 to 9-6 and 4-1 to 4-6 except that [9-e] was used instead of [1-c] and 1-bromo-3-fluoro-4-iodobenzene was used instead of 1-bromo-2-fluoro-3-iodobenzene in Synthesis Example 1-4 (yield 45%).
MS (MALDI-TOF): m/z 577.25 [M+]

### Synthesis Example 22: Synthesis of Formula 82

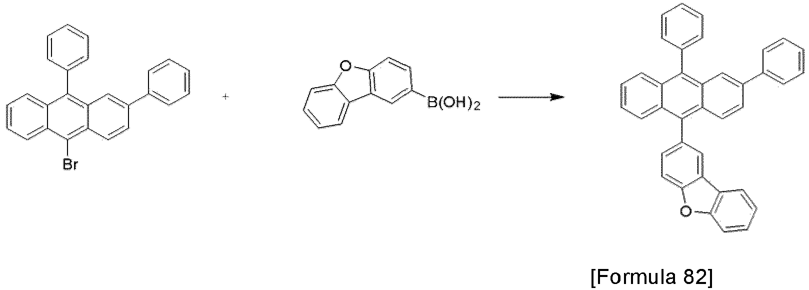

[Formula 82] was obtained by synthesis in the same manner as in Synthesis Examples 9-1 to 9-6, except that [9-e] was used instead of [1-c] and 2-dibenzofuran boronic acid was used instead of phenyl boronic acid in Synthesis Example 1-4 (yield 57%).
MS (MALDI-TOF): m/z 496.18 [M+]

### Synthesis Example 23: Synthesis of Formula 83

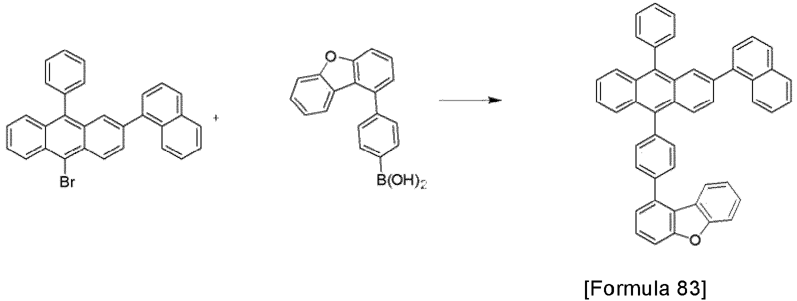

[Formula 83] was obtained by synthesis in the same manner as in Synthesis Examples 9-1 to 9-6, except that (4-(1-naphthyl)phenyl)boronic acid was used instead of 4-biphenyl boronic acid in Synthesis Example 9-1, and [79-b] was used instead of 1-dibenzofuran boronic acid in Synthesis Example 9-6 (yield 57%).
MS (MALDI-TOF): m/z 622.23 [M+]

### Synthesis Example 24: Synthesis of Formula 84

### Synthesis Example 24-(1): Synthesis of 84-a

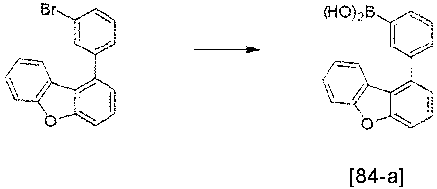

[84-a] was obtained by synthesis in the same manner as in Synthesis Examples 20-1 to 20-2 except that 1,3-bromoiodobenzene was used instead of 1,4-bromoiodobenzene in Synthesis Example 20-1 (yield 59%).

### Synthesis Example 24-(2): Synthesis of Formula 84

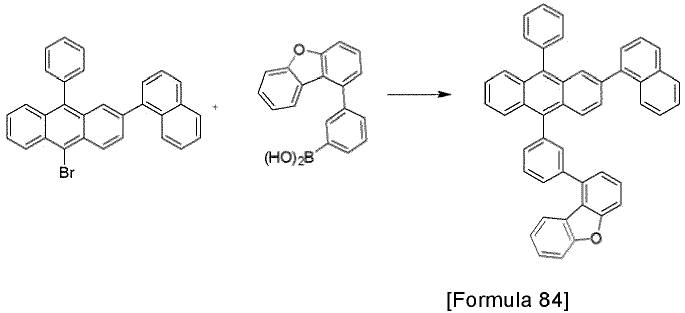

[Formula 84] was obtained by synthesis in the same manner as in Synthesis Examples 9-1 to 9-6 except that (4-(1-naphthyl)phenyl)boronic acid was used instead of 4-biphenyl boronic acid in Synthesis Example 9-1 and [84-a] was used instead of 1-dibenzofuran boronic acid in Synthesis Example 9-6 (yield 64%).
MS (MALDI-TOF): m/z 622.23 [M+]

### Synthesis Example 25: Synthesis of Formula 85

### Synthesis Example 25-(1): Synthesis of 85-a

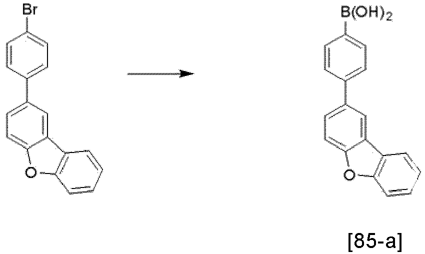

[85-a] was obtained by synthesis in the same manner as in Synthesis Examples 20-1 to 20-2 except that 2-dibenzoboronic acid was used instead of 1-dibenzofuran boronic acid in Synthesis Example 20-1 (yield 65%).

### Synthesis Example 25-(2): Synthesis of Formula 85

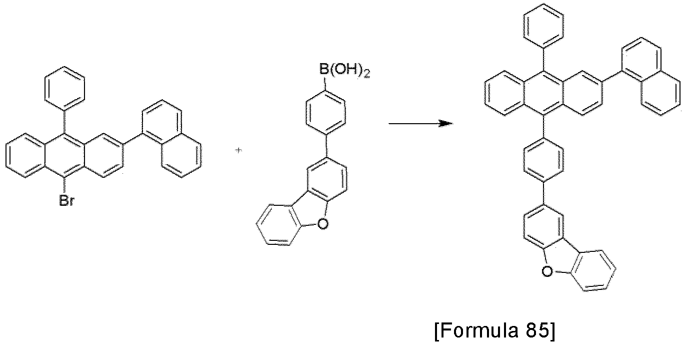

[Formula 85] was obtained by synthesis in the same manner as in Synthesis Examples 9-1 to 9-6 except that (4-(1-naphthyl)phenyl)boronic acid was used instead of 4-biphenyl boronic acid in Synthesis Example 9-1, and [85-a] was used instead of 1-dibenzofuran boronic acid in Synthesis Example 9-6 (yield 49%).
MS (MALDI-TOF): m/z 622.23 [M+]

### Synthesis Example 26: Synthesis of Formula 86

### Synthesis Example 26-(1): Synthesis of 86-a

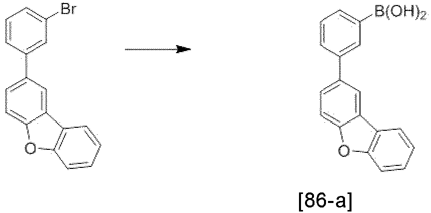

[86-a] was obtained by synthesis in the same manner as in Synthesis Examples 25-1 to 25-2 except that 1,3-bromoiodobenzene was used instead of 1-1,4-bromoiodobenzene in Synthesis Example 25-1 (yield 71%).

### Synthesis Example 26-(2): Synthesis of Formula 86

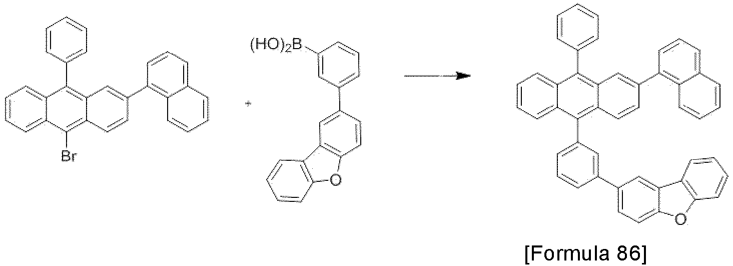

[Formula 86] was obtained by synthesis in the same manner as in Synthesis Examples 9-1 to 9-6, except that (4-(1-naphthyl)phenyl)boronic acid was used instead of 4-biphenyl boronic acid in Synthesis Example 9-1 and [86-a] was used instead of 1-dibenzofuran boronic acid in Synthesis Example 9-6 (yield 38%).
MS (MALDI-TOF): m/z 622.23 [M+]

### Synthesis Example 27: Synthesis of Formula 87

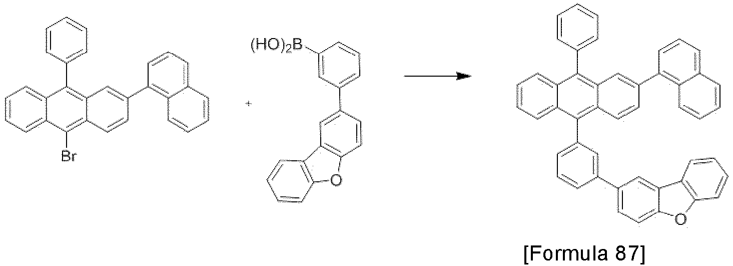

[Formula 87] was obtained by synthesis in the same manner as in Synthesis Examples 9-1 to 9-6, except that [9-e] was used instead of [1-c] and [86-a] was used instead of phenylboronic acid in Synthesis Example 1-4 (yield 43%).
MS (MALDI-TOF): m/z 572.21 [M+]

### Synthesis Example 28: Synthesis of Formula 88

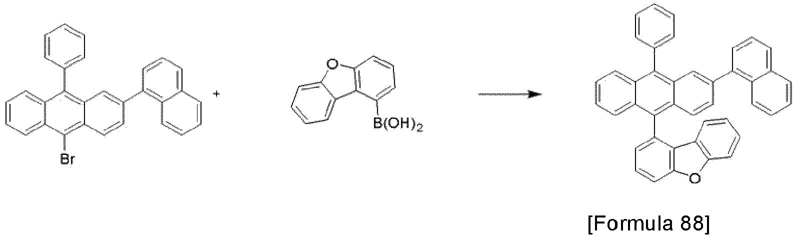

[Formula 88] was obtained in the same manner as in Synthesis Examples 9-1 to 9-6, except that (4-(1-naphthyl)phenyl)boronic acid was used instead of 4-biphenyl boronic acid in Synthesis Example 9-1 (yield 62%).
MS (MALDI-TOF): m/z 546.20 [M+]

### Synthesis Example 29: Synthesis of Formula 89

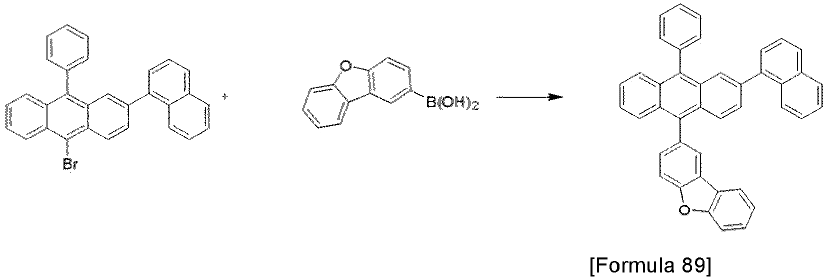

[Formula 89] was obtained by synthesis in the same manner as in Synthesis Examples 9-1 to 9-6 except that (4-(1-naphthyl)phenyl)boronic acid was used instead of 4-biphenyl boronic acid in Synthesis Example 9-1, and 2-dibenzofuran boronic acid was used instead of 1-dibenzofuran boronic acid in Synthesis Example 9-6 (yield 51%).
MS (MALDI-TOF): m/z 546.20 [M+]

### Synthesis Example 30: Synthesis of Formula 90

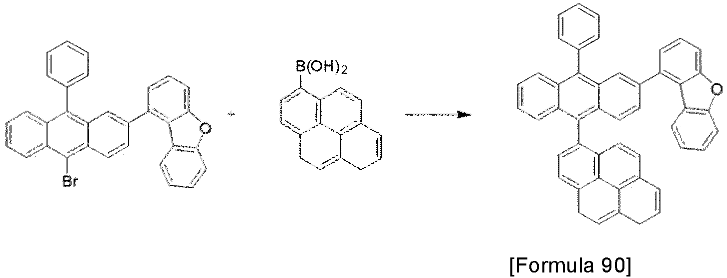

[Formula 90] was obtained by synthesis in the same manner as Synthesis Examples 9-1 to 9-6, except that 4-phenyl-1-dibenzofuran boronic acid was used instead of 4-biphenyl boronic acid in Synthesis Example 9-1, and 1-pyrene boronic acid was used instead of 1-dibenzofuran boronic acid in Synthesis Example 9-6 (yield 38%).
MS (MALDI-TOF): m/z 622.23 [M+]

### Synthesis Example 31: Synthesis of Formula 91

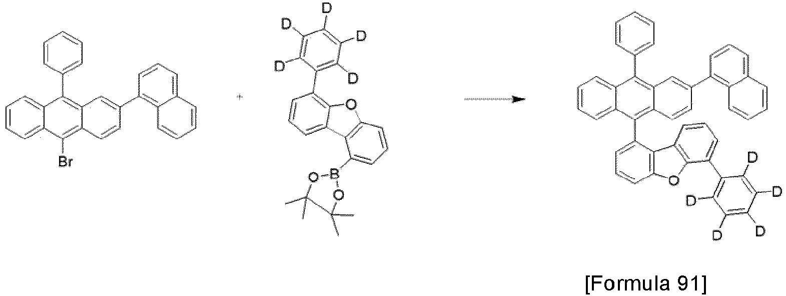

[Formula 91] was obtained by synthesis in the same manner as in Synthesis Examples 9-1 to 9-6, except that (4-(1-naphthyl)phenyl)boronic acid was used instead of 4-biphenyl boronic acid in Synthesis Example 9-1, and [4-g] was used instead of 1-dibenzofuran boronic acid in Synthesis Example 9-6 (yield 51%).
MS (MALDI-TOF): m/z 627.26 [M+]

### Synthesis Example 32: Synthesis of Formula 92

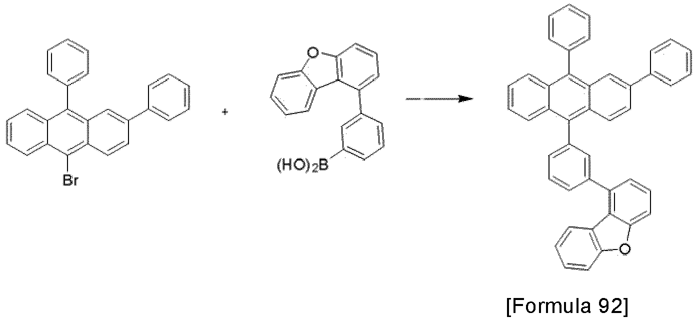

[Formula 92] was obtained by synthesis in the same manner as in Synthesis Examples 9-1 to 9-6, except that [9-e] was used instead of [1-c] and [84-a] was used instead of phenylboronic acid in Synthesis Example 1-4 (yield 44%).
MS (MALDI-TOF): m/z 572.21 [M+]

### Synthesis Example 33: Synthesis of Formula 93

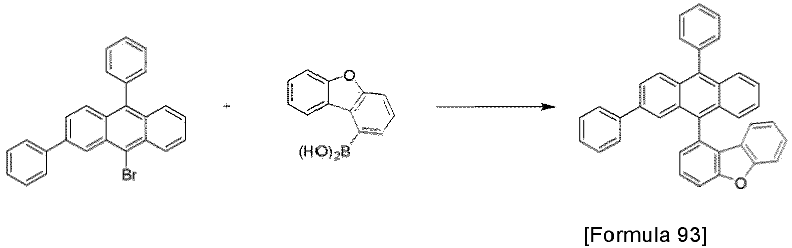

[Formula 93] was obtained by synthesis in the same manner as in Synthesis Examples 9-1 to 9-6 except that 3-biphenyl boronic acid was used instead of 4-biphenyl boronic acid in Synthesis Example 9-1, and phenyl boronic acid was used instead of phenyl-(d5)-boronic acid in Synthesis Example 4-3 (yield 43%).
MS (MALDI-TOF): m/z 496.18 [M+]

### Synthesis Example 34: Synthesis of Formula 94

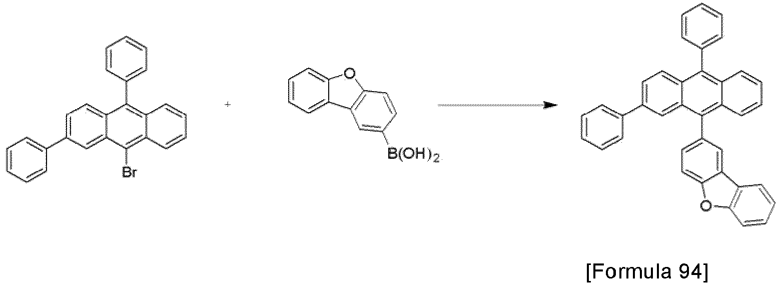

[Formula 94] was obtained by synthesis in the same manner as in Synthesis Examples 9-1 to 9-6 except that 3-biphenyl boronic acid was used instead of 4-biphenyl boronic acid in Synthesis Example 9-1, and phenyl boronic acid was used instead of phenyl-(d5)-boronic acid in Synthesis Example 4-3 (yield 40%).
MS (MALDI-TOF): m/z 496.18 [M+]

### Synthesis Example 35: Synthesis of Formula 95

### Synthesis Example 35-(1): Synthesis of 95-a

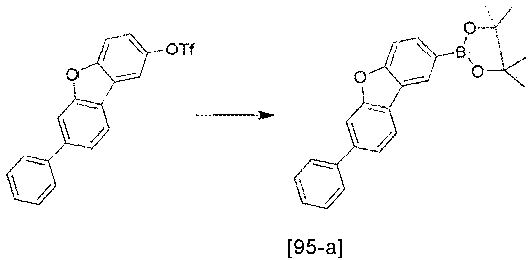

[95-a] was obtained by synthesis in the same manner as in Synthesis Examples 4-1 to 4-7 except that 2-bromo-1,4-dimethoxybenzene was used instead of 2-bromo-1,3-dimethoxybenzene in Synthesis Example 4-1, and phenyl boronic acid was used instead of phenyl-(d5)-boronic acid in Synthesis Example 4-3 (yield 71%).

### Synthesis Example 35-(2): Synthesis of Formula 95

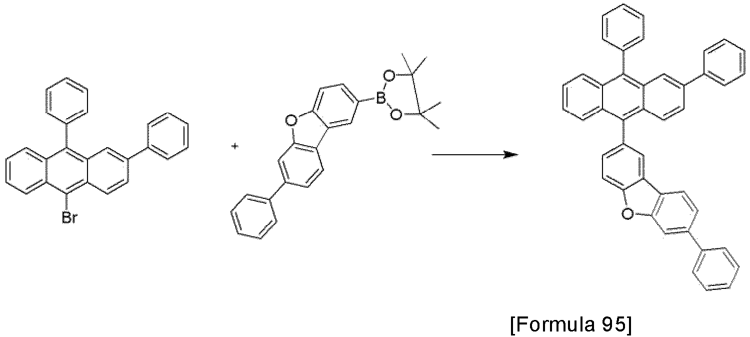

[Formula 95] was obtained by synthesis in the same manner as in Synthesis Examples 9-1 to 9-6 except that [9-e] was used instead of [1-c] and [95-a] was used instead of phenylboronic acid in Synthesis Example 1-4 (yield 52%).
MS (MALDI-TOF): m/z 572.21 [M+]

### Synthesis Example 36: Synthesis of Formula 96

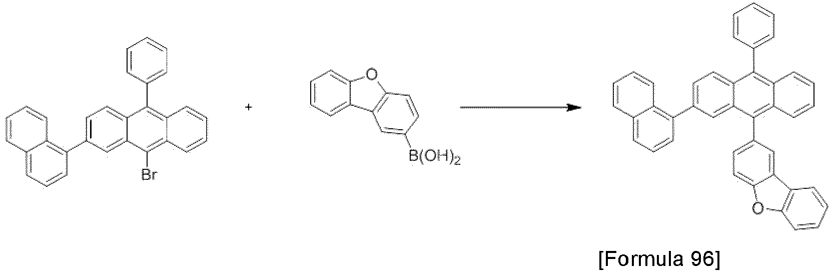

[Formula 96] was obtained by synthesis in the same manner as in Synthesis Examples 9-1 to 9-6 except that (3-(1-naphthyl)phenyl)boronic acid was used instead of 4-biphenyl boronic acid in Synthesis Example 9-1, and 2-dibenzofuran boronic acid was used instead of phenyl-(d5)-boronic acid in Synthesis Example 4-3 (yield 39%).
MS (MALDI-TOF): m/z 546.20 [M+]

### Synthesis Example 37: Synthesis of Formula 97

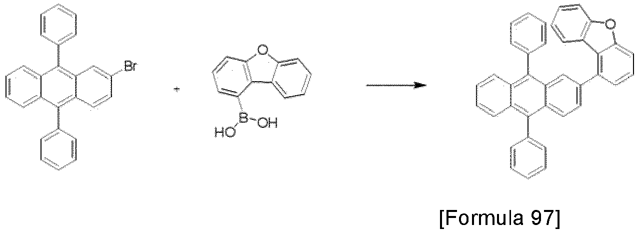

[Formula 97] was obtained by synthesis in the same manner as in Synthesis Examples 1-1 to 1-4 except that 2-bromo-anthraquinone was used instead of 2-bromo-6-chloro-anthraquinone in Synthesis Example 1-1, and 1-dibenzofuran boronic acid was used instead of phenylboronic acid in Synthesis Example 1-4 (yield 68%).
MS (MALDI-TOF): m/z 496.18 [M+]

### Synthesis Example 38: Synthesis of Formula 98

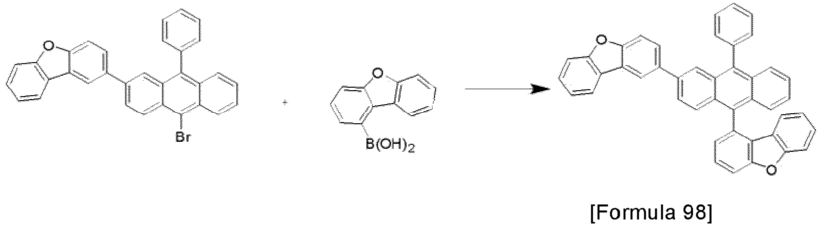

[Formula 98] was obtained by synthesis in the same manner as in Synthesis Examples 9-1 to 9-6, except that 4-phenyl-2-dibenzofuran boronic acid was used instead of 4-biphenyl boronic acid in Synthesis Example 9-1 (yield 51%).
MS (MALDI-TOF): m/z 586.19 [M+]

### Synthesis Example 39: Synthesis of Formula 99

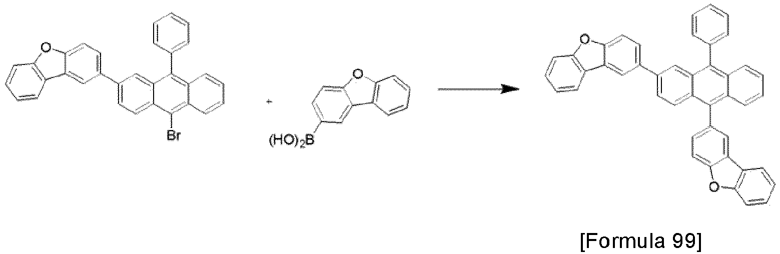

[Formula 99] was obtained by synthesis in the same manner as in Synthesis Examples 9-1 to 9-6, except that 4-phenyl-2-dibenzofuran boronic acid was used instead of 4-biphenyl boronic acid in Synthesis Example 9-1 and 2-dibenzofuran dibenzoic acid was used instead of 1-dibenzofuran boronic acid in Synthesis Example 9-6 (yield 56%).
MS (MALDI-TOF): m/z 586.19 [M+]

### Synthesis Example 40: Synthesis of Formula 100

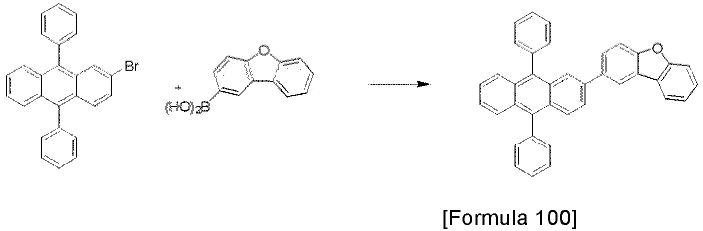

[Formula 100] was obtained by synthesis in the same manner as in Synthesis Examples 1-1 to 1-4 except that 2-bromo-anthraquinone was used instead of 2-bromo-6-chloro-anthraquinone in Synthesis Example 1-1, and 2-dibenzofuran boronic acid was used instead of phenylboronic acid in Synthesis Example 1-4 (yield 63%).
MS (MALDI-TOF): m/z 496.18 [M+]

### Synthesis Example 41: Synthesis of Formula 101

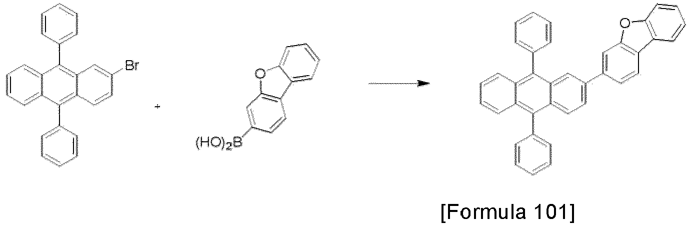

[Formula 101] was obtained by synthesis in the same manner as in Synthesis Examples 1-1 to 1-4 except that 2-bromo-anthraquinone was used instead of 2-bromo-6-chloro-anthraquinone in Synthesis Example 1-1, and 3-dibenzofuran boronic acid was used instead of phenylboronic acid in Synthesis Example 1-4 (yield 53%).
MS (MALDI-TOF): m/z 496.18 [M+]

### Synthesis Example 42: Synthesis of Formula 104

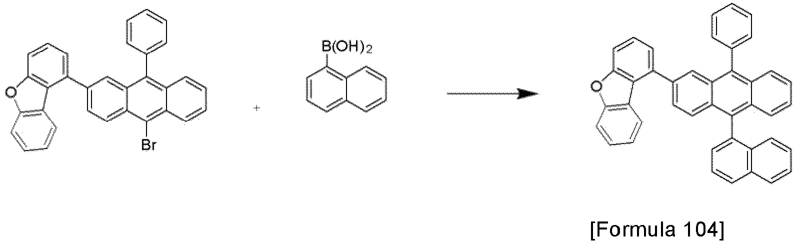

[Formula 104] was obtained by synthesis in the same manner as in Synthesis Examples 9-1 to 9-6, except that 4-phenyl-1-dibenzofuran boronic acid was used instead of 4-biphenyl boronic acid in Synthesis Example 9-1, and 1-naphthyl boronic acid was used instead of 1-dibenzofuran boronic acid in Synthesis Example 9-6 (yield 60%).
MS (MALDI-TOF): m/z 546.20 [M+]

### Synthesis Example 43: Synthesis of Formula 105

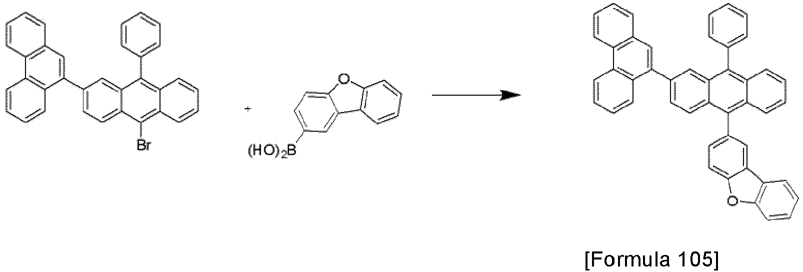

[Formula 105] was obtained by synthesis in the same manner as in Synthesis Examples 9-1 to 9-6 except that 4-phenyl-9-phenanthrene boronic acid was used instead of 4-biphenyl boronic acid in Synthesis Example 9-1, and 2-dibenzofuran was used instead of 1-dibenzofuran boronic acid in Synthesis Example 9-6 (yield 58%).
MS (MALDI-TOF): m/z 596.21 [M+]

### Synthesis Example 44: Synthesis of Formula 107

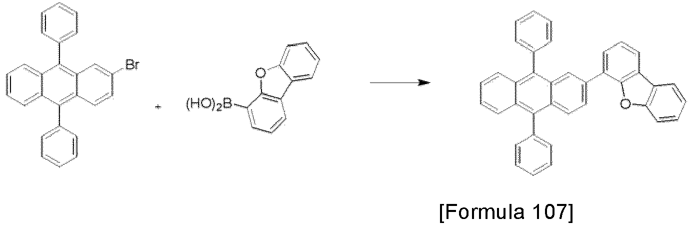

[Formula 107] was obtained by synthesis in the same manner as in Synthesis Examples 1-1 to 1-4 except that 2-bromo-anthraquinone was used instead of 2-bromo-6-chloro-anthraquinone in Synthesis Example 1-1, and 4-dibenzofuran boronic acid was used instead of phenylboronic acid in Synthesis Example 1-4 (yield 52%).
MS (MALDI-TOF): m/z 496.18 [M+]

### Synthesis Example 45: Synthesis of Formula 108

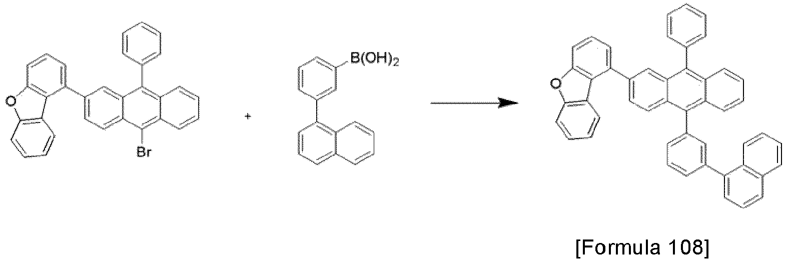

[Formula 108] was obtained by synthesis in the same manner as in Synthesis Examples 9-1 to 9-6, except that 4-phenyl-1-dibenzofuran boronic acid was used instead of 4-biphenyl boronic acid in Synthesis Example 9-1, and 3-phenyl-1-dibenzoic acid was used instead of 1-dibenzofuran boronic acid in Synthesis Example 9-6 (yield 48%).
MS (MALDI-TOF): m/z 622.23 [M+]

### Synthesis Example 46: Synthesis of Formula 110

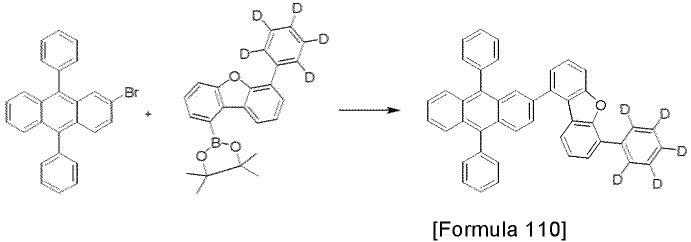

[Formula 110] was obtained by synthesis in the same manner as in Synthesis Examples 1-1 to 1-4 except that 2-bromo-anthraquinone was used instead of 2-bromo-6-chloro-anthraquinone in Synthesis Example 1-1, and [4-g] was used instead of phenylboronic acid in Synthesis Example 1-4 (yield 52%).
MS (MALDI-TOF): m/z 577.25 [M+]

### Synthesis Example 47: Synthesis of Formula 111

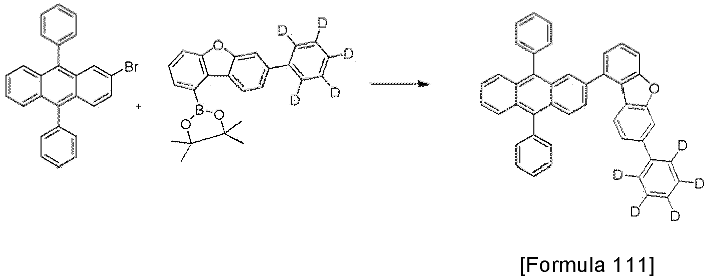

[Formula 111] was obtained by synthesis in the same manner as in Synthesis Examples 1-1 to 1-4 and 4-1 to 4-6 except that 2-bromo-anthraquinone was used instead of 2-bromo-6-chloroanthraquinone in Synthesis Example 1-1, and 1-bromo-2-fluoro-3-iodobenzene was used instead of 1-bromo-3-fluoro-4-iodobenzene (yield 52%).
MS (MALDI-TOF): m/z 577.25 [M+]

### <Synthesis of Formula A-1>

### Synthesis Example A1. Synthesis of A1

### Synthesis Example A1-1. Synthesis of A1-a

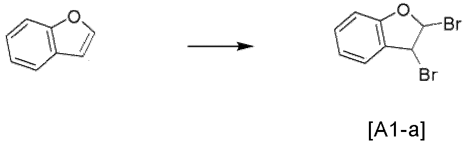

50 g (423 mmol) of benzofuran and 500 mL of dichloromethane were added to a 1 L reactor, followed by stirring. The reaction product was cooled to -10°C, and a dilution of 67.7 g (423 mmol) of bromine in 100 mL of dichloromethane was added dropwise thereto, followed by stirring at 0°C for 2 hours. After completion of the reaction, an aqueous sodium thiosulfate solution was added thereto, and the mixture was stirred, extracted with ethyl acetate and H₂O, and recrystallized from ethanol to obtain 100 g of [A1-a] (yield 93%).

### Synthesis Example A1-2. Synthesis of A1-b

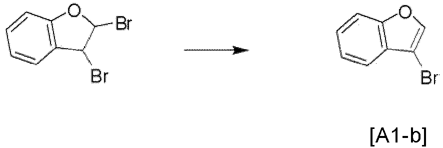

48.6 g (866 mmol) of potassium hydroxide was dissolved in 400 mL of ethanol in a 1 L reactor. A solution of 120 g (433 mmol) of [A1-a] in ethanol was added dropwise at 0°C, followed by stirring under reflux for 2 hours. After completion of the reaction, the reaction product was concentrated under reduced pressure and extracted with ethyl acetate and water, and the organic layer was concentrated and separated by column chromatography to obtain 42 g of [A1-b] (yield 50%).

### Synthesis Example A1-3. Synthesis of A1-c

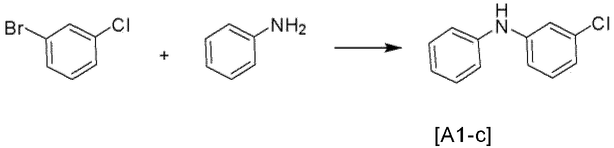

4.5 g (16 mmol) of 1-bromo-3-chlorobenzene, 5.8 g (16 mmol) of aniline, 0.1 g (1 mmol) of palladium acetate, 3 g (32 mmol) of sodium tert-butoxide, 0.2 g (1 mmol) of bis(diphenylphosphino)-1,1'-binaphthyl, and 45 mL of toluene were added to a 100 mL reactor and the mixture was stirred under reflux for 24 hours. After completion of the reaction, the mixture was filtered, concentrated and separated by column chromatography to obtain 5.2 g of [A1-c] (yield 82%).

### Synthesis Example A1-4. Synthesis of A1-d

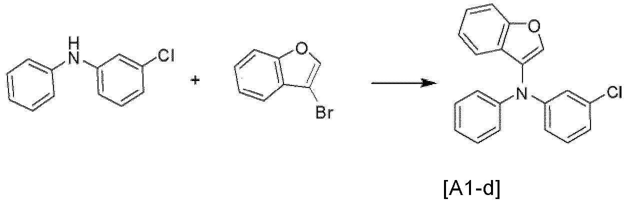

20 g (98 mmol) of [A1-c], 18.4 g (98 mmol) of [A1-b], 0.5 g (2 mmol) of palladium acetate, 18.9 g (196 mmol) of sodium tert-butoxide, 0.8 g (4 mmol) of tri-tert-butylphosphine and 200 mL of toluene were added to a 250 mL reactor, followed by stirring under reflux. After completion of the reaction, the reaction product was filtered, concentrated, and separated by column chromatography to obtain 22 g of [A1-d] (yield 75%).

### Synthesis Example A1-5. Synthesis of A1-e

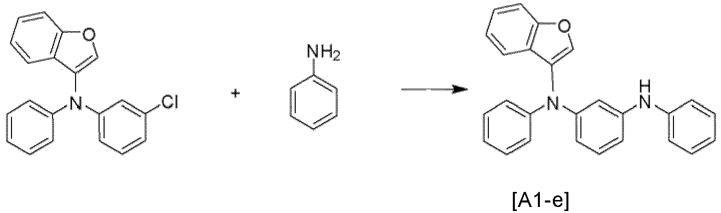

18.5 g of [A1-e] was obtained in the same manner as in Synthesis Example A1-3 except that [A1-d] was used instead of 1-bromo-3-chlorobenzene (yield 74.1%).

### Synthesis Example A1-6. Synthesis of A1-f

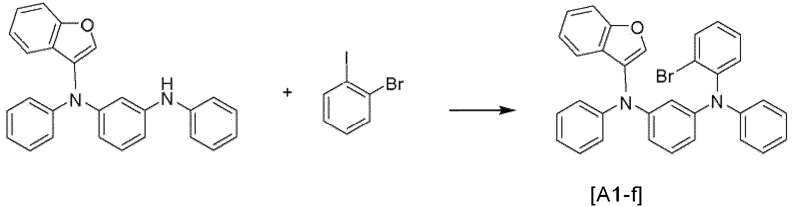

12 g of [A1-f] was obtained in the same manner as in Synthesis Example A1-4 except that [A1-e] and 1-bromo-2-iodobenzene were used instead of [A1-c] and [A1-b], respectively (yield 84.1%).

### Synthesis Example A1-7. Synthesis of Formula A1

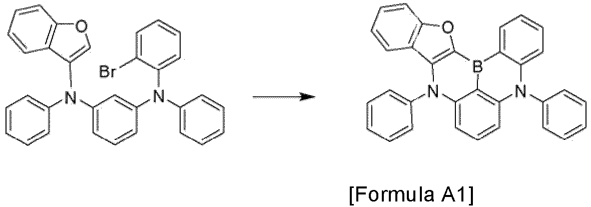

12 g (23 mmol) of [A1-f] and 120 mL of tert-butylbenzene were added to a 300 mL reactor, 42.5 mL (68 mmol) of n-butyllithium was added dropwise thereto at -78°C, the mixture was stirred at 60°C for 3 hours, and nitrogen was purged to remove heptane. 11.3 g (45 mmol) of boron tribromide was added dropwise thereto at -78°C, followed by stirring at room temperature for 1 hour, dropwise addition of 5.9 g (45 mmol) of N,N-diisopropylethylamine at 0°C, and stirring at 120°C for 2 hours. After completion of the reaction, an aqueous sodium acetate solution was added thereto at room temperature, the mixture was stirred and extracted with ethyl acetate, and the organic layer was concentrated and separated by column chromatography to obtain 0.8 g of [Formula A1] (yield 13%).
MS (MALDI-TOF): m/z 460.17 [M+]

### Synthesis Example A2. Synthesis of Formula A2

### Synthesis Example A2-1. Synthesis of A2-a

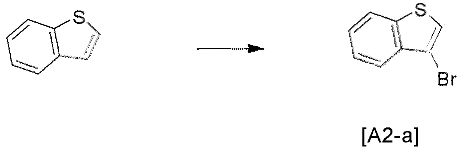

50 g (373 mmol) of benzothiophene and 500 mL of chloroform were added to a 1 L reactor, followed by stirring. A dilution of 59.5 g (373 mmol) of bromine in 100 mL of chloroform was added dropwise thereto at 0°C, followed by stirring at room temperature for 4 hours. After completion of the reaction, an aqueous sodium thiosulfate solution was added to the reaction product, the resulting mixture was stirred and extracted, and the organic layer was concentrated under reduced pressure and separated by column chromatography to obtain 70 g of [A2-a] (yield 91%).

### Synthesis Example A2-2. Synthesis of A2-b

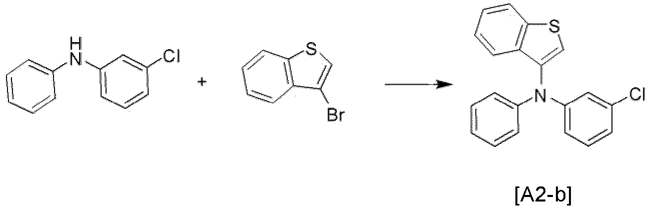

32 g of [A2-b] was obtained in the same manner as in Synthesis Example A1-4 except that [A2-

### a] was used instead of [A1-b] (yield 75.4%).

### Synthesis Example A2-3. Synthesis of A2-c

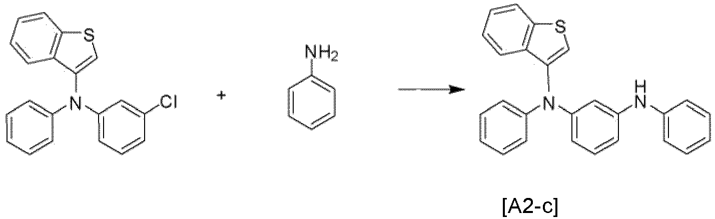

24.5 g of [A2-c] was obtained in the same manner as in Synthesis Example A1-3 except that [A2-b] was used instead of 1-bromo-4-iodobenzene (yield 73.1%).

### Synthesis Example A2-4. Synthesis of A2-d

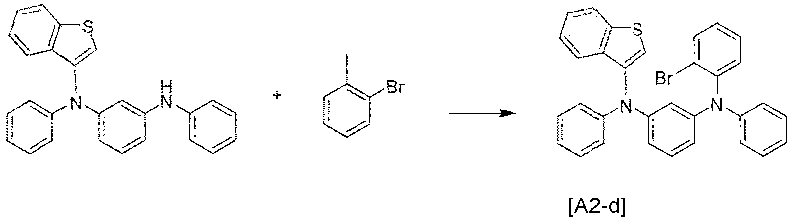

21 g of [A2-d] was obtained in the same manner as in Synthesis Example A1-4, except that [A2-c] and 1-bromo-2-iodobenzene were used instead of [A1-c] and [A1-b] (yield 77.5%).

### Synthesis Example A2-5. Synthesis of Formula A2

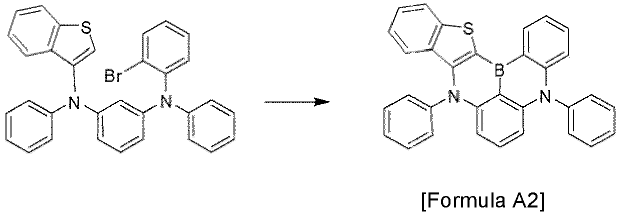

1.5 g of [Formula A2] was obtained in the same manner in Synthesis Example A1-7 except that [A2-d] was used instead of [A1-f] (yield 10.1%).
MS (MALDI-TOF): m/z 467.15 [M+]

### Synthesis Example A3. Synthesis of Formula A13

### Synthesis Example A3-1. Synthesis of A3-a

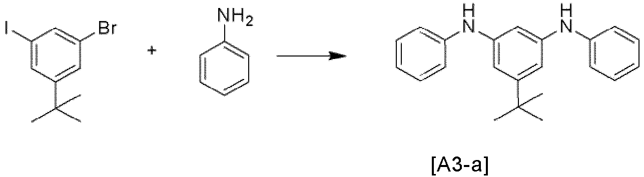

50 g (177 mmol) of 1-bromo-3-(tert-butyl)-5-iodobenzene, 36.2 g (389 mmol) of aniline, 1.6 g (7 mmol) of palladium acetate, 51 g (530 mmol) of sodium tert-butoxide, 4.4 g (7 mmol) of bis(diphenylphosphino)-1,1'-binaphthyl, and 500 mL of toluene were added to a 1 L reactor, followed by stirring under reflux for 24 hours. After completion of the reaction, the reaction product was filtered, concentrated, and separated by column chromatography to obtain 42.5 g of [A3-a] (yield 50%).

### Synthesis Example A3-2. Synthesis of A3-b

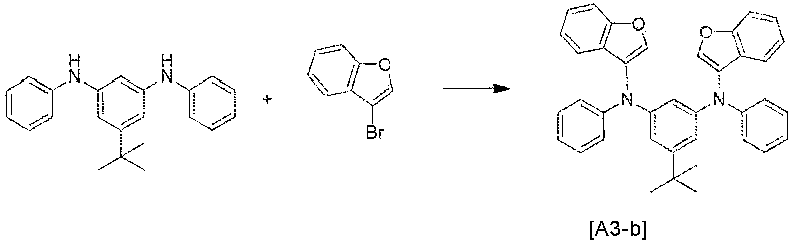

11 g (42 mmol) of [A3-a], 20 g (101 mmol) of [A1-b], 1 g (2 mmol) of palladium acetate, 12.2 g (127 mmol) of sodium tert-butoxide, 0.7 g (3 mmol) of tri-tert-butylphosphine, and 150 mL of toluene were added to a 250 mL reactor, followed by stirring under reflux. After completion of the reaction, the reaction product was filtered, concentrated, and separated by column chromatography to obtain 11 g of [A3-b] (yield 65%).

### Synthesis Example A3-3. Synthesis of Formula A13

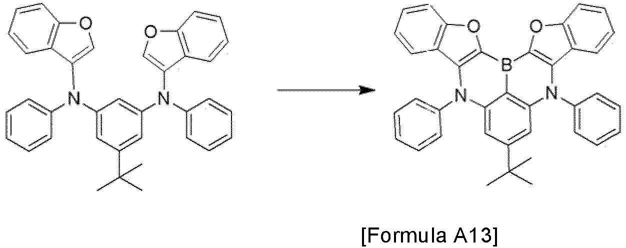

0.5 g of [Formula A13] was obtained in the same manner as in Synthesis Example A1-7 except that [A3-b] was used instead of [A1-f] (yield 8%).
MS (MALDI-TOF): m/z 556.23 [M+]

### Synthesis Example A4. Synthesis of Formula A65

### Synthesis Example A4-1. Synthesis of A4-a

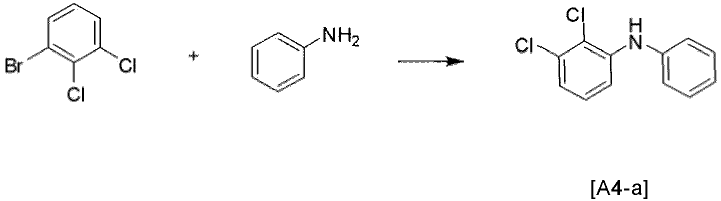

35.6 g of [A4-a] was obtained in the same manner as in Synthesis Example A1-3 except that 1-bromo-2,3-dichlorobenzene was used instead of 1-bromo-4-iodobenzene (yield 71.2%).

### Synthesis Example A4-2. Synthesis of A4-b

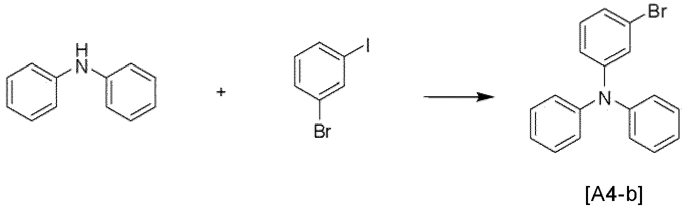

60.0 g (355 mmol) of diphenylamine, 100.3 g (355 mmol) of 1-bromo-3-iodobenzene, 0.8 g (4 mmol) of palladium acetate, 2 g (4 mmol) of xantphos, 68.2 g (709 mmol) of sodium tert-butoxide, and 700 mL of toluene were added to a 2 L reactor, followed by stirring under reflux for 2 hours. After completion of the reaction, the reaction product was filtered, concentrated under reduced pressure, and separated by column chromatography to obtain 97 g of [A4-b] (yield 91.2%).

### Synthesis Example A4-3. Synthesis of A4-c

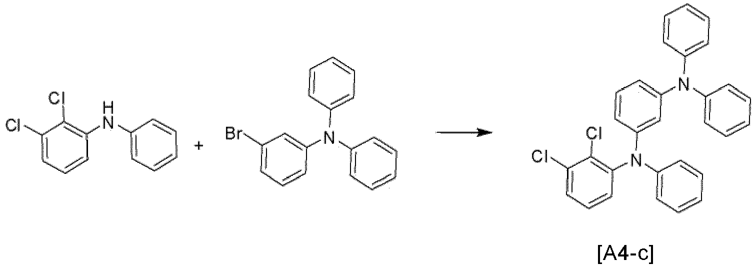

31 g of [A4-c] was obtained in the same manner as in Synthesis Example A1-4, except that [A4-a] and [A4-b] were used instead of [A1-c] and [A1-b] (yield 77.7%).

### Synthesis Example A4-4. Synthesis of A4-d

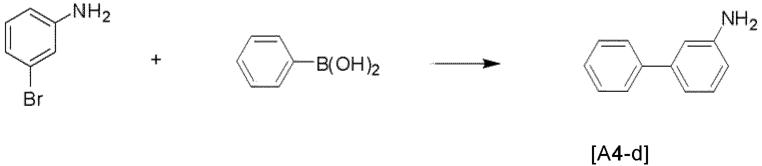

30 g (174 mmol) of 3-bromoaniline, 25.5 g (209 mmol) of phenylboronic acid, 4 g (3 mmol) of tetrakis(triphenylphosphine)palladium, 48.2 g (349 mmol) of potassium carbonate, 150 mL of 1,4-dioxane, 150 mL of toluene, and 90 mL of distilled water were added to a 1 L reactor, followed by stirring under reflux. After the reaction was completed, the layers were separated, and the organic layer was concentrated under reduced pressure and then separated by column chromatography to obtain 24 g of [A4-d] (yield 80%).

### Synthesis Example A4-5. Synthesis of A4-e

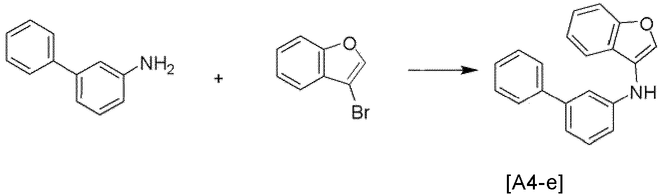

31.6 g of [A4-e] was obtained in the same manner as in Synthesis Example A1-3, except that [A4-d] and [A1-b] were used instead of 1-bromo-4-iodobenzene and aniline (yield 68.2%).

### Synthesis Example A4-6. Synthesis of A4-f

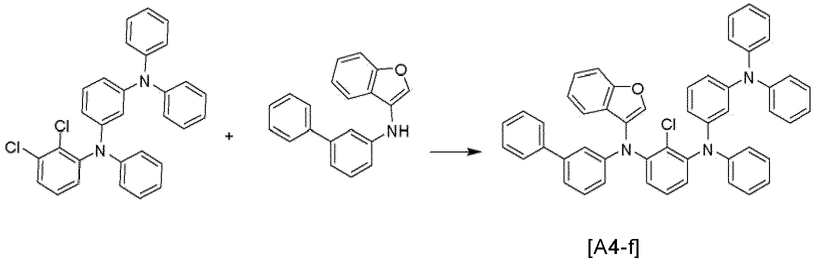

21 g of [A4-f] was obtained in the same manner as in Synthesis Example A1-4, except that [A4-c] and [A4-e] were used instead of [A1-c] and [A1-b] (yield 67.7%).

### Synthesis Example A4-7. Synthesis of Formula A65

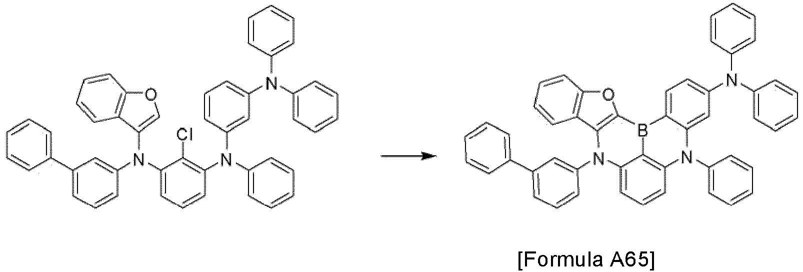

21 g (37 mmol) of [A4-f] and tert-butylbenzene were added to a 250 mL reactor. 42.4 mL (74 mmol) of tert-butyllithium was added dropwise at -78°C, followed by stirring at 60°C for 3 hours. Then, nitrogen was purged to remove pentane. 7.1 mL (74 mmol) of boron tribromide was added dropwise at -78°C, followed by stirring at room temperature for 1 hour. 6 g (74 mmol) of N,N-diisopropylethylamine was added dropwise at 0°C, followed by stirring at 120°C for 2 hours. After completion of the reaction, an aqueous sodium acetate solution was added thereto, followed by stirring. The reaction product was extracted with ethyl acetate, and the organic layer was concentrated and separated through column chromatography to obtain 2.0 g of [Formula A65] (yield 17.4%).
MS (MALDI-TOF): m/z 703.28 [M+]

### Synthesis Example A5. Synthesis of Formula A73

### Synthesis Example A5-1. Synthesis of A5-a

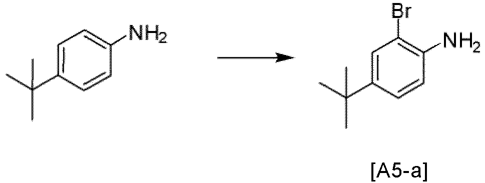

40 g (236 mmol) of 4-tert-butylaniline was dissolved in 400 mL of methylene chloride in a 1 L reactor, followed by stirring at 0°C. Then, 42 g (236 mmol) of N-bromosuccinimide was added thereto, followed by stirring at room temperature for 4 hours. After completion of the reaction, H₂O was added dropwise to the reaction product, followed by extraction with methylene chloride. The organic layer was concentrated and separated by column chromatography to obtain 48 g of [A5-a] (yield 80%).

### Synthesis Example A5-2. Synthesis of A5-b

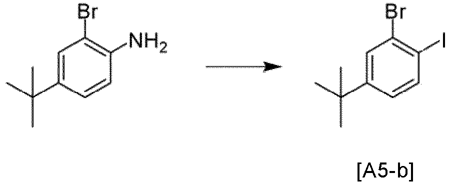

80 g (351 mmol) of [A5-a] and 450 mL of water were added to a 2 L reactor, followed by stirring and addition of sulfuric acid 104 mL thereto. A solution of 31.5 g (456 mmol) of sodium nitrite in 240 mL of water was added dropwise at 0°C, followed by stirring at 0°C for 2 hours. A solution of 116.4 g (701 mmol) of sodium nitrite in 450 mL of water was added dropwise, followed by stirring at room temperature for 6 hours. After completion of the reaction, an aqueous sodium thiosulfate solution was added at room temperature, followed by stirring. The reaction product was extracted with ethyl acetate, and the organic layer was separated by column chromatography to obtain 58 g of [A5-b] (yield 51%).

### Synthesis Example A5-3. Synthesis of A5-c

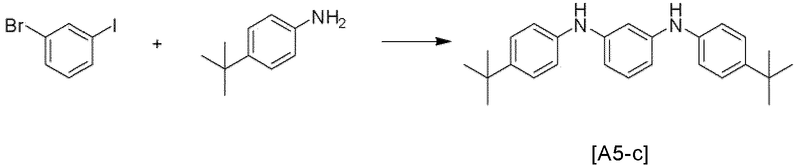

95 g of [A5-c] was obtained in the same manner as in Synthesis Example except that 4-tert-butylaniline was used instead of aniline A3-1 (yield 80.4%).

### Synthesis Example A5-4. Synthesis of A5-d

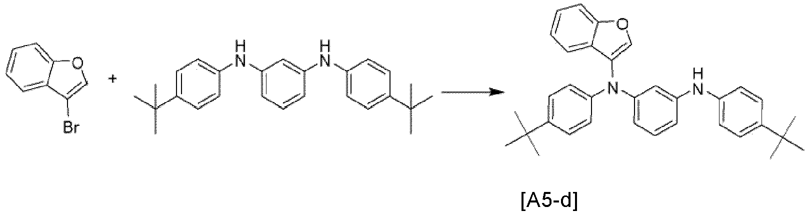

31 g of [A5-d] was obtained in the same manner as in Synthesis Example A1-4 except that [A5-c] was used instead of [A1-c] (yield 71.5%).

### Synthesis Example A5-5. Synthesis of A5-e

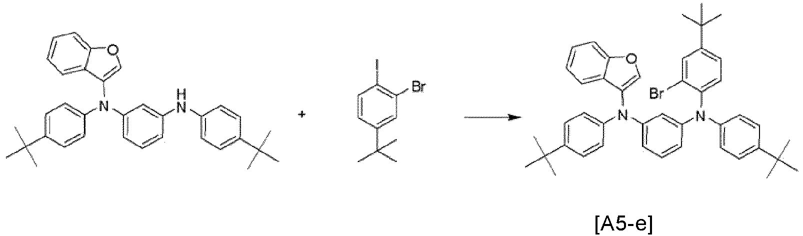

24 g of [A5-e] was obtained in the same manner as in Synthesis Example A1-4, except that [A5-d] and [A5-b] were used instead of [A1-c] and [A1-b] (yield 67.1%).

### Synthesis Example A5-6. Synthesis of Formula A73

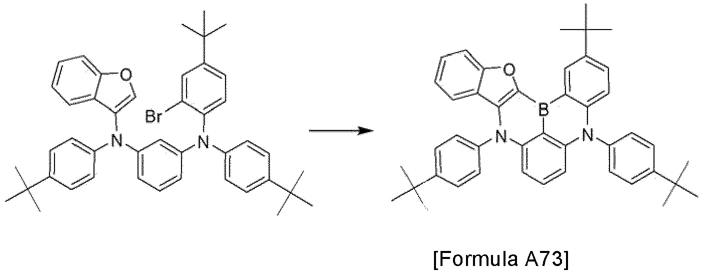

2.4 g of [Formula A73] was obtained in the same manner as in Synthesis Example A1-7 except that [A5-e] was used instead of [A1-f] (yield 15%).
MS (MALDI-TOF): m/z 628.36 [M+]

### Synthesis Example A6. Synthesis of Formula A109

### Synthesis Example A6-1. Synthesis of A6-a

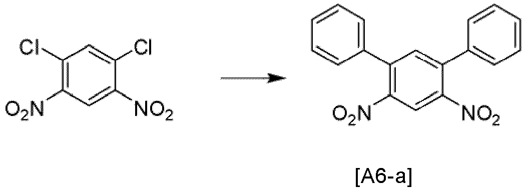

40.0 g (123 mmol) of 1,5-dichloro-2,4-dinitrobenzene, 44.9 g (368 mmol) of phenylboronic acid, 2.8 g (2.5 mmol) of tetrakis(triphenylphosphine) palladium, 50.9 g (368 mmol) of potassium carbonate, 120 mL of 1,4-dioxane, 200 mL of toluene, and 120 mL of water were added to a 1 L reactor, followed by stirring under reflux. After completion of the reaction, the reaction product was extracted and the organic layer was separated by column chromatography to obtain 27.5 g of [A6-a] (yield 70%).

### Synthesis Example A6-2. Synthesis of A6-b

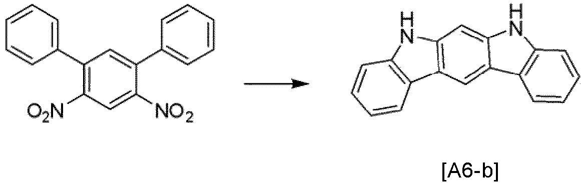

27.5 g (86 mmol) of [A6-a], 57.8 g (348 mmol) of triphenylphosphine and 300 mL of dichlorobenzene were added to a 1 L reactor, followed by stirring under reflux for 3 days. After completion of the reaction, dichlorobenzene was removed and the residue was separated by column chromatography to obtain 10.8 g of [A6-b] (yield 49.0%).

### Synthesis Example A6-3. Synthesis of A6-c

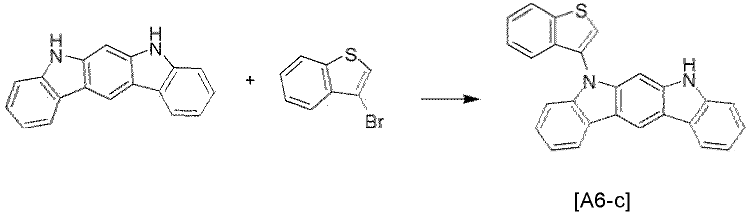

10.8 g (42 mmol) of [A6-b], 11.0 g (10.8 mmol) of [A2-a], 10.7 g (1 mmol) of a copper powder, and 4.5 g (17 mmol) of 18-crown-6-ether were added to a 250 mL reactor, 34.9 g (253 mmol) of potassium carbonate was added thereto, and 110 mL of dichlorobenzene was added thereto, followed by stirring under reflux at 180°C for 24 hours. After completion of the reaction, dichlorobenzene was removed and the residue was separated by column chromatography to obtain 9.5 g of [A6-c] (yield 52%).

### Synthesis Example A6-4. Synthesis of A6-d

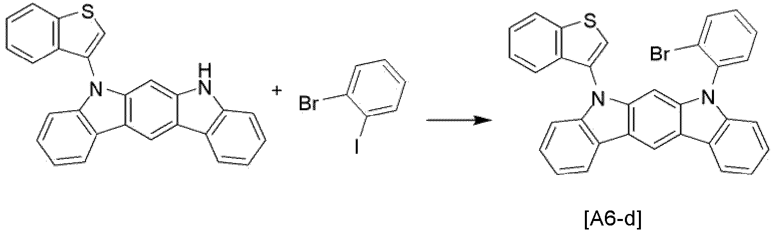

14 g of [A6-d] was obtained in the same manner as in Synthesis Example A6-3 except that [A6-c] and 1-bromo-2-iodobenzene were used instead of [A1-c] and [A2-a] (yield 67.1%).

### Synthesis Example A6-5. Synthesis of Formula A109

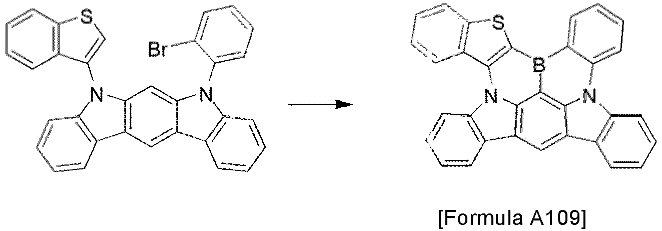

2.1 g of [Formula A109] was obtained in the same manner as in Synthesis Example A1-7 except that [A6-d] was used instead of [A1-f] (yield 14%).
MS (MALDI-TOF): m/z 472.12 [M+]

### Synthesis Example A7. Synthesis of formula A126

### Synthesis Example A7-1. Synthesis of A7-a

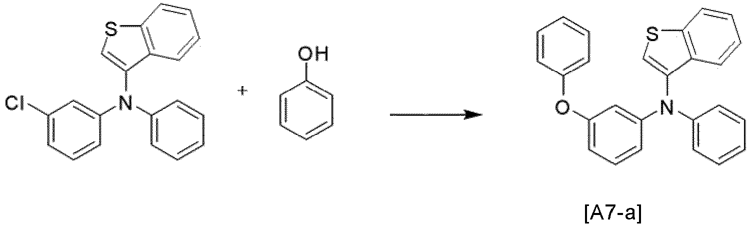

30.0 g (150 mmol) of [A2-b], 31.2 g (160 mmol) of phenol, 45.7 g (300 mmol) of potassium carbonate and 250 mL of NMP were added to a 500 mL reactor, followed by stirring under reflux at 160°C for 12 hours. After completion of the reaction, the reaction product was cooled to room temperature, NMP was distilled off under reduced pressure, and the residue was extracted with water and ethyl acetate. The solvent was concentrated under reduced pressure and separated by column chromatography to obtain 22 g of [A7-a] (yield 68%).

### Synthesis Example A7-2. Synthesis of formula A126

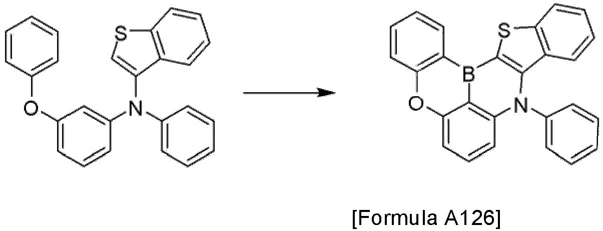

1.2 g of [Formula A126] was obtained in the same manner as in Synthesis Example A1-7, except that [A7-a] was used instead of [A1-f] (yield 13.4%).
MS (MALDI-TOF): m/z 401.10 [M+]

### Synthesis Example A8. Synthesis of Formula A145

### Synthesis Example A8-1. Synthesis of A8-a

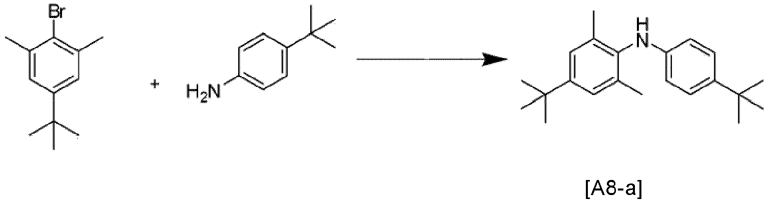

41.6 g of [A8-a] was obtained in the same manner as in Synthesis Example A1-3 except that 2-bromo-5-tert-butyl-1,3-dimethylbenzene and 4-tert-butylaniline were used instead of 1-bromo-3-iodobenzene and aniline (yield 88.2%).

### Synthesis Example A8-2. Synthesis of A8-b

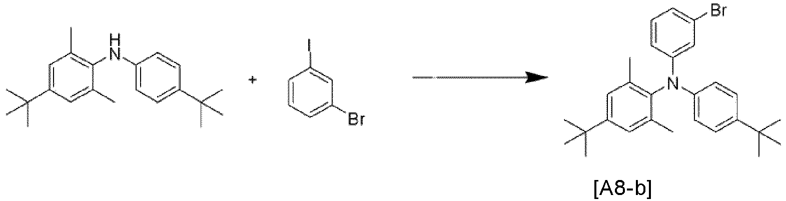

37.6 g of [A8-b] was obtained in the same manner as in Synthesis Example A4-2 except that [A8-a] was used instead of diphenylamine (yield 78.4%).

### Synthesis Example A8-3. Synthesis of A8-c

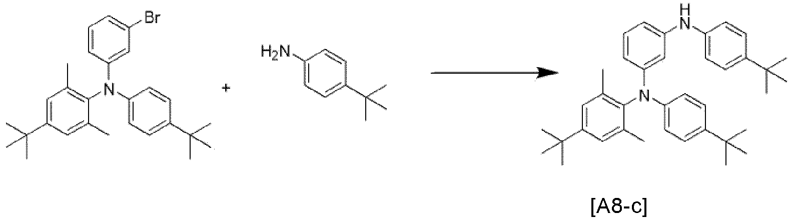

31.2 g of [A8-c] was obtained in the same manner as in Synthesis Example A1-3, except that [A8-b] and 4-tert-butylaniline were used instead of 1-bromo-3-iodobenzene and aniline (yield 74.2%).

### Synthesis Example A8-4. Synthesis of A8-d

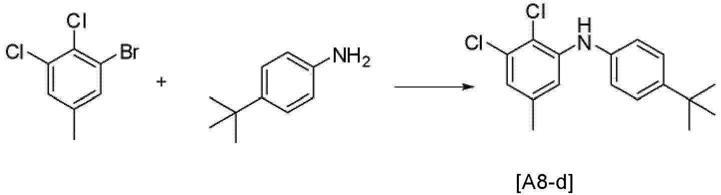

30.3 g of [A8-d] was obtained in the same manner as in Synthesis Example A1-3, except that 1-bromo-2,3-dichloro-5-methylbenzene and 4-tert-butylaniline were used instead of 1-bromo-3-iodobenzene and aniline (yield 89.8%).

### Synthesis Example A8-5. Synthesis of A8-e

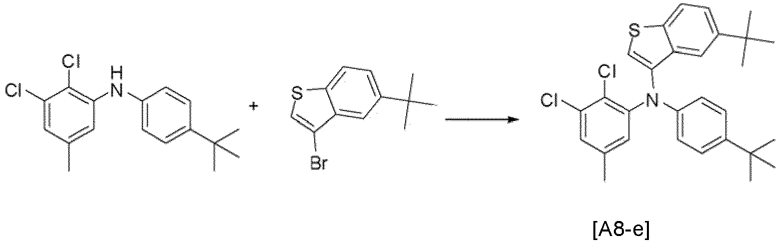

27.4 g of [A8-e] was obtained in the same manner as in Synthesis Example A1-4 g except that [A8-d] and 3-bromo-5-(tert-butyl)benzothiophene were used instead of [A1-c] and [A1-b] (yield 77.1%).

### Synthesis Example A8-6. Synthesis of A8-f

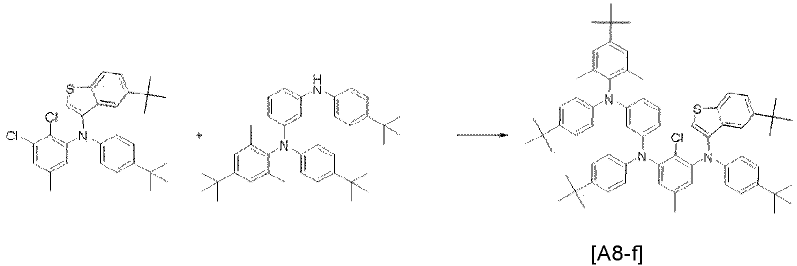

21 g of [A8-f] was obtained in the same manner as in Synthesis Example A1-4, except that [A8-e] and [A8-c] were used instead of [A1-c] and [A1-b] (yield 74.1%).

### Synthesis Example A8-7. Synthesis of Formula A145

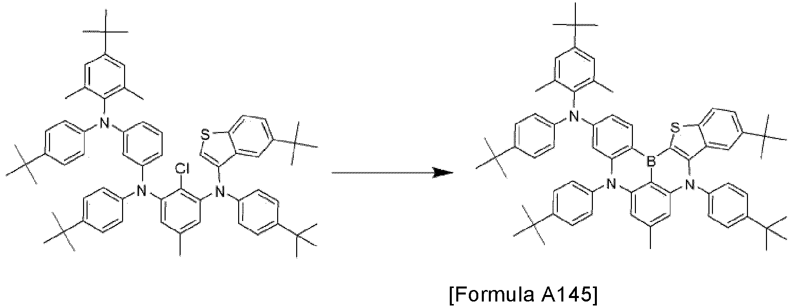

3.4 g of [Formula A145] was obtained in the same manner as in Synthesis Example A1-7, except that [A8-f] was used instead of [A1-f] (yield 19.4%).
MS (MALDI-TOF): m/z 979.60 [M]+

### Synthesis Example A9. Synthesis of Formula A150

### Synthesis Example A9-1. Synthesis of A9-a

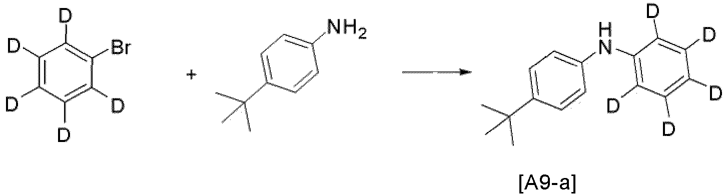

32.7 g of [A9-a] was obtained in the same manner as in Synthesis Example A1-3, except that 1-bromobenzene (D-substituted) and 4-tert-butylaniline were used instead of 1-bromo-3-iodobenzene and aniline (yield 78.2%).

### Synthesis Example A9-2. Synthesis of A9-b

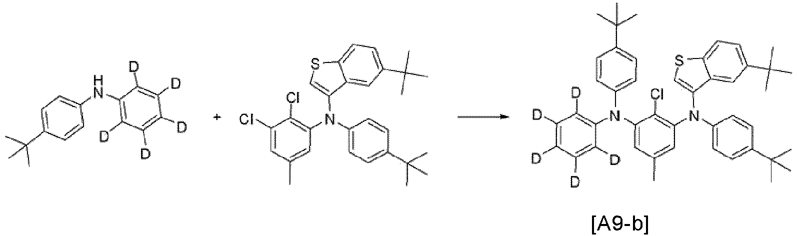

34.2 g of [A9-b] was obtained in the same manner as in Synthesis Example A1-4, except that [A8-e] and [A9-a] were used instead of [A1-c] and [A1-b] (yield 84.1%).

### Synthesis Example A9-3. Synthesis of Formula A150

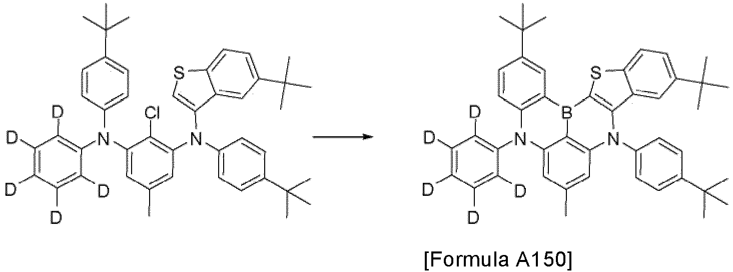

2.7 g of [Formula A150] was obtained in the same manner as in Synthesis Example A1-7, except that [A9-b] was used instead of [A1-f] (yield 11.4%).
MS (MALDI-TOF): m/z 663.39 [M]+

### Synthesis Example A10. Synthesis of formula A153

### Synthesis Example A10-1. Synthesis of A10-a

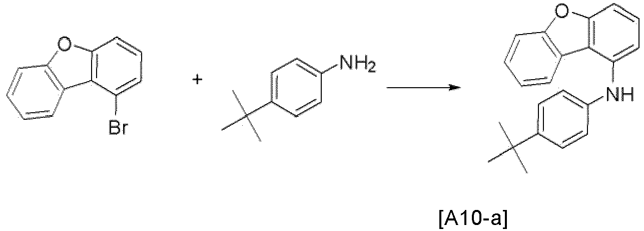

25.6 g of [A10-a] was obtained in the same manner in Synthesis Example A1-3 except that 1-bromo-dibenzofuran and 4-tert-butylaniline were used instead of 1-bromo-3-iodobenzene and aniline (yield 79.2%).

### Synthesis Example A10-2. Synthesis of A10-b

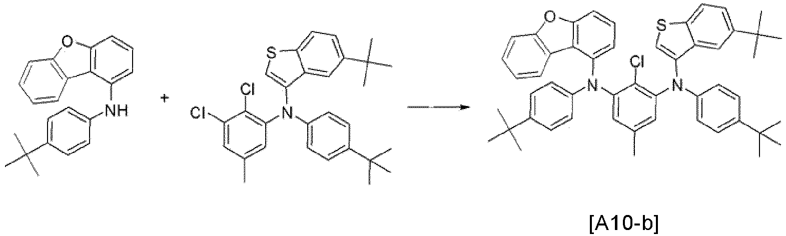

18.6 g of [A10-b] was obtained in the same manner as in Synthesis Example A1-4, except that [A8-e] and [A10-a] were used instead of [A1-c] and [A1-b] (yield 74.1%).

### Synthesis Example A10-3. Synthesis of formula A153

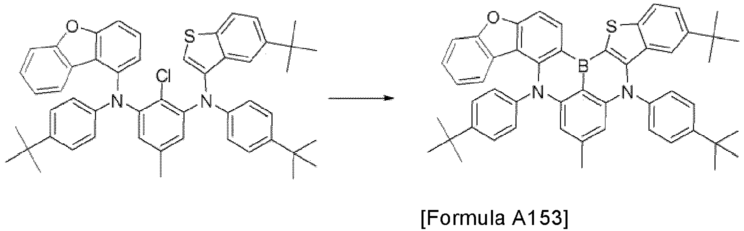

3.4 g of [Formula A153] was obtained in the same manner as in Synthesis Example A1-7 except that [A10-b] was used instead of [A1-f] (yield 15.4%).
MS (MALDI-TOF): m/z 748.37 [M]+

### Synthesis Example A11. Synthesis of Formula A185

2.1 g of [Formula A185] were obtained in the same manner as in Synthesis Examples A3-1 to A3-3, except that in Synthesis Example A3, 1-bromo-3-iodobenzene was used instead of 1-bromo-3-(tert-butyl)-5-iodobenzene and 4-tert-butylaniline was used instead of aniline in Synthesis Example A3-1, and 3-bromo-5-methylbenzofuran was used instead of 3-bromobenzofuran [A1-b] in Synthesis Example 3-2 (yield 12%).
MS (MALDI-TOF): m/z 640.33 [M]⁺

### <Synthesis of Formula B>

### Synthesis Example B1: Synthesis of Formula B4

### Synthesis Example B1-1 : Synthesis of B1-a

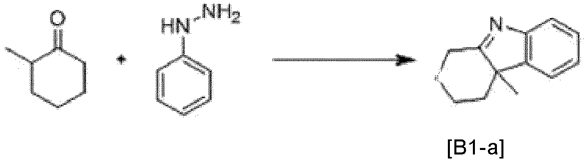

100 g (0.924 mol) of phenylhydrazine and 500 mL of acetic acid were stirred in a round bottom flask, followed by heating to 60°C. 103.6 g (0.924 mol) of 2-methyl cyclohexanone was slowly added dropwise, followed by reflux for 8 hours. After completion of the reaction, the mixture was extracted with water and ethyl acetate, concentrated, and separated by column chromatography to obtain 130 g of [B1-a] (yield 76%).

### Synthesis Example B1-2. Synthesis of B1-b

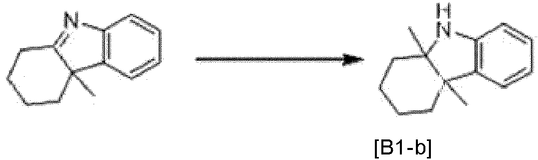

75 g (405 mmol) of [B1-a] was added to a round-bottom flask containing 750 mL of toluene under a nitrogen atmosphere, the mixture was cooled to 10°C, and 380 mL (608 mmol) of 1.6 M methyllithium was slowly added dropwise, followed by stirring at -10°C for about 3 hours. After completion of the reaction, the mixture was extracted with water and ethyl acetate, concentrated, and separated by column chromatography to obtain 50.5 g of [B1-b] (yield 62%).

### Synthesis Example B1-3. Synthesis of B1-c

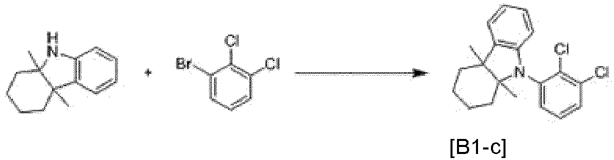

50 g (251 mmol) of [B1-b], 56.7 g (251 mmol) of 1-bromo-2,3-dichlorobenzene, 4.5 g (5 mmol) of tris(dibenzylideneacetone)dipalladium, 2 g (10 mmol) of tri-tert-butyl phosphine, 35.8 g (373 mmol) of sodium tert-butoxide, and 500 mL of toluene were added to a round-bottom flask under nitrogen atmosphere, followed by refluxing for 24 hours. After completion of the reaction, the organic layer was concentrated under reduced pressure and the residue was separated by column chromatography to obtain 35.6 g of [B1-c] (yield 41%).

### Synthesis Example B1-4. Synthesis of B1-d

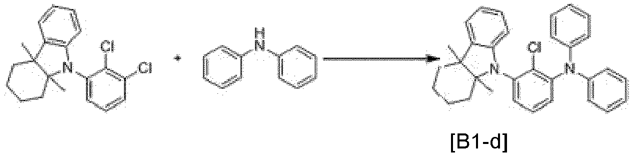

[B1-d] was obtained by synthesis in the same manner except that diphenylamine was used instead of [B1-b] and [B1-c] was used instead of 1-bromo-2,3-dichlorobenzene in Synthesis Example B1-3 (yield 73%).

### Synthesis Example B1-5. Synthesis of Formula B4

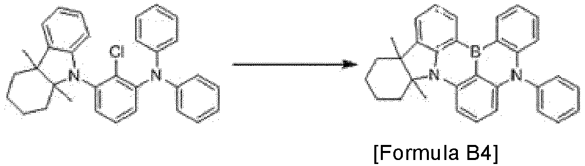

20 g (42 mmol) of [Intermediate B1-d] was added to a round-bottom flask containing 200 mL of tert-butylbenzene under a nitrogen atmosphere, the mixture was cooled to -30°C, and 49.1 mmL (84 mmol) of a 1.7 M tert-butyllithium pentane solution was slowly added dropwise. After completion of the dropwise addition, the reaction product was heated to 60°C and stirred for 3 hours, and then pentane was distilled off. The residue was cooled to -50°C, 20.8 g (84 mmol) of boron tribromide was added dropwise thereto, and the mixture was warmed to room temperature and then was stirred for 1 hour. The reaction product was cooled to 0°C again, and 10.7 g (84 mmol) of N,N-diisopropylethylamine was added thereto, followed by stirring at 120°C for 3 hours. After completion of the reaction, tert-butylbenzene was distilled off under reduced pressure, and the residue was extracted using water and ethyl acetate, concentrated and separated by column chromatography to obtain 5.3 g of [Formula B4] (yield 28%).
MS (MALDI-TOF): m/z 452.24 [M +]

### Synthesis Example B2: Synthesis of Formula B106

[Formula B106] was obtained by synthesis in the same manner except that 1-bromo-2,3-dichloro-5-tert-butylbenzene was used instead of 1-bromo-2,3-dichlorobenzene in Synthesis Example B1-3, and N1,N2,N3-triphenyl-1,3-benzenediamine was used instead of diphenylamine in Synthesis Example B1-4 (yield 21%).
MS(MALDI-TOF): m/z 675.38 [M+]

### Synthesis Example B3: Synthesis of Compound B91

### Synthesis Example B3-1: Synthesis of B3-a

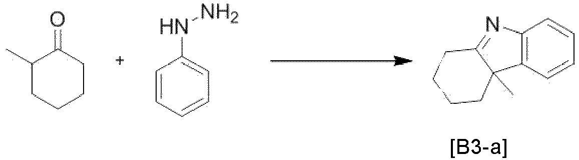

100 g (0.924 mol) of phenylhydrazine and 500 mL of acetic acid were stirred in a round bottom flask, followed by heating to 60°C. 103.6 g (0.924 mol) of 2-methyl cyclohexanone was slowly added dropwise, followed by reflux for 8 hours. After completion of the reaction, the mixture was extracted using water and ethyl acetate, concentrated, and separated by column chromatography to obtain 130 g of [B3-a] (yield 76%).

### Synthesis Example B3-2. Synthesis of B3-b

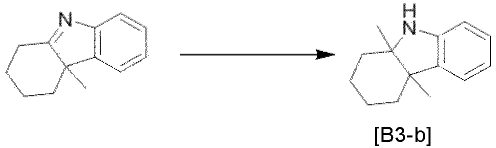

75 g (405 mmol) of [B3-a] was added to a round-bottom flask containing 750 mL of toluene under a nitrogen atmosphere, the mixture was cooled to -10°C, and 80 mL (608 mmol) of 1.6 M methyl lithium was slowly added dropwise, followed by stirring to -10°C for about 3 hours. After completion of the reaction, the mixture was extracted with water and ethyl acetate, concentrated, and separated by column chromatography to obtain 50.5 g of [B3-b] (yield 62%).

### Synthesis Example B3-3. Synthesis of B3-c

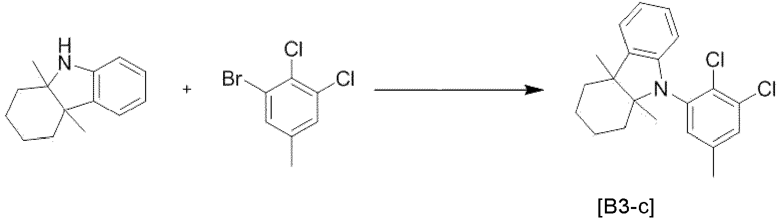

40 g (199 mmol) of [B3-b], 47.7 g (251 mmol) of 1-bromo-2,3-dichloro-5-methylbenzene, 7.3 g (7.9 mmol) of tris(dibenzylideneacetone)dipalladium, 3.2 g (15.9 mmol) of tri-tert-butylphosphine, 38.2 g (397 mmol) of sodium tert-butoxide, and 400 mL of toluene were added to a round-bottom flask under nitrogen atmosphere, followed by refluxing for 12 hours. After completion of the reaction, the organic layer was concentrated under reduced pressure, and then separated by column chromatography to obtain 23 g of [B3-c] (yield 32%).

### Synthesis Example B3-4. Synthesis of B3-d

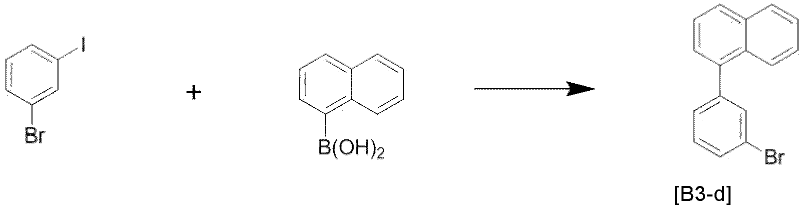

30 g (106 mmol) of 1-bromo-3-iodobenzene, 16.4 g (95 mmol) of 1-naphthaleneboronic acid, 1.8 g (1.6 mmol) of tetrakis(triphenylphosphine)palladium, 17.6 g (127 mmol) of potassium carbonate, 300 mL of tetrahydrofuran, 300 mL of toluene, and 120 mL of water were added to a round bottom flask under nitrogen atmosphere, followed by refluxing for 24 hours. After the reaction was completed, the reaction product was cooled to room temperature and extracted. The organic layer was concentrated under reduced pressure and separated by column chromatography to obtain 19 g of [B3-d] (yield 70.3%).

### Synthesis Example B3-5. Synthesis of B3-e

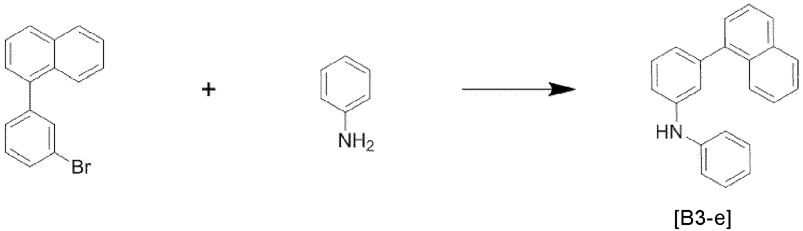

19 g (67 mmol) of [B3-d], 6.2 g (67 mmol) of aniline, 1.2 g (1 mmol) of tris(dibenzylideneacetone)dipalladium, 7.7 g (81 mmol) of sodium tert-butoxide, 0.8 g (1 mmol) of bis(diphenylphosphino)-1,1'-binaphthyl, and 200 mL of toluene were added to a round bottom flask, followed by stirring under reflux for 24 hours. After completion of the reaction, the reaction product was extracted and the organic layer was concentrated under reduced pressure and separated by column chromatography to obtain 15.2 g of [B3-e] (yield 77%).

### Synthesis Example B3-6. Synthesis of B3-f

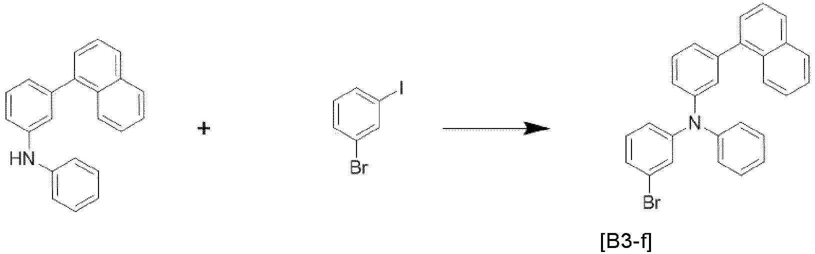

15.2 g (51 mmol) of [B3-e], 18.9 g (67 mmol) of 1-bromo-3-iodobenzene, 0.9 g (1 mmol) of tris(dibenzylideneacetone)dipalladium, 6.4 g (67 mmol) of sodium tert-butoxide, 0.6 g (1 mmol) of bis(diphenylphosphino)-1,1'-binaphthyl, and 150 mL of toluene were added to a round bottom flask, followed by stirring under reflux for 24 hours. After completion of the reaction, the reaction product was extracted and the organic layer was concentrated under reduced pressure and separated by column chromatography to obtain 14.9 g of [B3-fJ (yield 64%).

### Synthesis Example B3-7. Synthesis of B3-q

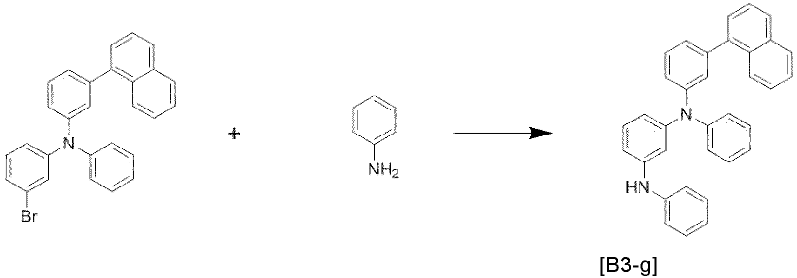

14.9 g (33 mmol) of [B3-f], 3.1 g (33 mmol) of aniline, 0.6 g (0.7 mmol) of tris(dibenzylideneacetone)dipalladium, 3.8 g (40 mmol) of sodium tert-butoxide, 0.4 g (0.7 mmol) of bis(diphenylphosphino)-1,1'-binaphthyl and 150 mL of toluene were added to a round bottom flask, followed by stirring under reflux for 20 hours. After completion of the reaction, the reaction product was extracted and the organic layer was concentrated under reduced pressure and separated by column chromatography to obtain 11.4 g of [B3-g] (yield 75%).

### Synthesis Example B3-8. Synthesis of B3-h

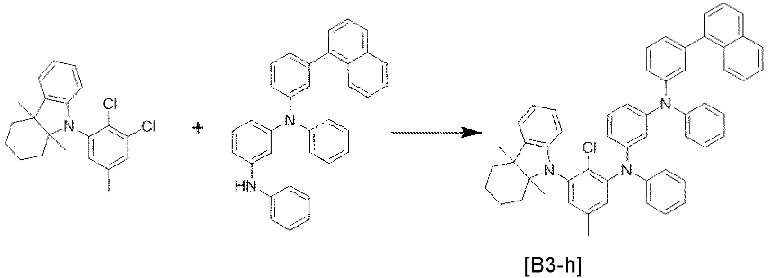

8.9 g (25 mmol) of [Intermediate B3-c], 11.4 g (25 mmol) of [Intermediate B3-g], 0.5 g (0.5 mmol) of tris(dibenzylideneacetone)dipalladium, 3.6 g (37 mmol) of sodium tert-butoxide, 0.1 g (0.5 mmol) of tri-tert-butylphosphine, and 120 mL of toluene are added to a round bottom flask, followed by stirring under reflux for 6 hours. After completion of the reaction, the reaction product was extracted and the organic layer was concentrated under reduced pressure and separated by column chromatography. The result was recrystallized from dichloromethane and methanol to obtain 12.7 g of [Intermediate B3-h] (yield 65%).

### Synthesis Example B3-9. Synthesis of Formula B91

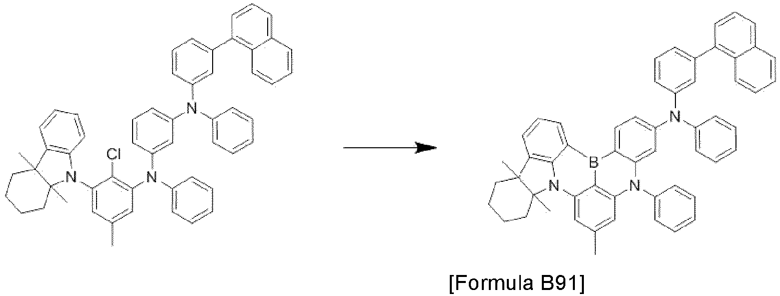

12.7 g (16 mmol) of [B3-h] and 89 mL of tert-butylbenzene are added to a reactor under a nitrogen atmosphere. 20.2 mL (32 mmol) of 1.6 M tert-butyllithium is added dropwise at -78°C. After dropwise addition, the mixture was stirred at 60°C for 2 hours. 8.1 mL (32 mmol) of boron tribromide is added dropwise at -78°C. After the dropwise addition, the mixture was stirred at room temperature for 1 hour, and 4.2 g (32 mmol) of N,N-diisopropylethylamine was added dropwise at 0°C. After dropwise addition, the mixture was stirred at 120°C for 3 hours. After completion of the reaction, an aqueous sodium acetate solution was added at room temperature, followed by stirring. The reaction product was extracted with ethyl acetate, and the organic layer was concentrated and separated by column chromatography to obtain 1.3 g of [Formula B91] (yield 10.6%).

### Synthesis Example B4: Synthesis of Formula B92

[Formula B92] was obtained by synthesis in the same manner except that 4-tert-butylaniline was used instead of aniline in Synthesis Example B3-5 (yield 13%).

### Synthesis Example B5: Synthesis of Formula B112

[Formula B112] was obtained by synthesis in the same manner except that 1-bromo-2,3-dichloro-5-tert-butylbenzene was used instead of 1-bromo-2,3-dichloro-5-methylbenzene in Synthesis Example B3-3 and 1-bromonaphthalene was used instead of [Intermediate B3-d] in Synthesis Example B3-5 (yield 14.5%).

### Examples 1 to 54: Fabrication of organic light-emitting devices

ITO glass was patterned such that a light-emitting area of the ITO glass was adjusted to 2 mm × 2 mm and was then washed. The ITO glass was mounted in a vacuum chamber, a base pressure was set to 1 × 10⁻⁷ torr, and DNTPD (700Å) and α-NPD (300Å) were sequentially deposited on ITO. Then, a mixture of the host compound according to the present invention and the dopant compound (3 wt%) according to the present invention shown in [Table 1] to [Table 4] was deposited to a thickness of 250Â to form a light-emitting layer. Then, a mixture of [Formula E-1] and [Formula E-2] in a ratio of 1:1 was deposited thereon to a thickness of 300Â to form an electron transport layer, [Formula E-1] was deposited thereon to a thickness of 5Â to form an electron injection layer, and AI was deposited thereon to a thickness of 1,000Å to form an anode. As a result, an organic light-emitting device was fabricated. The luminous efficacy of the organic light-emitting device was measured at 0.4 mA.

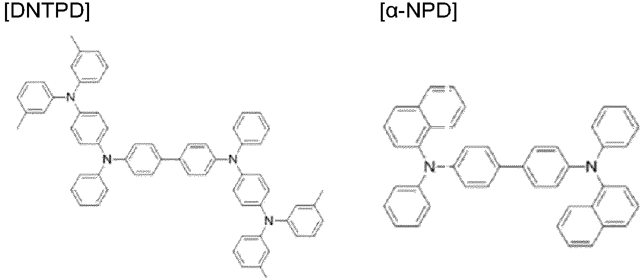

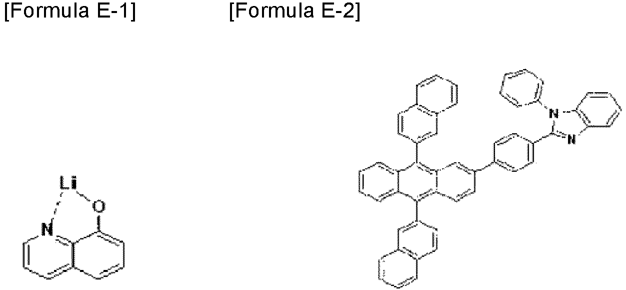

### Comparative Examples 1 to 38

Organic light-emitting devices of Comparative Examples were fabricated in the same manner as in Examples above, except that combinations of the host and dopant compounds shown in [Table 1] to [Table 4] were used instead of the compounds for the device structures of Examples. The luminous efficacy of the organic light-emitting devices was measured at 0.4 mA. The structures of BH1 to BH9, and BD1 to BD3 are as follows.

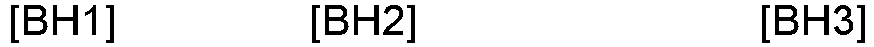

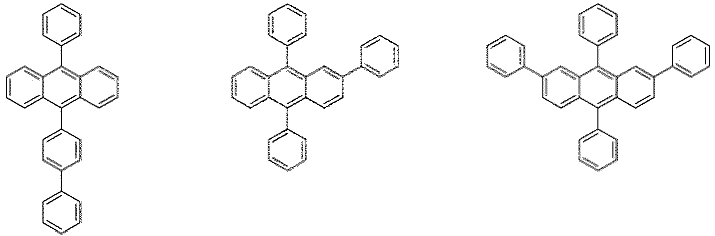

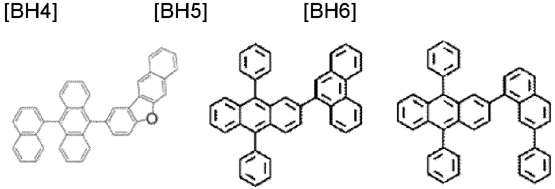

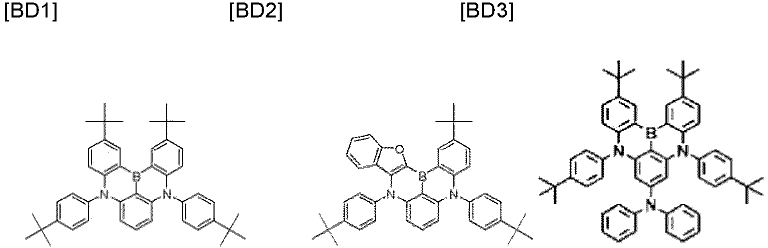

The voltage, external quantum efficiency, and lifespan of the organic light emitting devices fabricated according to Examples and Comparative Examples were measured and the results are shown in [Table 1] to [Table 4].

**[Table 1]**

| Item | Host | Dopant | Voltage(V) | External quantum efficiency(EQE) |
|---|---|---|---|---|
| Example 1 | Compound 1 | BD1 | 3.81 | 8.3 |
| Example 2 | Compound 3 | BD1 | 3.75 | 8.3 |
| Example 3 | Compound 4 | BD1 | 3.83 | 8.2 |
| Example 4 | Compound 9 | BD1 | 3.73 | 8.5 |
| Example 5 | Compound 10 | BD1 | 3.74 | 8.4 |
| Example 6 | Compound 12 | BD1 | 3.73 | 8.4 |
| Example 7 | Compound 24 | BD1 | 3.69 | 8.3 |
| Example 8 | Compound 30 | BD1 | 3.65 | 8.4 |
| Example 9 | Compound 31 | BD1 | 3.51 | 8.8 |
| Example 10 | Compound 34 | BD1 | 3.68 | 8.6 |
| Comparative Example 1 | BH1 | BD1 | 4.22 | 7.1 |
| Comparative Example 2 | BH2 | BD1 | 4.03 | 7.3 |
| Comparative Example 3 | BH3 | BD1 | 3.81 | 7.6 |
| Comparative Example 4 | BH4 | BD1 | 3.76 | 8.0 |
| Comparative Example 5 | BH6 | BD3 | 4.04 | 8.0 |

**[Table 2]**

| Item | Host | Dopant | Voltage(V) | External quantum efficiency(EQE) |
|---|---|---|---|---|
| Example 11 | Compound 40 | BD2 | 3.13 | 8.6 |
| Example 12 | Compound 41 | BD2 | 3.36 | 8.5 |
| Example 13 | Compound 45 | BD2 | 3.68 | 8.5 |
| Example 14 | Compound 47 | BD2 | 3.63 | 8.4 |
| Example 15 | Compound 50 | BD2 | 3.63 | 8.5 |
| Example 16 | Compound 58 | BD2 | 3.52 | 8.8 |
| Example 17 | Compound 64 | BD2 | 3.33 | 8.4 |
| Example 18 | Compound 70 | BD2 | 3.65 | 8.6 |
| Example 19 | Compound 73 | BD2 | 3.75 | 8.7 |
| Example 20 | Compound 79 | A31 | 3.70 | 8.5 |
| Example 21 | Compound 80 | A31 | 3.78 | 8.6 |
| Example 22 | Compound 81 | A62 | 3.55 | 8.7 |
| Example 23 | Compound 82 | A145 | 3.60 | 8.9 |
| Example 24 | Compound 83 | A62 | 3.71 | 8.6 |
| Example 25 | Compound 84 | A80 | 3.68 | 8.6 |
| Example 26 | Compound 85 | A180 | 3.70 | 8.6 |
| Example 27 | Compound 86 | A181 | 3.67 | 8.7 |
| Example 28 | Compound 87 | A153 | 3.68 | 8.5 |
| Example 29 | Compound 88 | A179 | 3.61 | 8.6 |
| Example 30 | Compound 89 | A62 | 3.66 | 8.5 |
| Example 31 | Compound 90 | A147 | 3.65 | 8.7 |
| Example 32 | Compound 91 | A145 | 3.58 | 8.7 |
| Example 33 | Compound 92 | A146 | 3.66 | 8.5 |
| Example 34 | Compound 93 | A181 | 3.71 | 8.6 |
| Example 35 | Compound 94 | A150 | 3.67 | 9.1 |
| Example 36 | Compound 95 | A31 | 3.63 | 8.6 |
| Example 37 | Compound 96 | A185 | 3.69 | 8.5 |
| Example 38 | Compound 97 | A145 | 3.68 | 9.3 |
| Example 39 | Compound 98 | A179 | 3.42 | 8.4 |
| Example 40 | Compound 99 | A205 | 3.17 | 8.3 |
| Example 41 | Compound 100 | A206 | 3.73 | 8.7 |
| Example 42 | Compound 101 | A207 | 3.78 | 8.7 |
| Example 43 | Compound 102 | A208 | 3.67 | 8.4 |
| Example 44 | Compound 104 | A209 | 3.58 | 8.5 |
| Example 45 | Compound 105 | A210 | 3.62 | 8.5 |
| Example 46 | Compound 107 | A211 | 3.80 | 8.4 |
| Comparative Example 6 | Compound 31 | BD3 | 3.65 | 8.3 |
| Comparative Example 7 | Compound 79 | BD3 | 3.86 | 8.1 |
| Comparative Example 8 | Compound 82 | BD3 | 3.77 | 8.3 |
| Comparative Example 9 | Compound 89 | BD3 | 3.85 | 8.0 |
| Comparative Example 10 | Compound 93 | BD3 | 3.80 | 8.0 |
| Comparative Example 11 | Compound 96 | BD3 | 3.84 | 8.1 |
| Comparative Example 12 | Compound 98 | BD3 | 3.61 | 7.8 |
| Comparative Example 13 | Compound 100 | BD3 | 3.90 | 8.3 |
| Comparative Example 14 | Compound 101 | BD3 | 3.97 | 8.2 |
| Comparative Example 15 | Compound 104 | BD3 | 3.79 | 7.9 |
| Comparative Example 16 | Compound 105 | BD3 | 3.82 | 8.0 |
| Comparative Example 17 | Compound 108 | BD3 | 3.91 | 7.5 |
| Comparative Example 18 | Compound 121 | BD3 | 3.85 | 8.2 |
| Comparative Example 19 | Compound 122 | BD3 | 3.55 | 8.2 |
| Comparative Example 20 | Compound 123 | BD3 | 3.60 | 8.1 |
| Comparative Example 21 | BH5 | BD3 | 4.08 | 8.3 |

**[Table 3]**

| Item | Host | Dopant | Voltage(V) | External quantum efficiency(EQE) |
|---|---|---|---|---|
| Example 47 | Compound 82 | B92 | 3.66 | 8.8 |
| Example 48 | Compound 82 | B106 | 3.63 | 8.5 |
| Example 49 | Compound 82 | B112 | 3.61 | 8.8 |
| Example 50 | Compound 94 | B121 | 3.67 | 8.9 |
| Example 51 | Compound 97 | B92 | 3.66 | 9.2 |
| Comparative Example 22 | Compound 31 | BD3 | 3.65 | 8.3 |
| Comparative Example 23 | Compound 79 | BD3 | 3.86 | 8.1 |
| Comparative Example 24 | Compound 82 | BD3 | 3.77 | 8.3 |
| Comparative Example 25 | Compound 89 | BD3 | 3.85 | 8.0 |
| Comparative Example 26 | Compound 93 | BD3 | 3.80 | 8.0 |
| Comparative Example 27 | Compound 98 | BD3 | 3.61 | 7.8 |
| Comparative Example 28 | Compound 100 | BD3 | 3.9 | 8.3 |
| Comparative Example 29 | Compound 101 | BD3 | 3.97 | 8.2 |
| Comparative Example 30 | Compound 104 | BD3 | 3.79 | 7.9 |
| Comparative Example 31 | Compound 105 | BD3 | 3.82 | 8.0 |
| Comparative Example 32 | Compound 108 | BD3 | 3.91 | 7.5 |

**[Table 4]**

| Item | Host | Dopant | Voltage (V) | External quantum efficiency(EQE) | T95 |
|---|---|---|---|---|---|
| Example 52 | Compound 82 | A145 | 3.60 | 8.9 | 252 |
| Example 53 | Compound 94 | A150 | 3.67 | 9.1 | 282 |
| Example 54 | Compound 97 | A145 | 3.68 | 9.3 | 225 |
| Comparative Example 33 | Compound 82 | BD3 | 3.77 | 8.3 | 153 |
| Comparative Example 34 | Compound 82 | BD1 | 3.75 | 8.0 | 159 |
| Comparative Example 35 | Compound 94 | BD3 | 3.86 | 8.5 | 175 |
| Comparative Example 36 | Compound 94 | BD1 | 3.83 | 8.1 | 169 |
| Comparative Example 37 | Compound 97 | BD3 | 3.89 | 8.7 | 138 |
| Comparative Example 38 | Compound 97 | BD1 | 3.85 | 8.3 | 141 |

As can be seen from [Table 1] to [Table 4] above, the organic light-emitting device including the anthracene derivative having a characteristic structure represented by [Formula I] as a host compound in the light-emitting layer according to the present invention has excellent external quantum efficiency and improved low voltage driving characteristics compared to an organic light-emitting device including a conventional anthracene-based host compound having a different characteristic structure from the anthracene derivative according to the present invention.

In addition, the organic light-emitting device including an anthracene derivative having a characteristic structure represented by [Formula I] as a host compound and the compound represented by [Formula A-1], [Formula A-2], [Formula B], or [Formula C] as a dopant in the light-emitting layer has much better external quantum efficiency and improved low voltage driving characteristics.

In addition, the organic light-emitting device including both an anthracene derivative having a characteristic structure represented by [Formula I] as a host compound and the compound represented by [Formula A-1] or [Formula A-2] as a dopant in the light-emitting layer has improved low voltage driving characteristics, excellent external quantum efficiency and remarkably lengthened lifespan.

The organic light-emitting device according to the present invention is capable of realizing improved low voltage driving using an anthracene derivative having a characteristic structure as a host and a polycyclic aromatic derivative compound as a dopant in a light-emitting layer, thus being useful for various display devices such as flat panel, flexible, and wearable displays as well as lighting devices.

## Claims

1. An organic light-emitting device comprising:
a first electrode;
a second electrode facing the first electrode; and
a light-emitting layer interposed between the first electrode and the second electrode,
wherein the light-emitting layer comprises a compound represented by the following [Formula I], and
wherein the light-emitting layer further comprises a compound represented by the following [Formula B-1]: 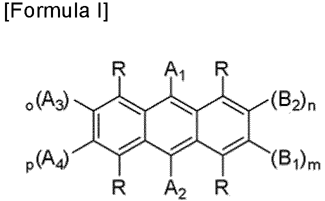 wherein
A₁ to A₄, B₁ and B₂ are identical to or different from each other and are each independently substituted or unsubstituted dibenzofuran, substituted or unsubstituted dibenzothiophene, or substituted or unsubstituted C6-C30 aromatic hydrocarbon;
m, n, o, and p are each independently an integer from 0 to 1, with the proviso that m+n+o+p is an integer from 1 to 3; and
R's are identical to or different from each other and are each independently selected from hydrogen, deuterium, a substituted or unsubstituted C1-C30 alkyl group, a substituted or unsubstituted C3-C30 cycloalkyl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylsilyl group, and a substituted or unsubstituted C6-C30 arylsilyl group,
wherein the compound of [Formula I] comprises at least one of dibenzofuran or dibenzothiophene 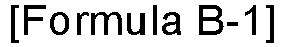 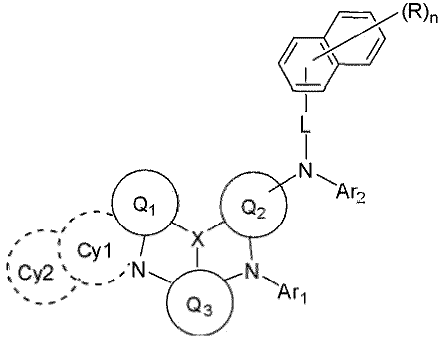 wherein
Q1 to Q3 are identical to or different from each other and are each independently a substituted or unsubstituted C6-C50 aromatic hydrocarbon ring, or a substituted or unsubstituted C2-C50 aromatic heterocyclic ring;
X is selected from B, P and P=O,
Cy1 is linked to the nitrogen (N) atom and an aromatic carbon atom in the Q1 ring to form a fused ring and the formed Cy1 ring is a substituted or unsubstituted C1-C10 alkylene group, provided that the nitrogen (N) atom, the aromatic carbon atom in the Q1 ring to which the nitrogen (N) atom is bonded, and the aromatic carbon atom in the Q1 ring to which the Cy1 ring is bonded are excluded;
Cy2 is added to Cy1 to form a saturated hydrocarbon ring and the formed Cy2 ring is a substituted or unsubstituted C1-C10 alkylene group, provided that carbon atoms included in Cy1 are excluded;
Ar1 and R are each independently selected from hydrogen, deuterium, a substituted or unsubstituted C1-C30 linear, branched or cyclic alkyl group, a substituted or unsubstituted C1-C30 halogenated linear, branched or cyclic alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C6-C30 arylsilyl group, a nitro group, a cyano group, and a halogen group; and
Ar2's are identical to or different from each other and are each independently a substituted or unsubstituted C6-C50 aryl group, or a substituted or unsubstituted C2-C50 heteroaryl group,
wherein each of Ar1 is optionally bonded to Q2 or Q3 to further form an alicyclic or aromatic monocyclic or polycyclic ring,
n is an integer from 1 to 7, wherein R's are identical to or different from each other provided that n is 2 or more; and
L is a single bond or is selected from a substituted or unsubstituted C6-C30 arylene group or a substituted or unsubstituted C1-C30 heteroarylene group.

2. The organic light-emitting device according to claim 1, wherein the compound of [Formula I] is selected from the compounds represented by the following [Formula 1] to [Formula 123]:

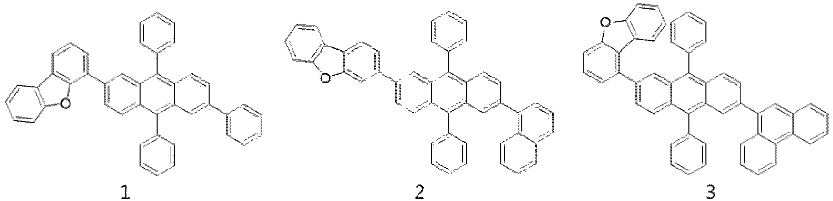

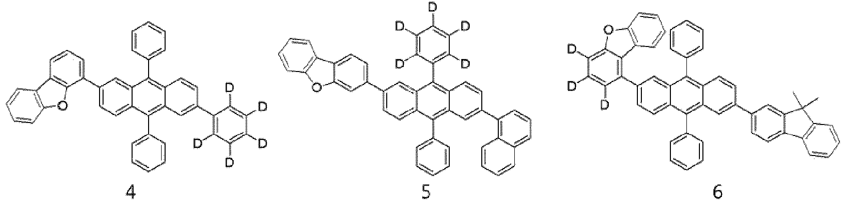

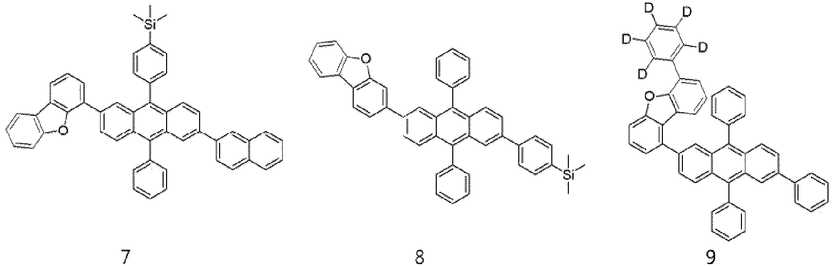

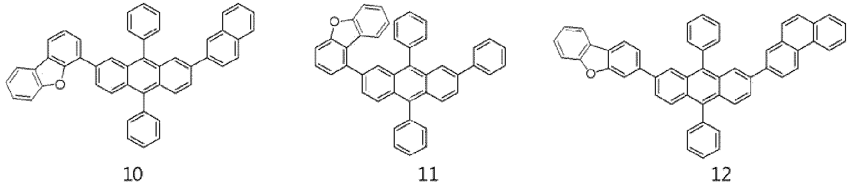

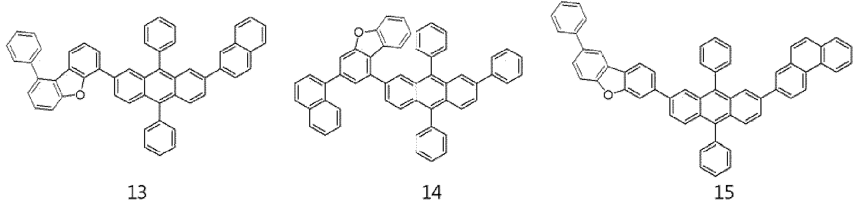

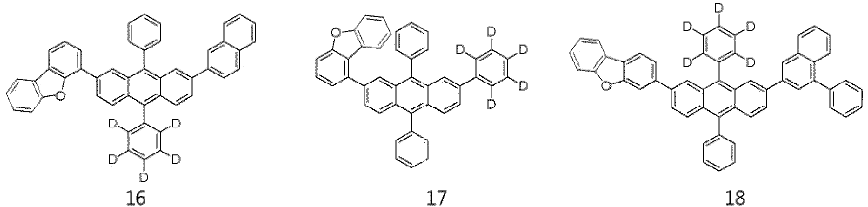

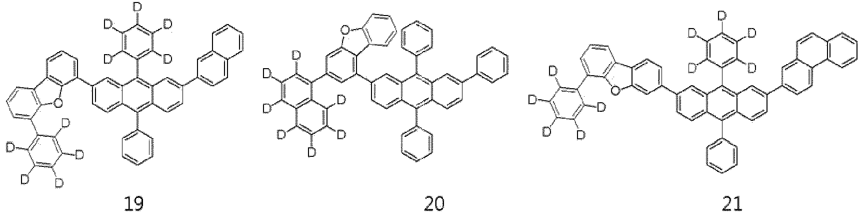

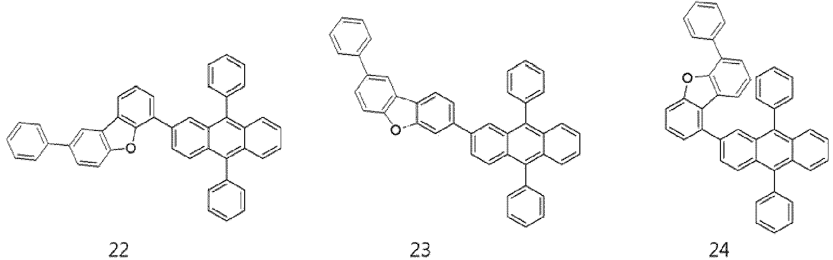

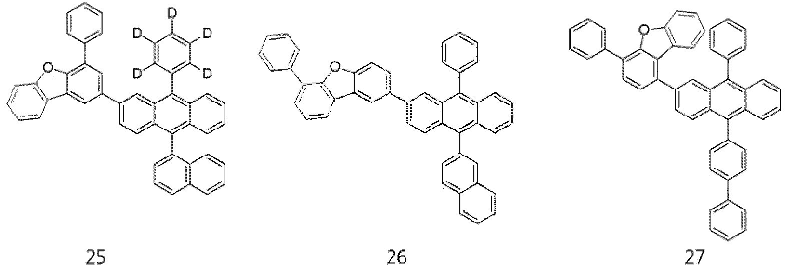

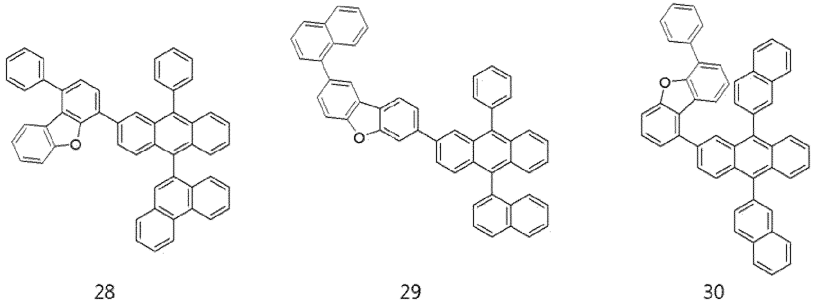

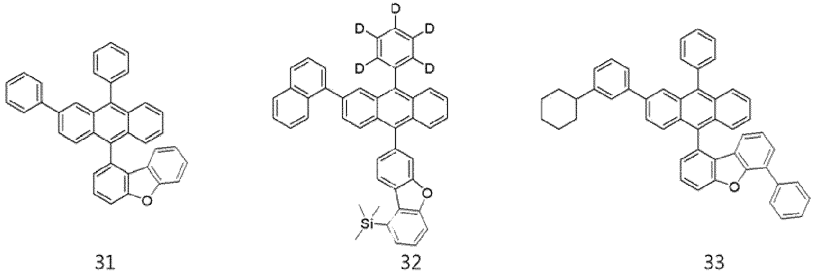

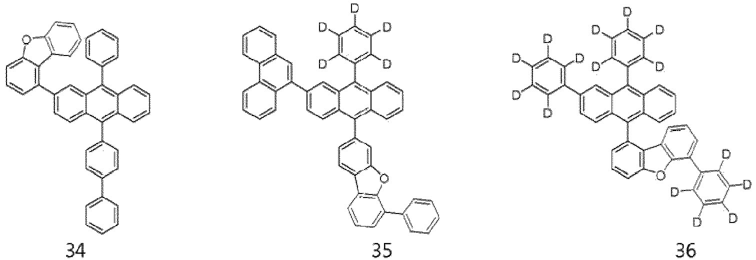

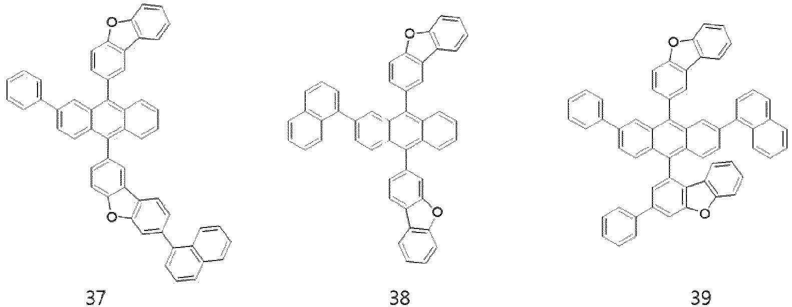

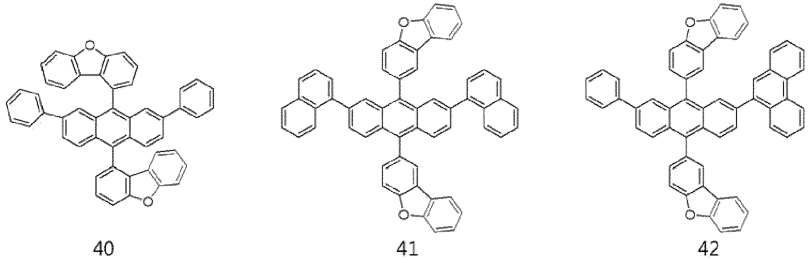

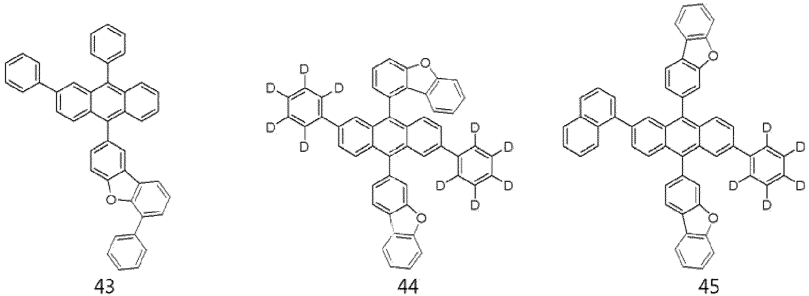

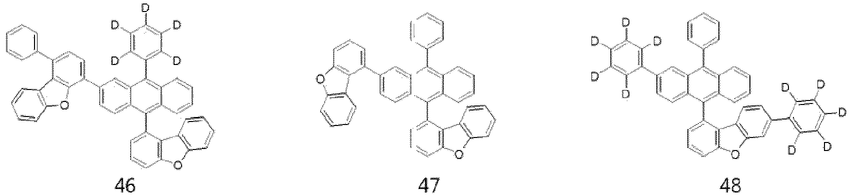

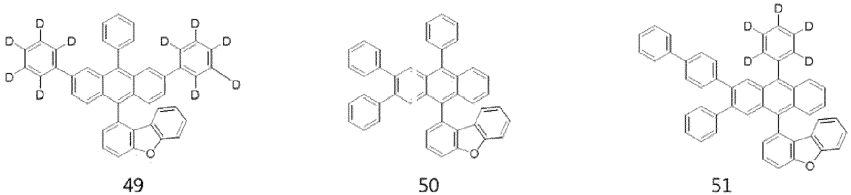

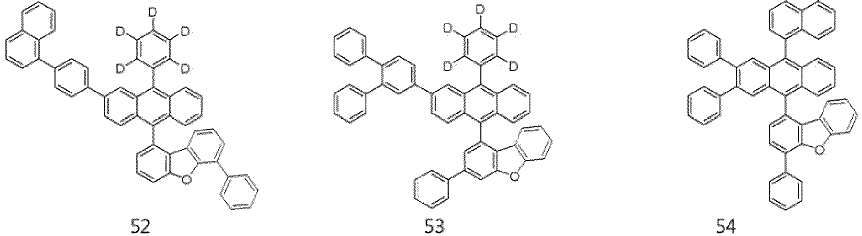

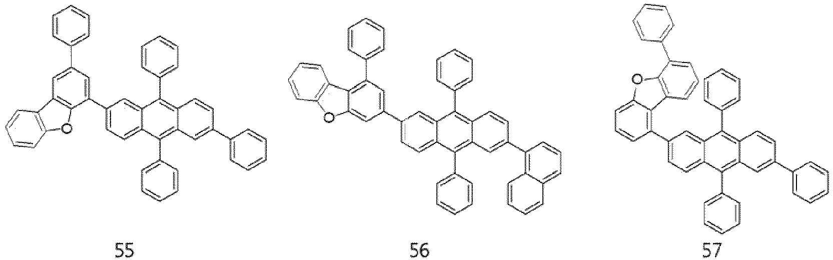

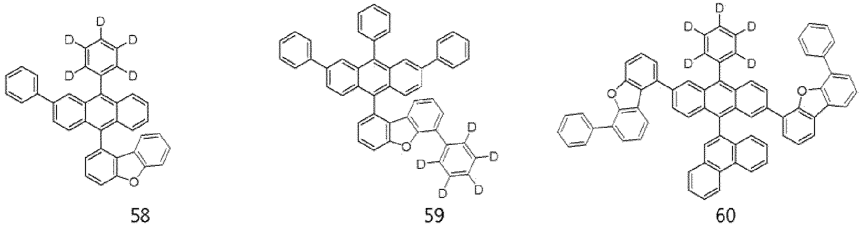

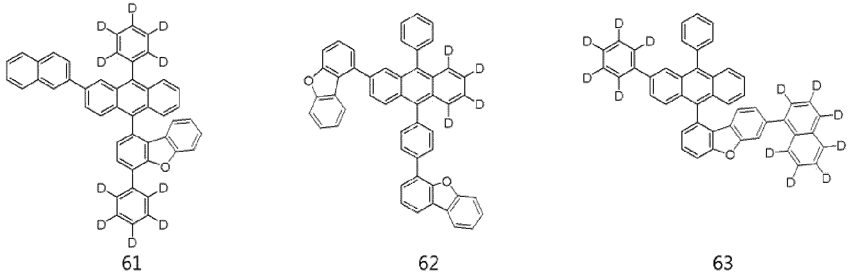

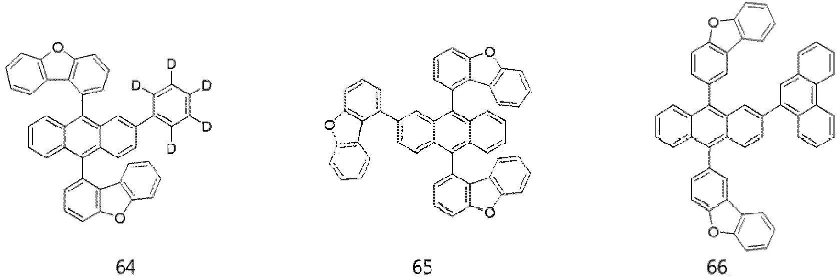

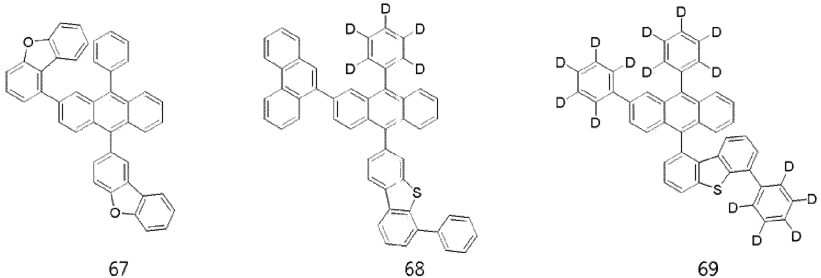

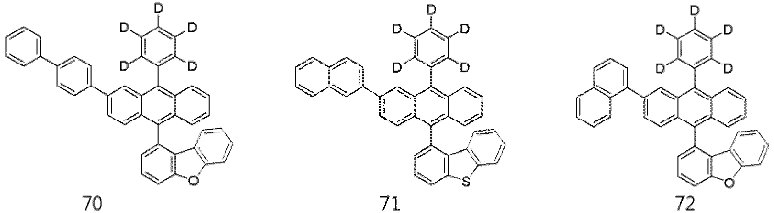

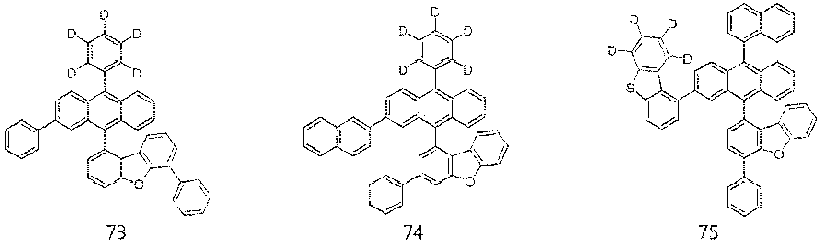

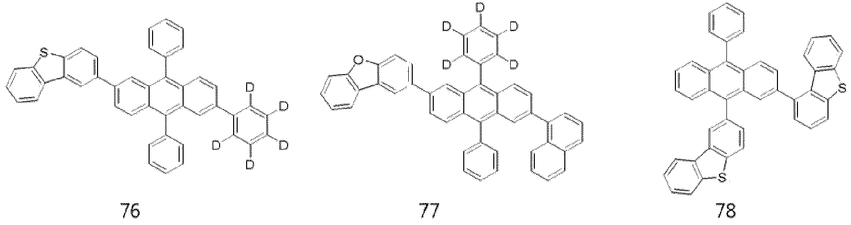

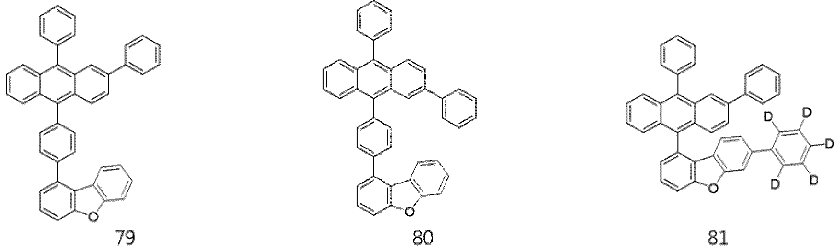

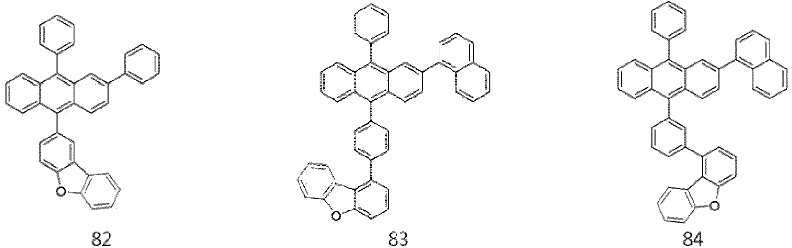

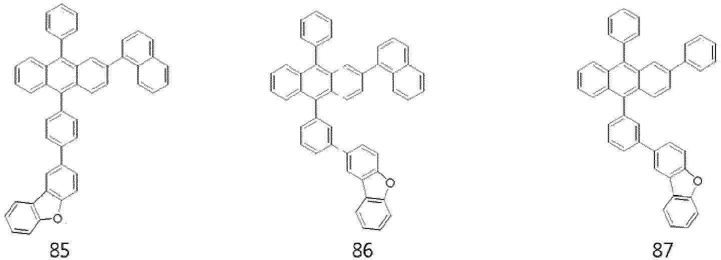

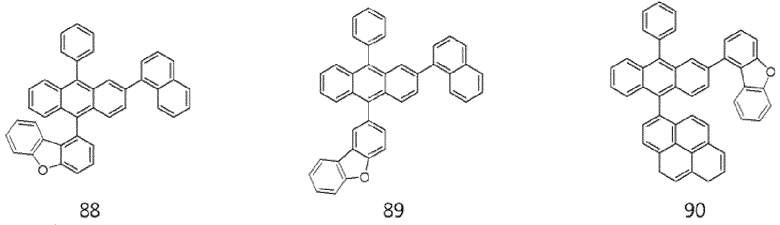

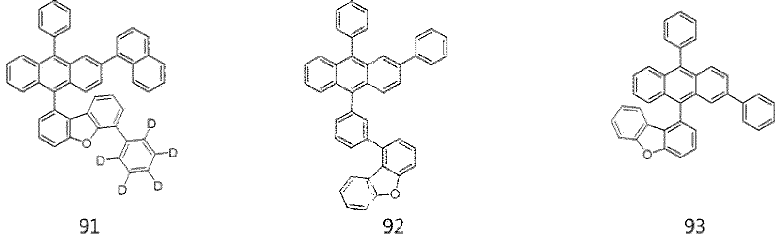

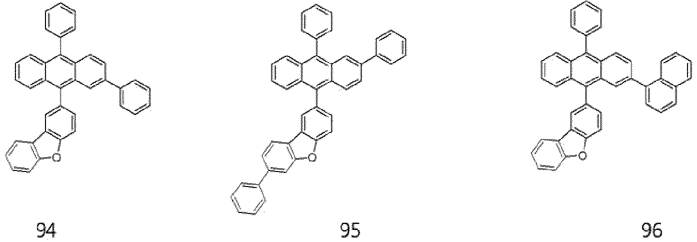

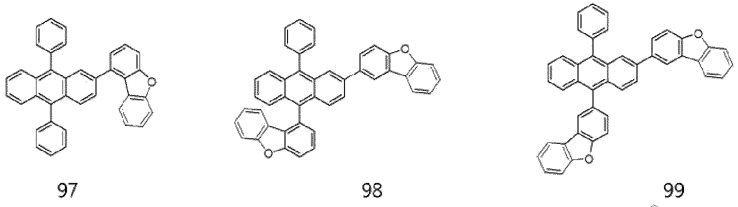

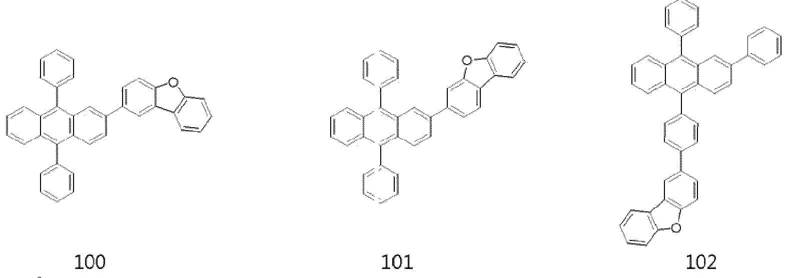

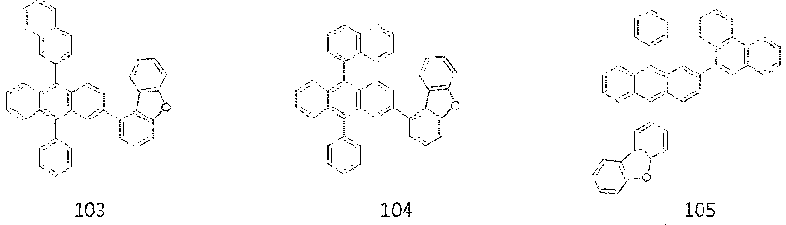

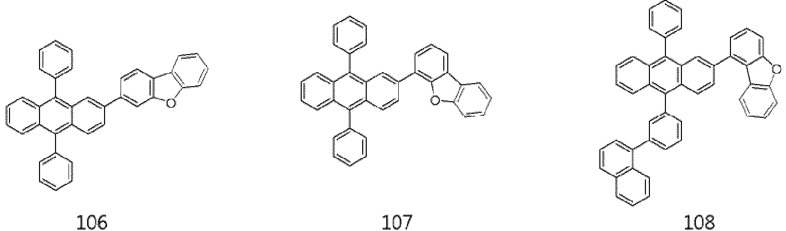

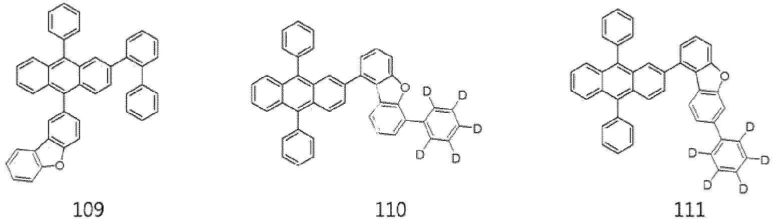

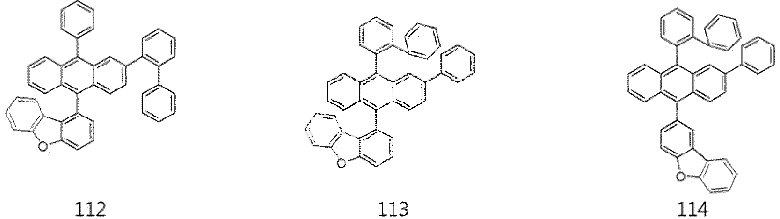

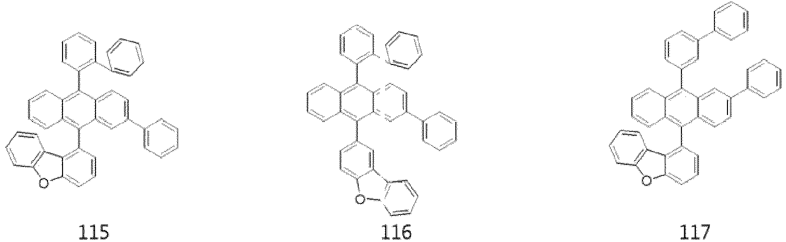

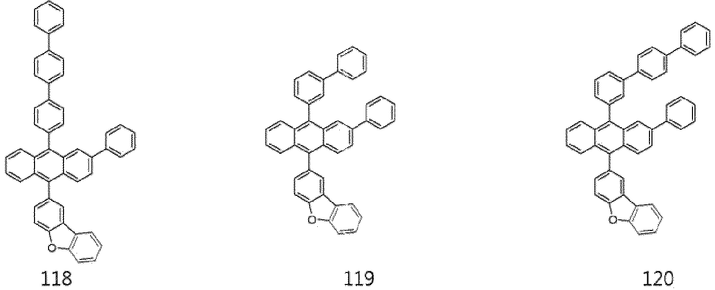

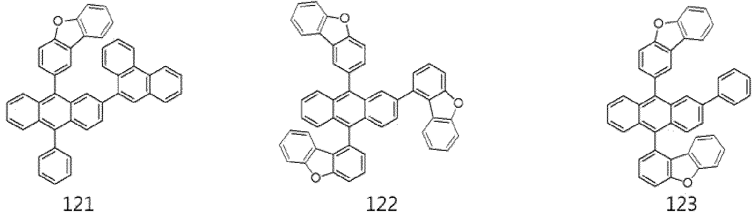

3. The organic light-emitting device according to claim 1, wherein the compound of [Formula B-1] is selected from the compounds represented by the following [Formula B91] to [Formula B105], and [Formula B112] to [Formula B135]:

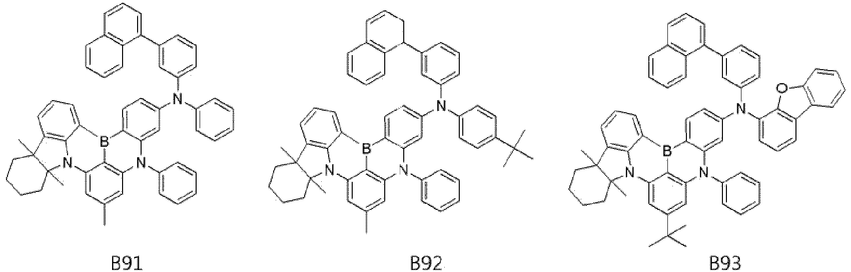

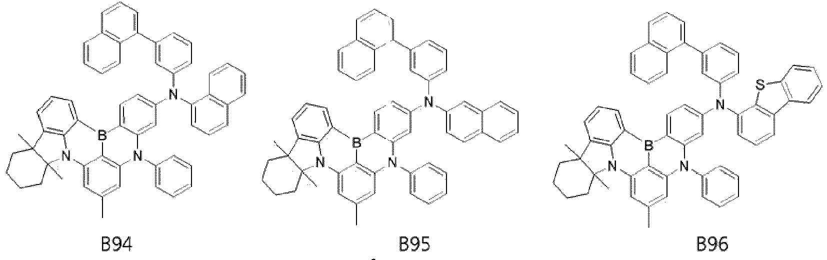

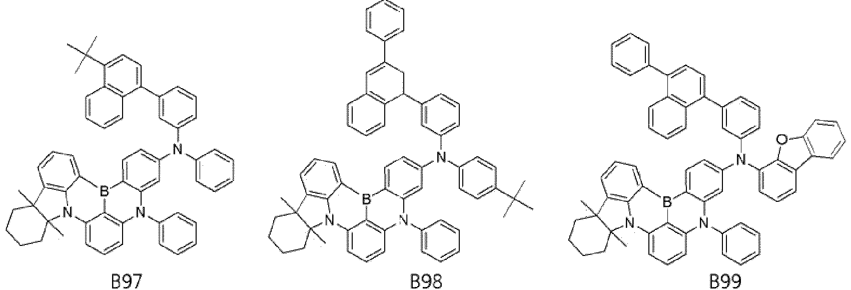

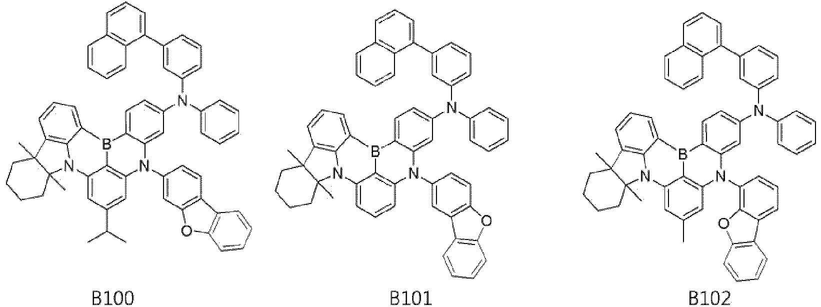

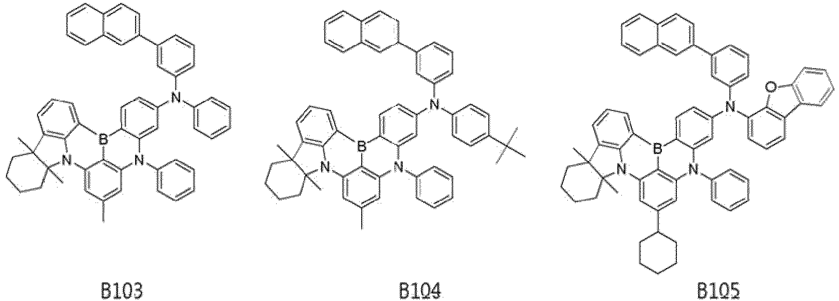

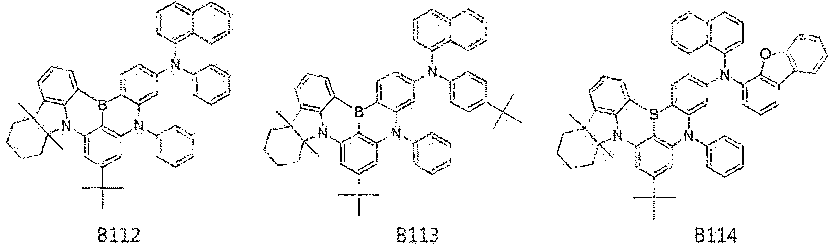

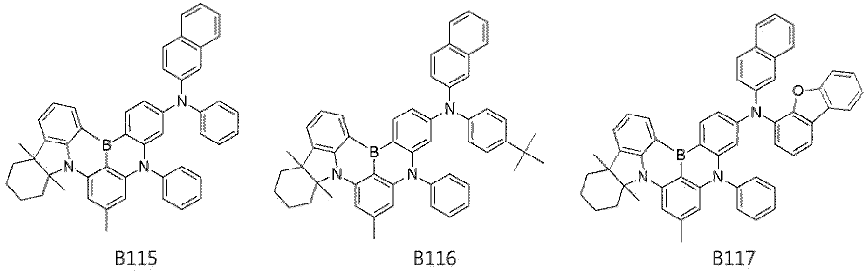

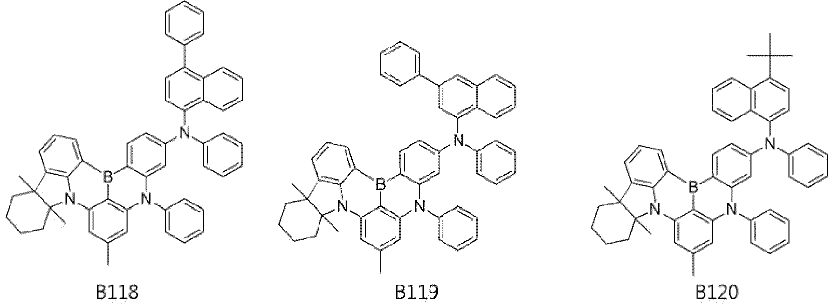

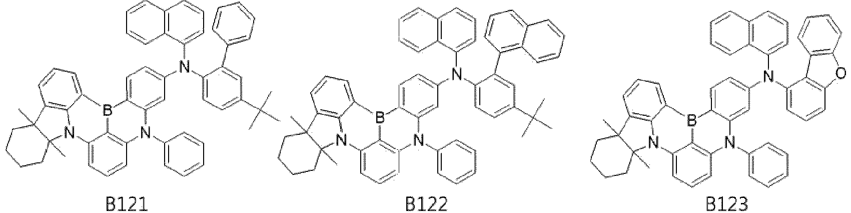

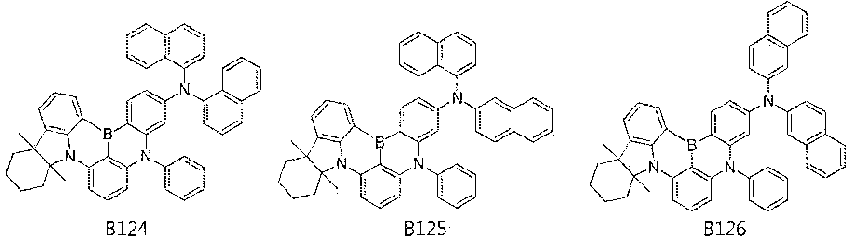

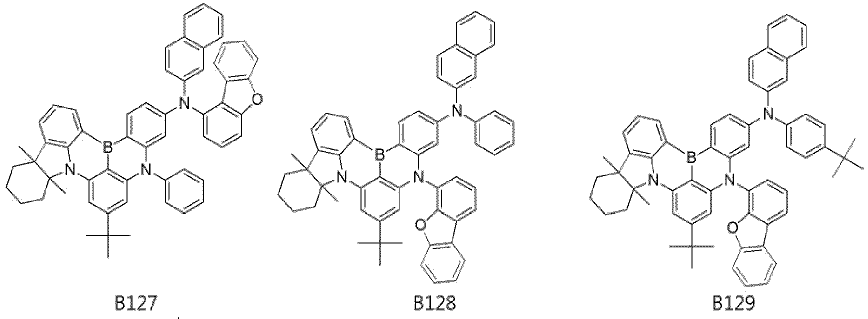

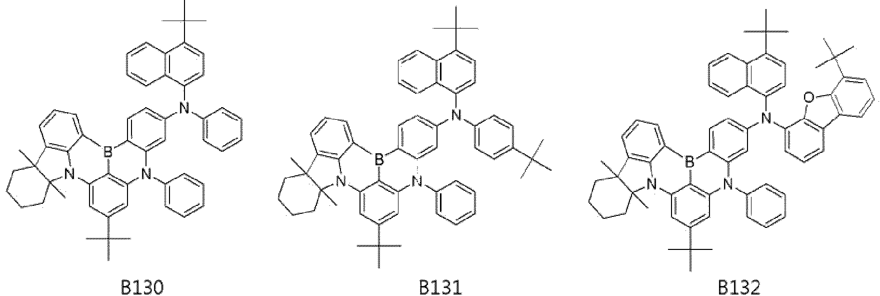

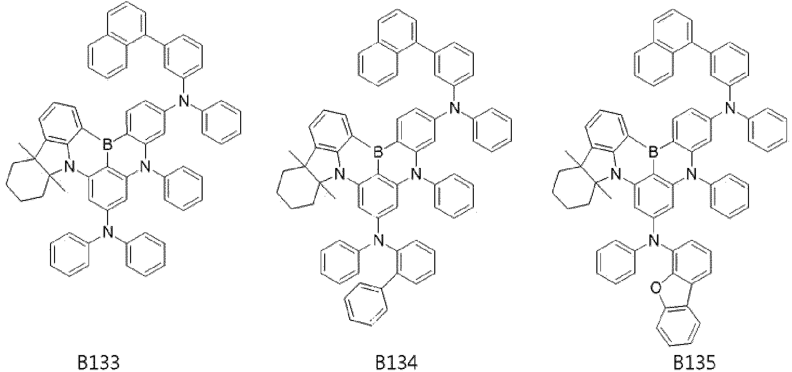

## Patentansprüche

1. Eine organische Leuchtvorrichtung, umfassend:
eine erste Elektrode;
eine zweite Elektrode, die der ersten Elektrode gegenüberliegt; und
eine zwischen der ersten Elektrode und der zweiten Elektrode angeordnete lichtemittierende Schicht,
wobei die lichtemittierende Schicht eine Verbindung umfasst, die durch die folgende [Formel I] dargestellt wird, und
wobei die lichtemittierende Schicht ferner eine Verbindung umfasst, die durch die folgende [Formel B-1] dargestellt wird: 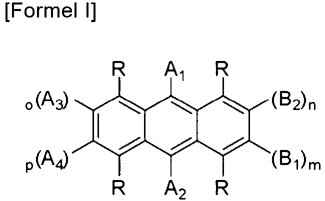 wobei
A₁ bis A₄ , B₁ und B₂ identisch oder voneinander verschieden sind und jeweils unabhängig voneinander substituiertes oder unsubstituiertes Dibenzofuran, substituiertes oder unsubstituiertes Dibenzothiophen oder substituiertes oder unsubstituiertes C6-C30-aromatisches Kohlenwasserstoff sind;
m, n, o und p jeweils unabhängig voneinander eine ganze Zahl von 0 bis 1 sind, mit der Maßgabe, dass m+n+o+p eine ganze Zahl von 1 bis 3 ist; und
die Reste R identisch oder voneinander verschieden sind und jeweils unabhängig voneinander aus Wasserstoff, Deuterium, einer substituierten oder unsubstituierten C1-C30-Alkylgruppe, einer substituierten oder unsubstituierten C3-C30-Cycloalkylgruppe, einer substituierten oder unsubstituierten C1-C30-Alkoxygruppe, einer substituierten oder unsubstituierten C6-C30-Aryloxygruppe, einer substituierten oder unsubstituierten C1-C30-Alkylthioxygruppe, einer substituierten oder unsubstituierten C5-C30-Arylthioxygruppe, einer substituierten oder unsubstituierten C1-C30-Alkylsilylgruppe und einer substituierten oder unsubstituierten C6-C30-Arylsilylgruppe ausgewählt sind,
wobei die Verbindung der [Formel I] mindestens ein Dibenzofuran oder ein Dibenzothiophen enthält 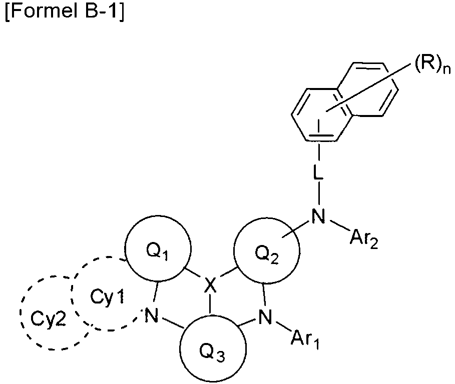 wobei
Q1 bis Q3 identisch oder voneinander verschieden sind und jeweils unabhängig voneinander ein substituierter oder unsubstituierter aromatischer C6-C50-Kohlenwasserstoffring oder ein substituierter oder unsubstituierter aromatischer heterocyclischer C2-C50-Ring sind;
X ausgewählt ist aus B, P und P=O,
wobei Cy1 an das Stickstoffatom (N) und an ein aromatisches Kohlenstoffatom im Q1-Ring gebunden ist, um einen kondensierten Ring zu bilden, und der gebildete Cy1-Ring eine substituierte oder unsubstituierte C1-C10-Alkylengruppe ist, mit der Maßgabe, dass das Stickstoffatom (N), das aromatische Kohlenstoffatom im Q1-Ring, an das das Stickstoffatom (N) gebunden ist, und das aromatische Kohlenstoffatom im Q1-Ring, an das der Cy1-Ring gebunden ist, ausgeschlossen sind;
Cy2 ist an Cy1 angebracht, um einen gesättigten Kohlenwasserstoffring zu bilden, und der gebildete Cy2-Ring ist eine substituierte oder unsubstituierte C1-C10-Alkylengruppe, unter der Maßgabe, dass in Cy1 enthaltene Kohlenstoffatome ausgeschlossen sind;
Ar1 und R sind jeweils unabhängig voneinander ausgewählt aus Wasserstoff, Deuterium, einer substituierten oder unsubstituierten, linearen, verzweigten oder cyclischen C1-C30-Alkylgruppe, einer substituierten oder unsubstituierten, linearen, verzweigten oder cyclischen C1-C30-halogenierten Alkylgruppe, einer substituierten oder unsubstituierten C6-C50-Arylgruppe, einer substituierten oder unsubstituierten C3-C30-Cycloalkylgruppe, einer substituierten oder unsubstituierten C2-C50-Heteroarylgruppe, einer substituierten oder unsubstituierten C1-C30-Alkoxygruppe, einer substituierten oder unsubstituierten C6-C30-Aryloxygruppe, einer substituierten oder unsubstituierten C1-C30-Alkylthioxygruppe, einer substituierten oder unsubstituierten C5-C30-Arylthioxygruppe, einer substituierten oder unsubstituierten C1-C30-Alkylamin-Gruppe, einer substituierten oder unsubstituierten C5-C30-Arylamin-Gruppe, einer substituierten oder unsubstituierten C1-C30-Alkylsilyl-Gruppe, einer substituierten oder unsubstituierten C6-C30-Arylsilyl-Gruppe, einer Nitro-Gruppe, einre CyanoGruppe und einer Halogen-Gruppe; und
Die Ar2-Gruppen identisch oder voneinander verschieden sind und jeweils unabhängig voneinander eine substituierte oder unsubstituierte C6-C50-Arylgruppe oder eine substituierte oder unsubstituierte C2-C50-Heteroarylgruppe sind,
wobei jedes Ar1 optional an Q2 oder Q3 gebunden ist, um einen alicyclischen oder aromatischen monocyclischen oder polycyclischen Ring zu bilden,
n eine ganze Zahl von 1 bis 7 ist, wobei die Reste R identisch oder voneinander verschieden sind, falls n 2 oder mehr ist; und
L eine Einfachbindung ist oder aus einer substituierten oder unsubstituierten C6-C30-Arylengruppe oder einer substituierten oder unsubstituierten C1-C30-Heteroarylengruppe ausgewählt wird.

2. Die organische Leuchtvorrichtung gemäß Anspruch 1, wobei die Verbindung der [Formel I] aus den durch die folgenden dargestellten Verbindungen [Formel 1] bis [Formel 123] ausgewählt ist:

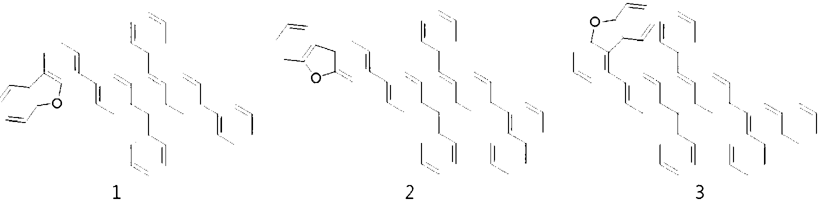

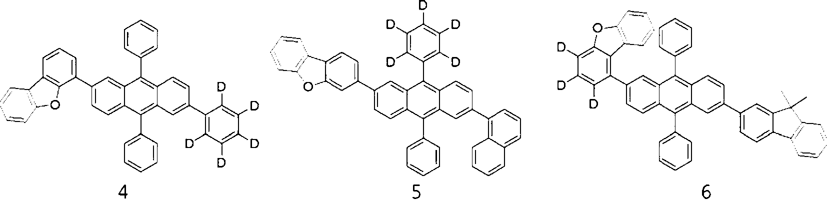

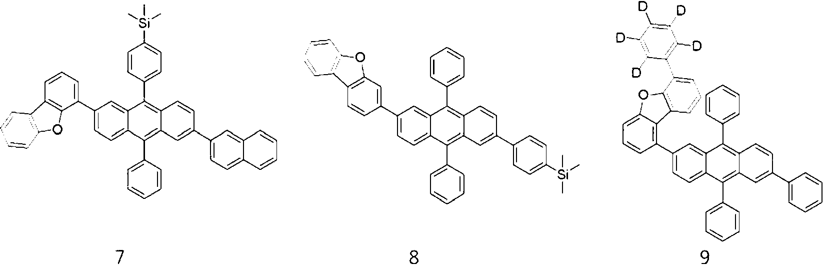

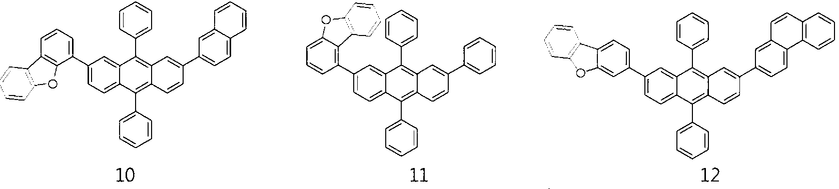

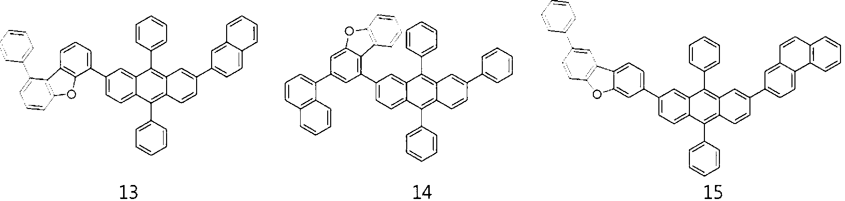

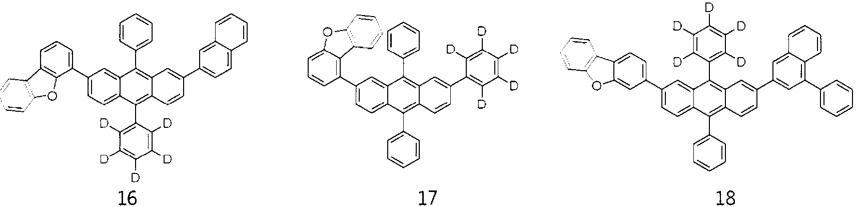

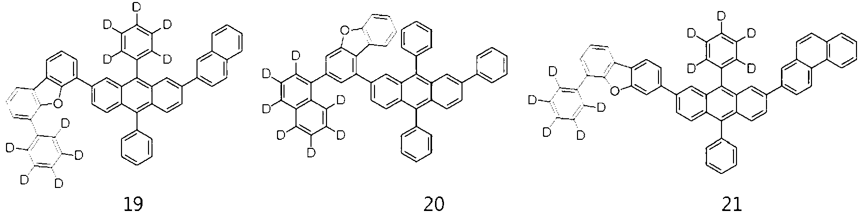

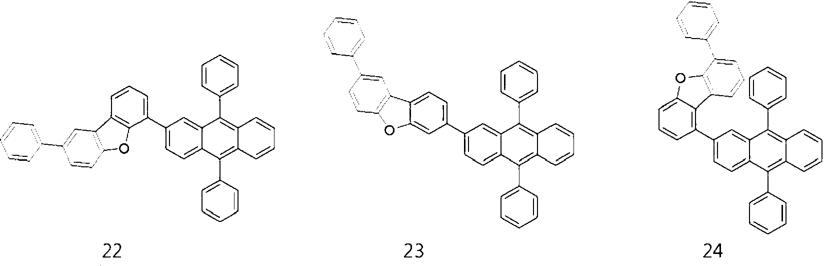

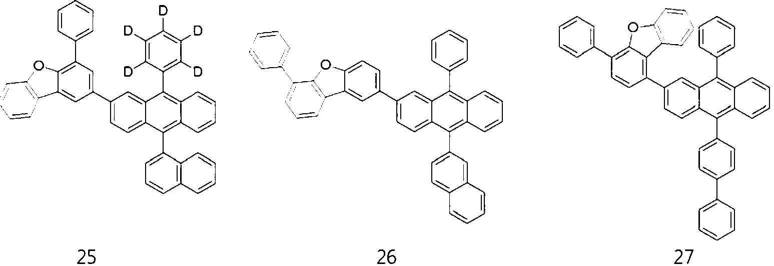

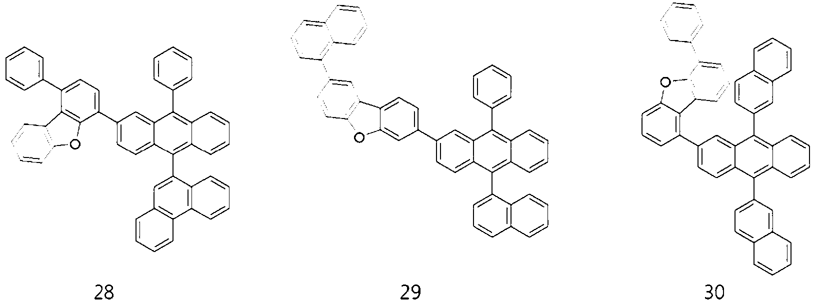

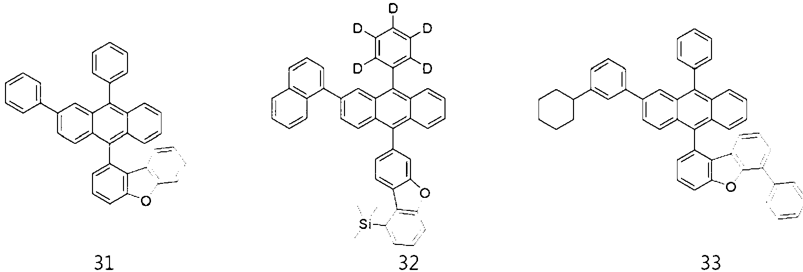

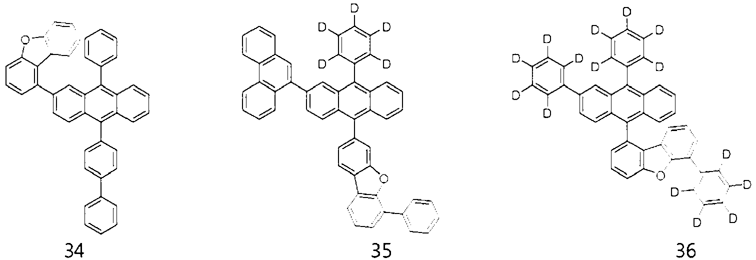

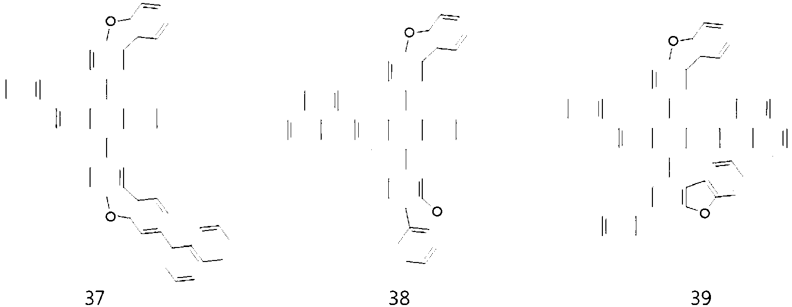

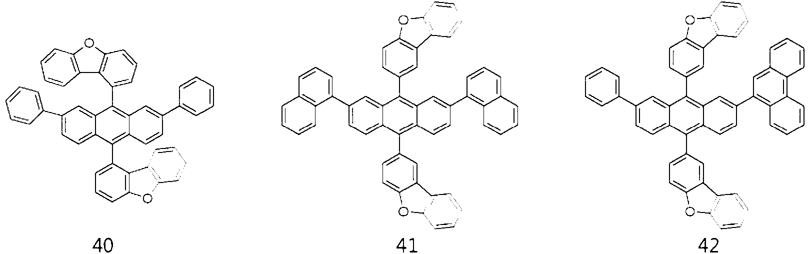

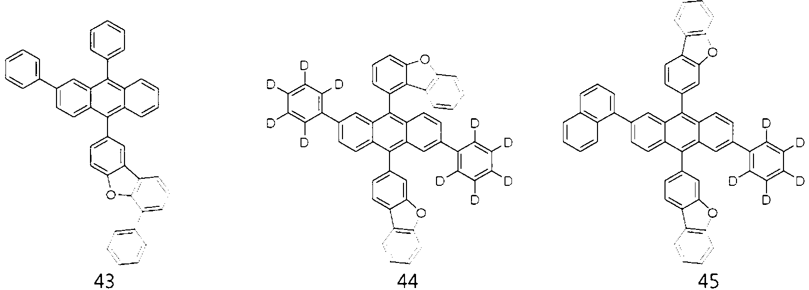

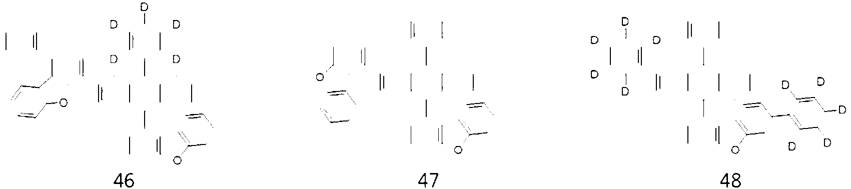

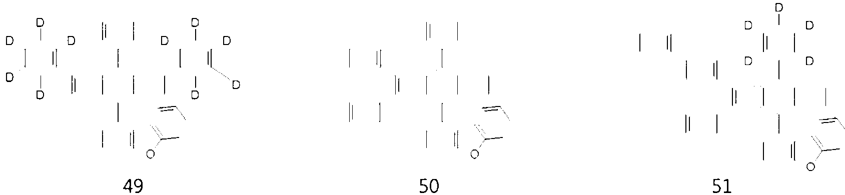

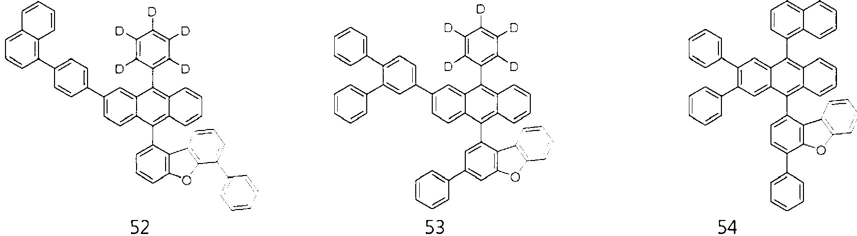

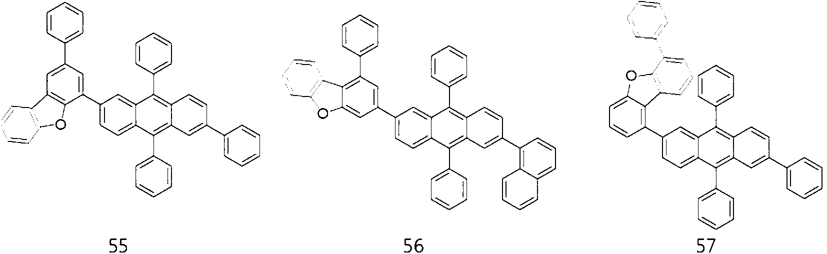

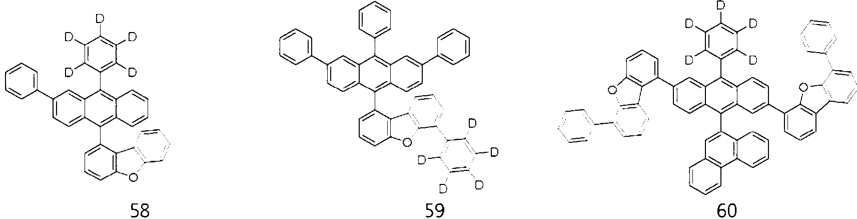

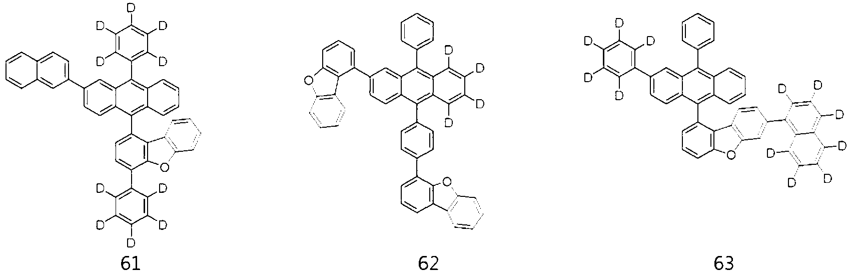

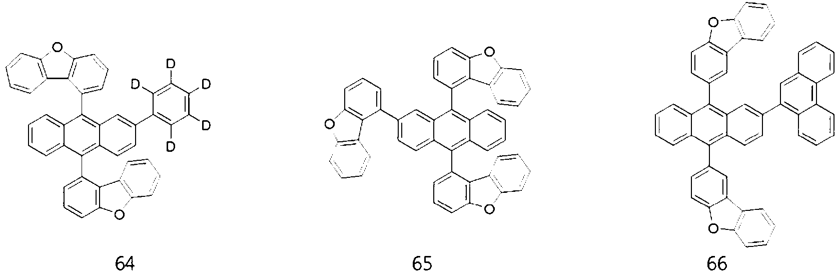

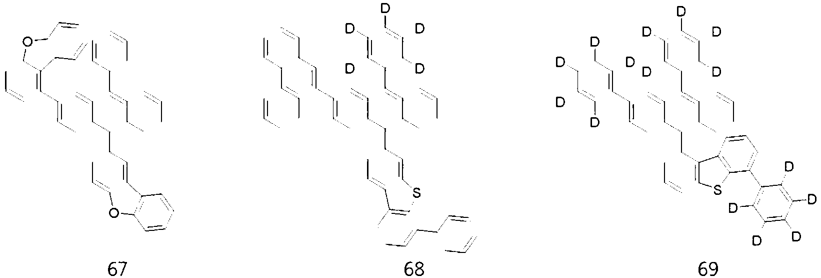

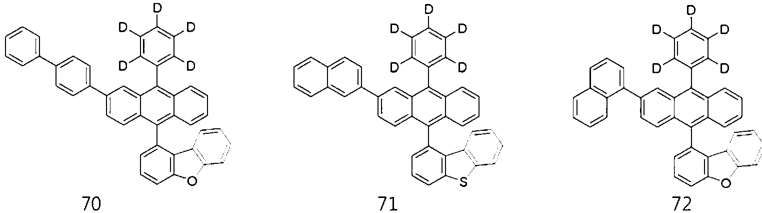

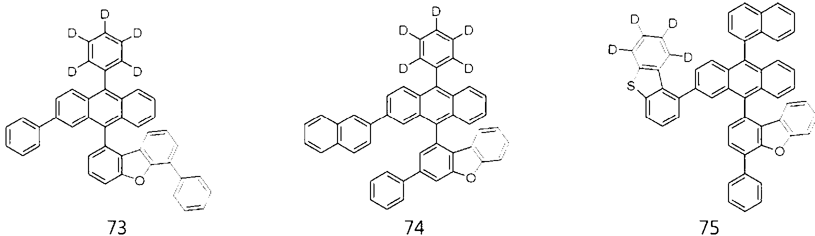

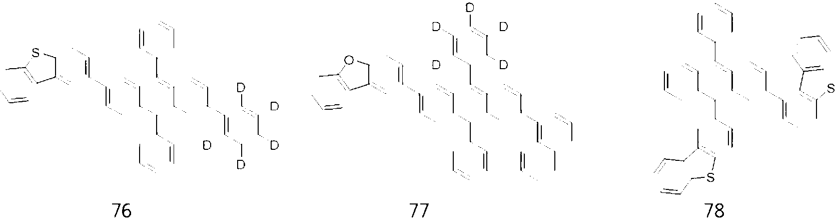

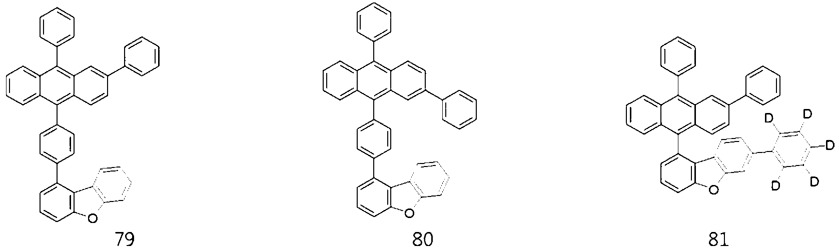

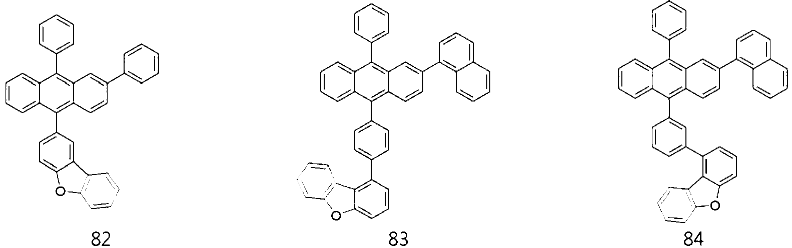

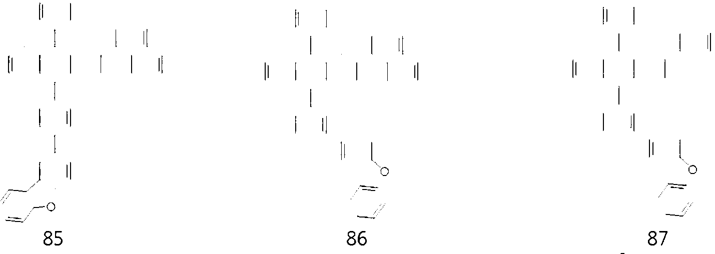

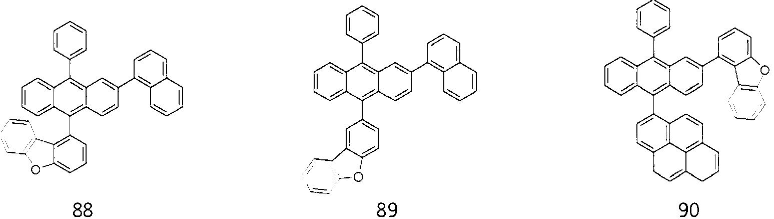

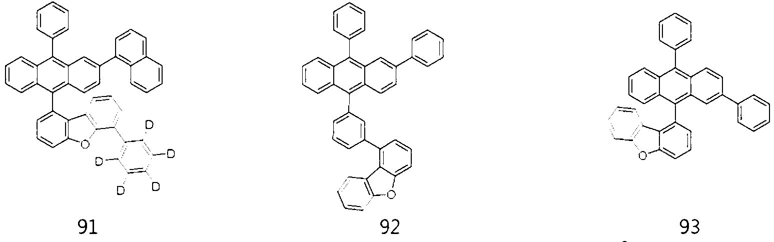

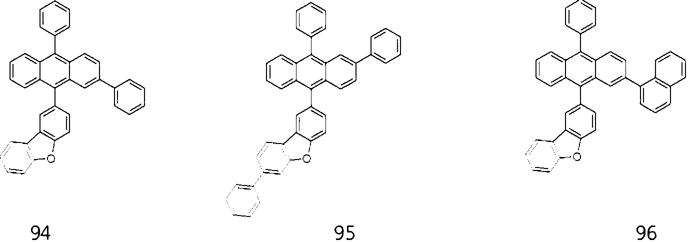

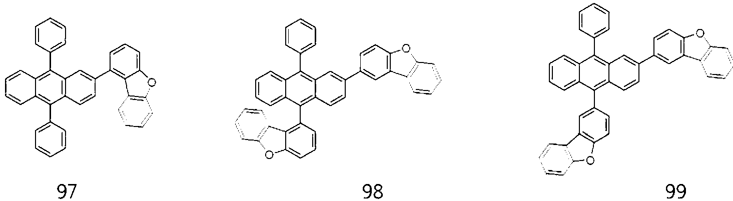

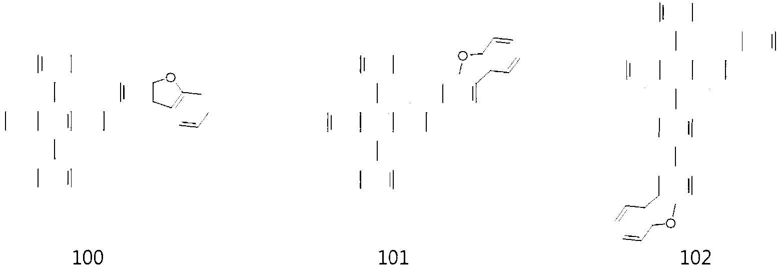

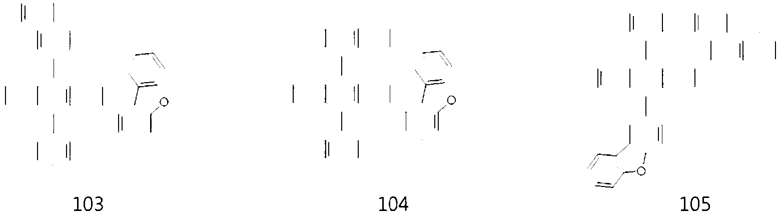

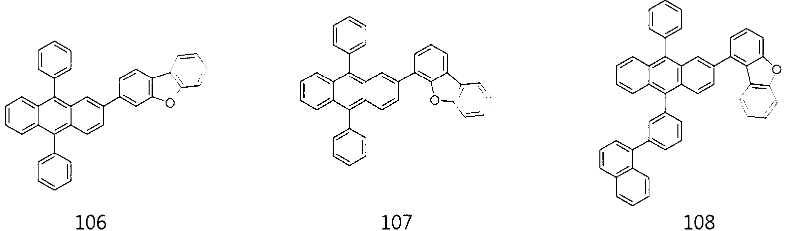

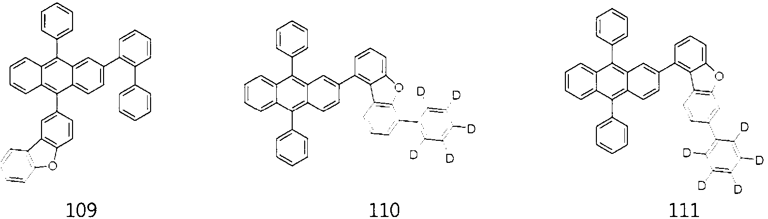

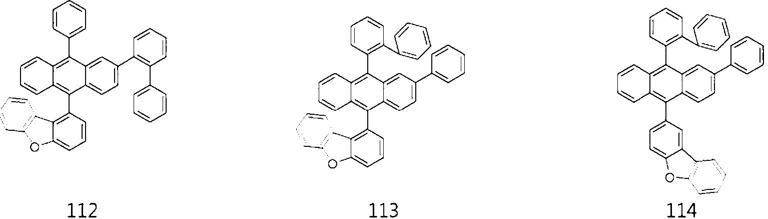

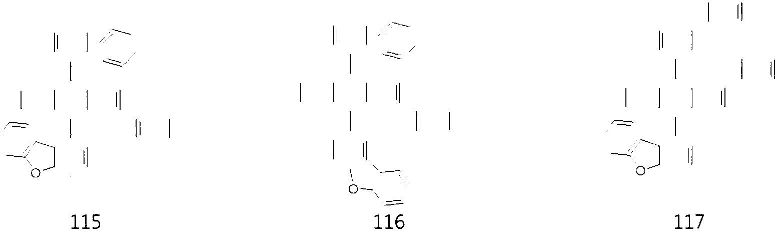

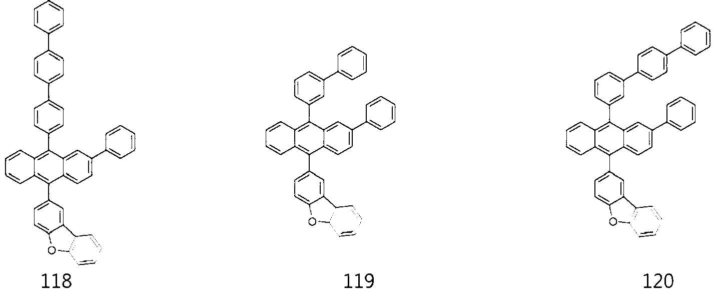

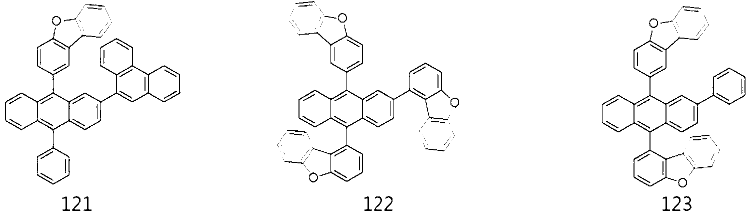

3. Organische Leuchtvorrichtung nach Anspruch 1, wobei die Verbindung der [Formel B-1] aus den durch die folgenden dargestellten Verbindungen [Formel B91] bis [Formel B105] und [Formel B112] bis [Formel B135] ausgewählt ist:

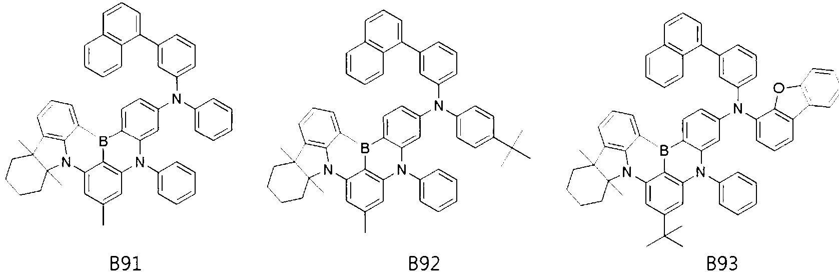

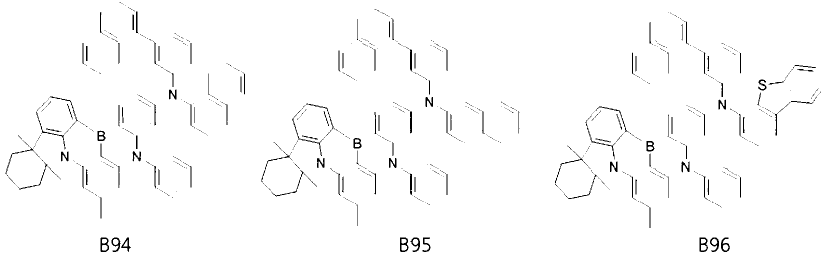

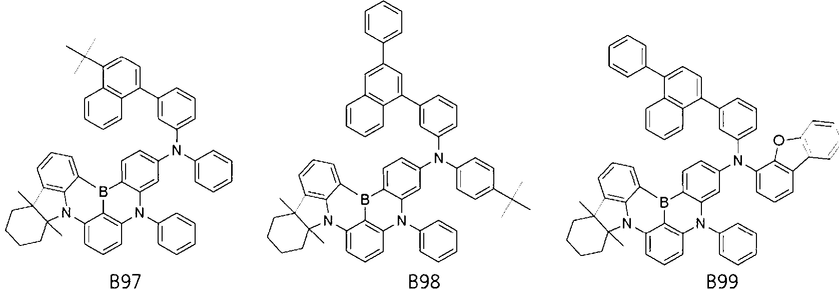

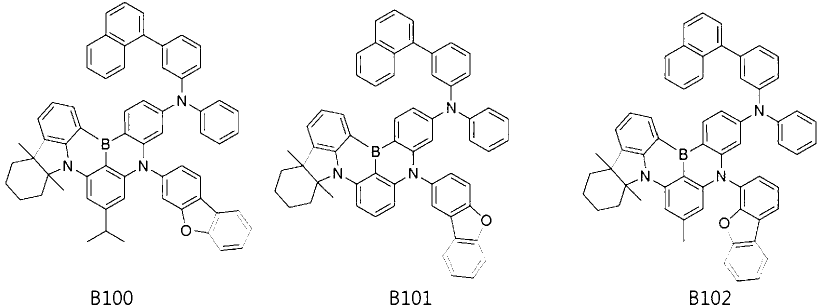

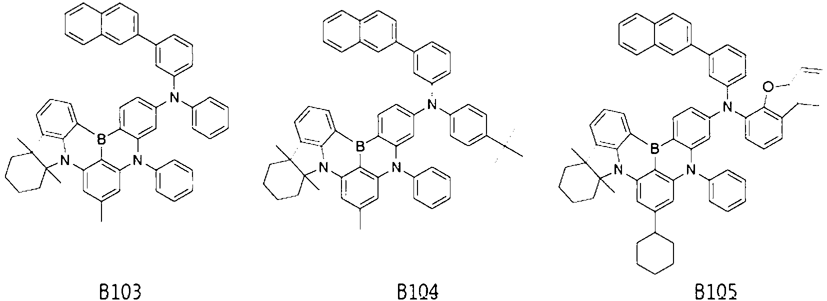

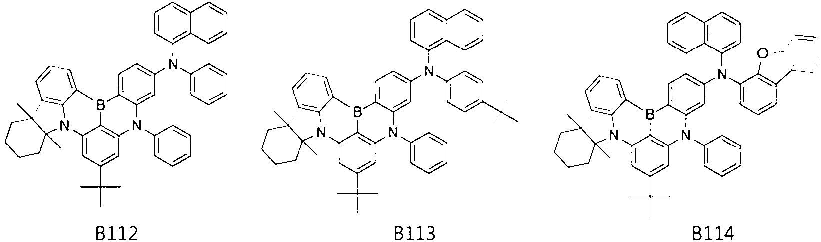

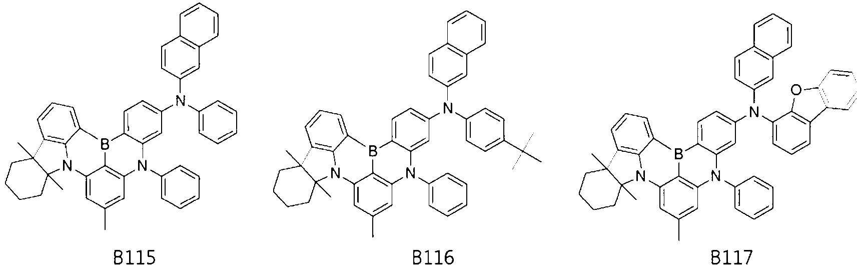

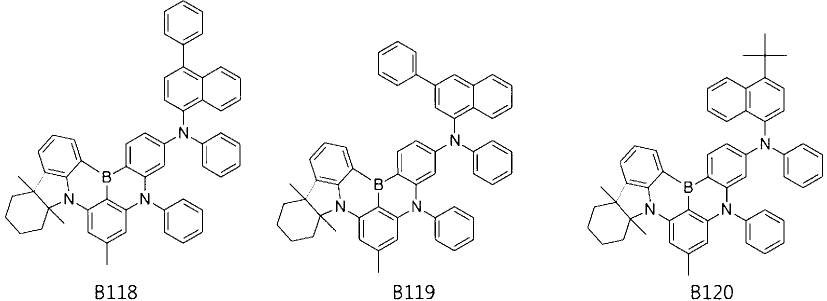

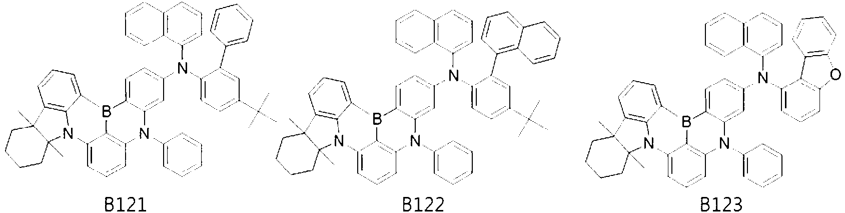

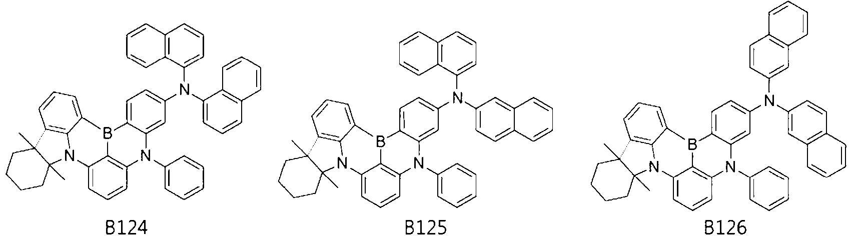

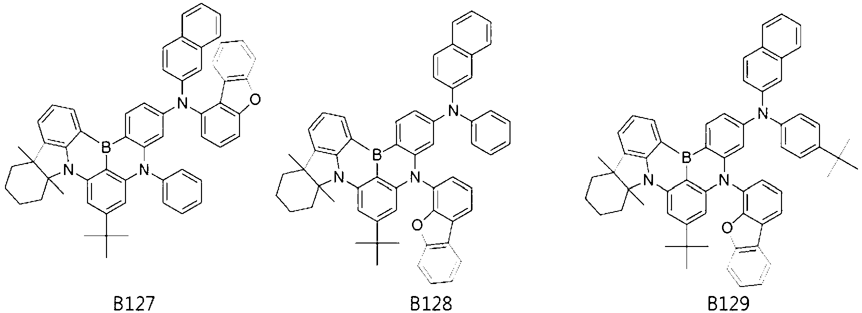

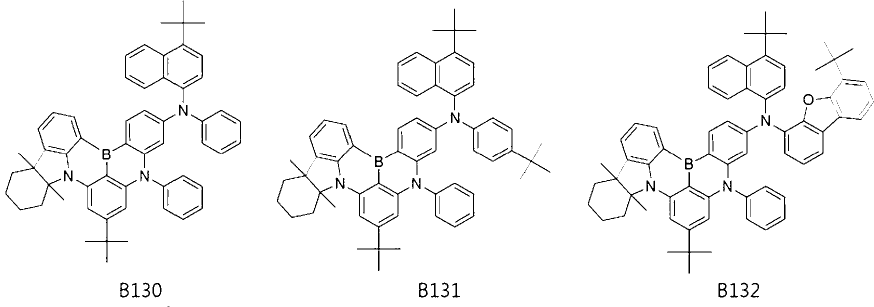

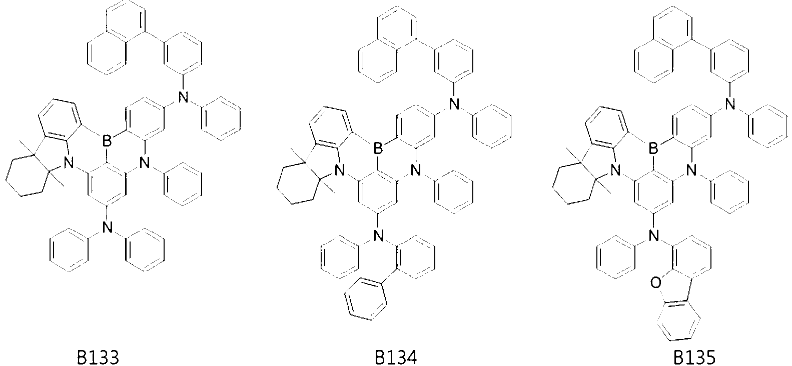

## Revendications

1. Dispositif électroluminescent organique comprenant :
une première électrode ;
une deuxième électrode faisant face à la première électrode ; et
une couche d'émission de lumière interposée entre la première électrode et la deuxième électrode,
ladite couche d'émission de lumière comprenant un composé représenté par la [formule I] suivante, et
ladite couche d'émission de lumière comprenant en outre un composé représenté par la [formule B-1] suivante 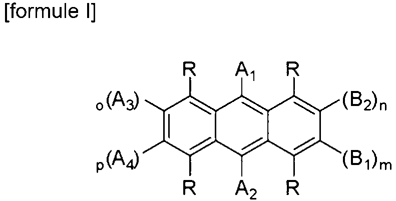
A₁ à A₄, B₁ et B₂ étant identiques ou différents l'un de l'autre et étant chacun indépendamment dibenzofurane substitué ou non substitué, dibenzothiophène substitué ou non substitué, ou hydrocarbure aromatique en C6-C30 substitué ou non substitué;
m, n, o, et p étant chacun indépendamment un entier de 0 à 1, sous réserve que m+n+o+p soit un entier de 1 à 3; et
les R's étant identiques ou différents l'un de l'autre et étant chacun indépendamment choisi parmi hydrogène, deutérium, un groupe alkyle en C1-C30 substitué ou non substitué, un groupe cycloalkyle en C3-C30 substitué ou non substitué, un groupe alkoxy en C1-C30 substitué ou non substitué, un groupe aryloxy en C6-C30 substitué ou non substitué, un groupe alkylthioxy en C1-C30 substitué ou non substitué, un groupe arylthioxy en C5-C30 substitué ou non substitué, un groupe alkylsilyle en C1-C30 substitué ou non substitué, et un groupe arylsilyle en C6-C30 substitué ou non substitué,
le composé de [formule I] comprenant au moins un de dibenzofurane ou dibenzothiophène 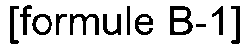 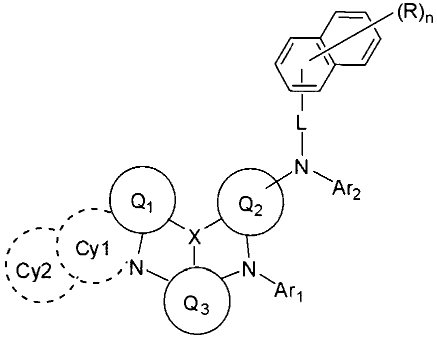
Q₁ à Q₃ étant identiques ou différents l'un de l'autre et étant chacun indépendamment un cycle hydrocarboné aromatique en C6-C50 substitué ou non substitué, ou un cycle hétérocyclique aromatique en C2-C50 substitué ou non substitué ;
X étant choisi parmi B, P et P=O,
Cy1 étant relié à l'atome d'azote (N) et un atome de carbone aromatique dans le cycle Q1 pour former un cycle condensé et le cycle formé Cy1 est un groupe alkylène en C1-C10 substitué ou non substitué, pourvu que l'atome d'azote (N), l'atome de carbone aromatique dans le cycle Q1 auquel l'atome d'azote (N) est lié, et l'atome de carbone aromatique dans le cycle Q1 auquel le cycle Q1 est lié soient exclus ;
Cy2 étant ajouté à Cy1 pour former un cycle hydrocarboné saturé et le cycle Cy2 formé est un groupe alkylène en C1-C10 substitué ou non substitué, pourvu que les atomes de carbone compris dans Cy1 soient exclus ;
Ar1 et R étant choisis chacun indépendamment parmi hydrogène, deutérium, un groupe alkyle linéaire, ramifié ou cyclique en C1-C30 substitué ou non substitué, un groupe alkyle halogéné linéaire, ramifié ou cyclique en C1-C30 substitué ou non substitué, un groupe aryle en C6-C50 substitué ou non substitué, un groupe cycloalkyle en C3-C30 substitué ou non substitué, un groupe hétéroaryle en C2-C50 substitué ou non substitué, un groupe alkoxy en C1-C30 substitué ou non substitué, un groupe aryloxy en C6-C30 substitué ou non substitué, un groupe alkylthioxy en C1-C30 substitué ou non substitué, un groupe arylthioxy en C5-C30 substitué ou non substitué, un groupe alkylamine en C1-C30 substitué ou non substitué, un groupe arylamine en C5-C30 substitué ou non substitué, un groupe alkylsilyle en C1-C30 substitué ou non substitué, un groupe arylsilyle en C6-C30 substitué ou non substitué, un groupe nitro, un groupe cyano, et un groupe halogène; et
les Ar2's étant identiques ou différents l'un de l'autre et étant chacun indépendamment un groupe aryle en C6-C50 substitué ou non substitué, ou un groupe hétéroaryle en C2-C50 substitué ou non substitué,
chacun des Ar1 étant optionnellement lié à Q2 ou Q3 pour former en outre un monocycle ou polycycle alicyclique ou aromatique,
n étant un entier de 1 à 7, les R's étant identiques ou différents l'un de l'autre pourvu que n soit égal ou supérieur à 2, et
L étant une liaison simple ou étant choisi parmi un groupe arylène en C6-C30 substitué ou non substitué ou un groupe hétéroarylène en C1-C30 substitué ou non substitué.

2. Dispositif électroluminescent organique selon la revendication 1, dans lequel le composé de [formule 1] est choisi parmi les composés représentés par les [formule 1] à [formule 123] suivantes :

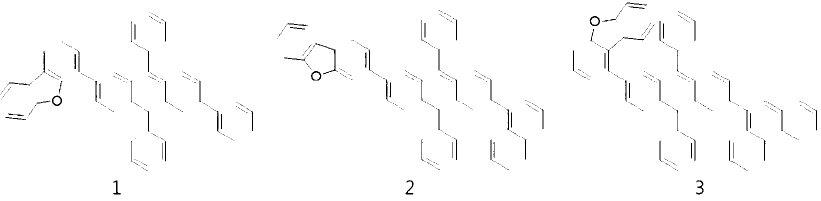

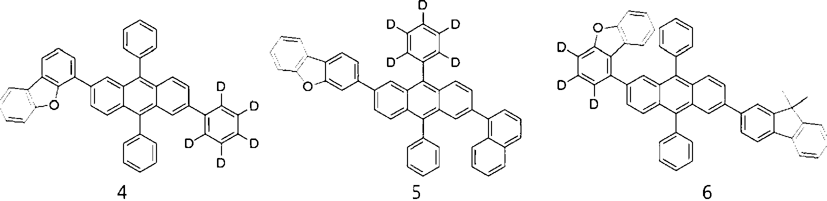

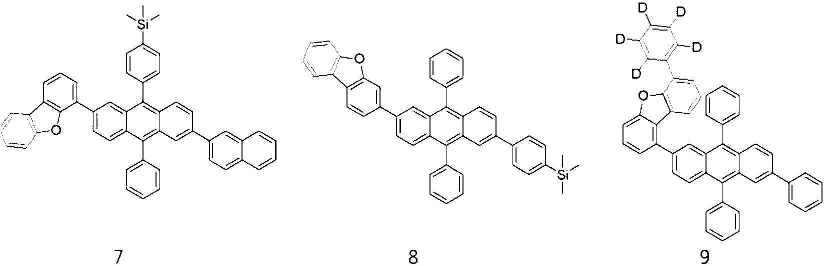

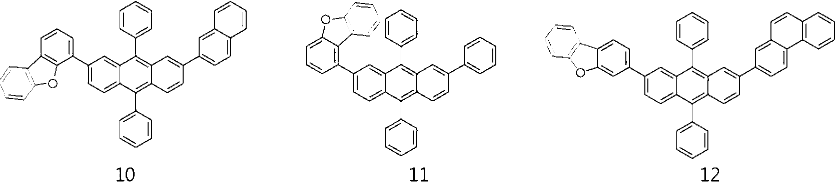

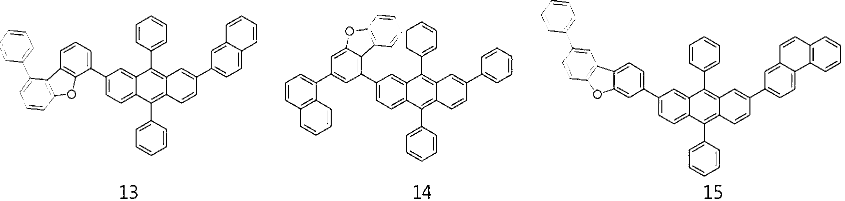

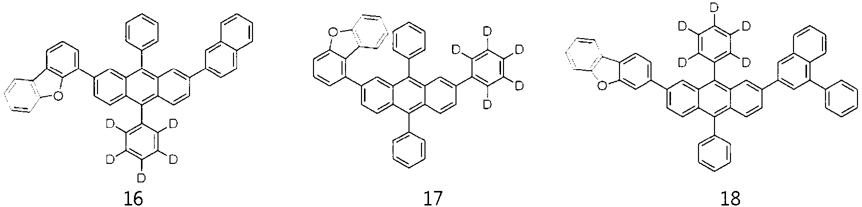

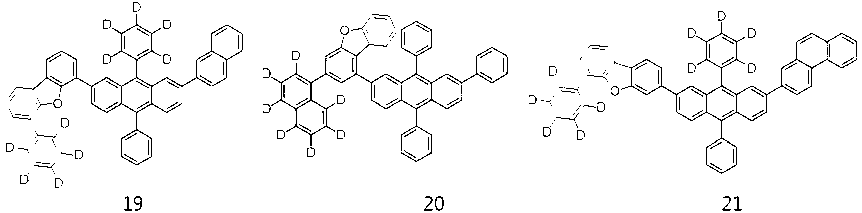

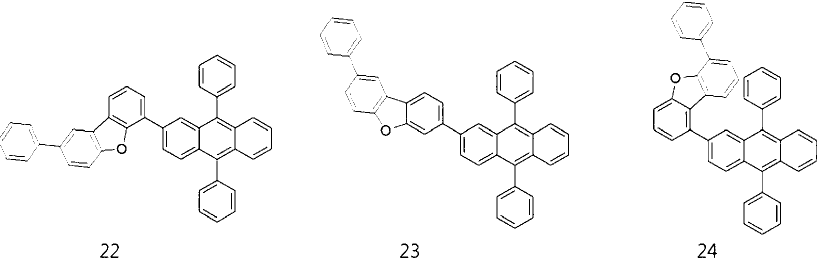

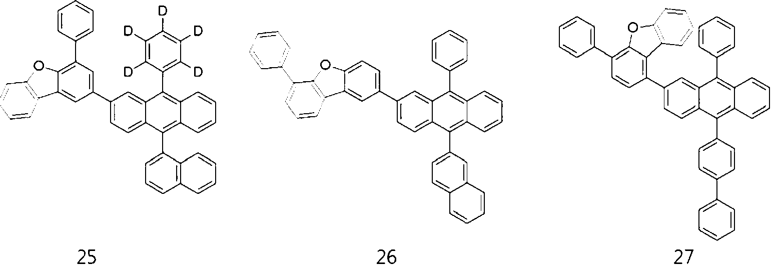

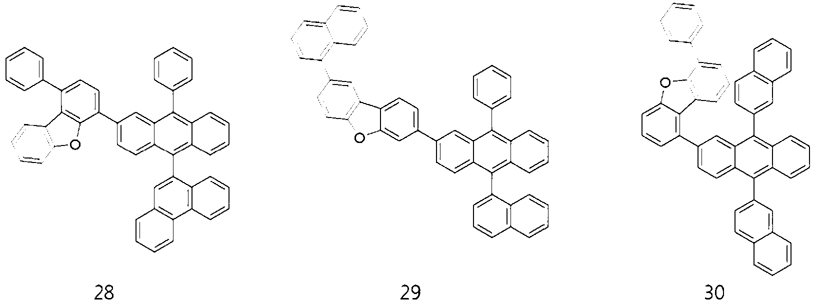

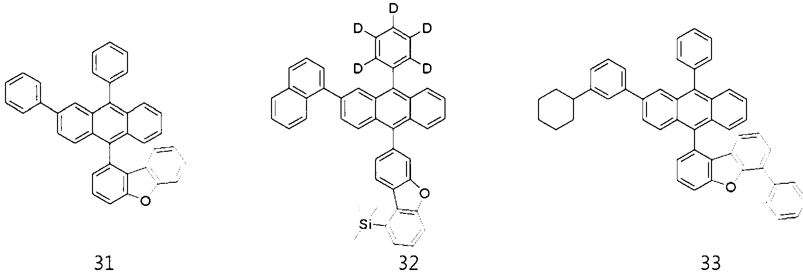

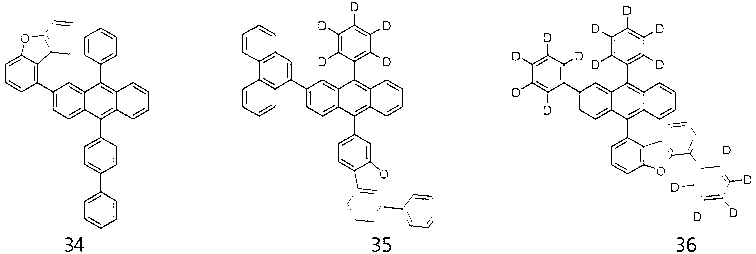

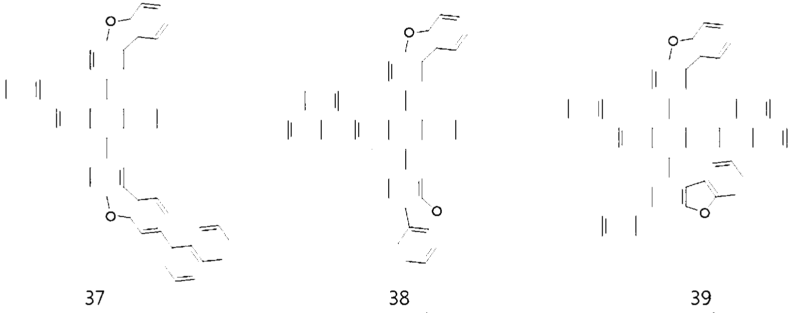

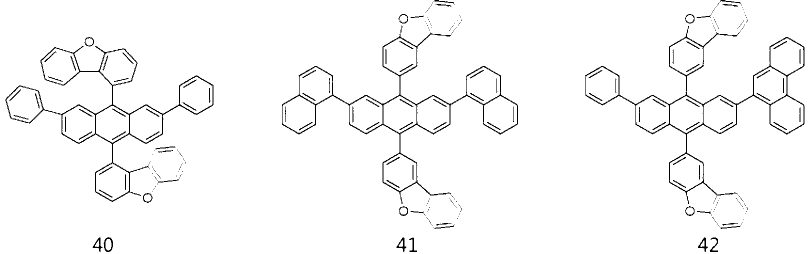

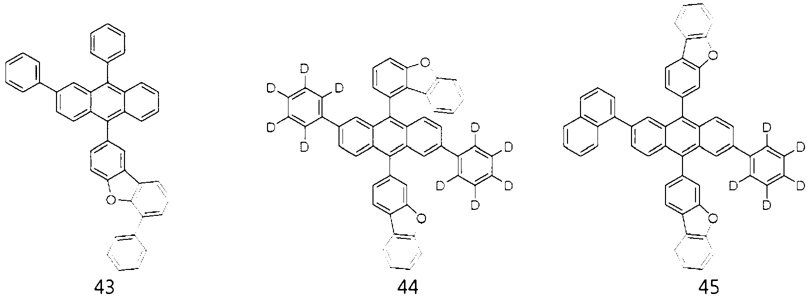

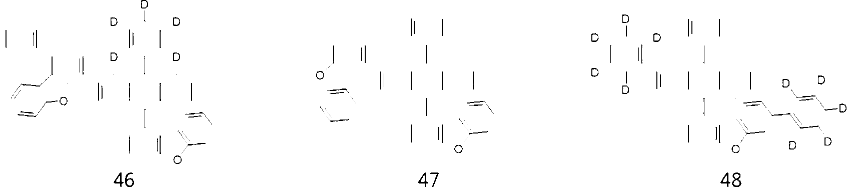

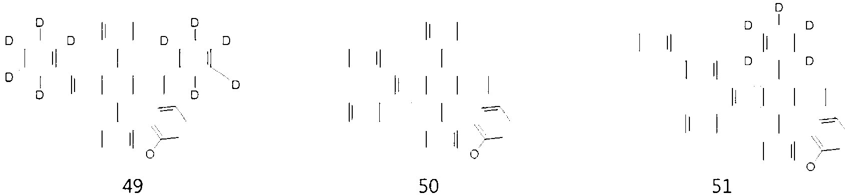

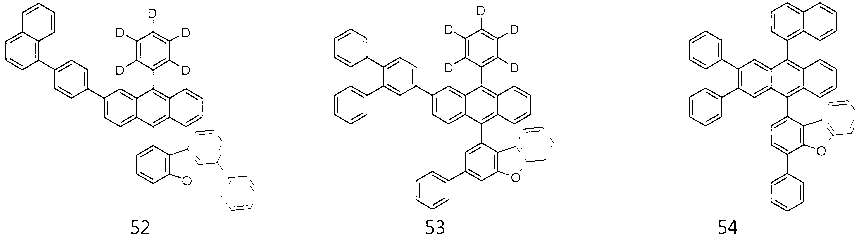

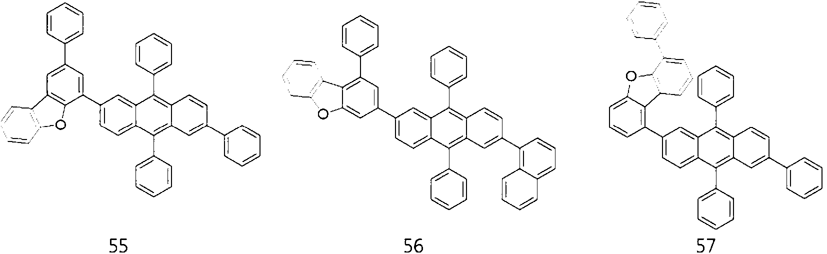

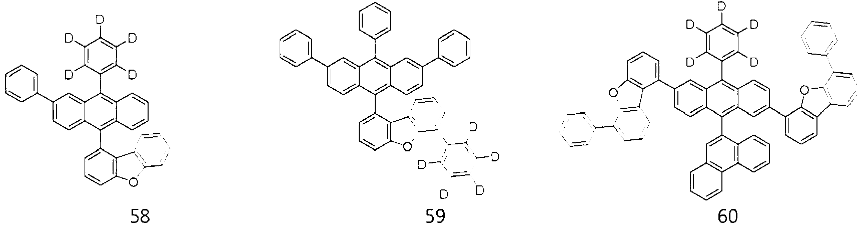

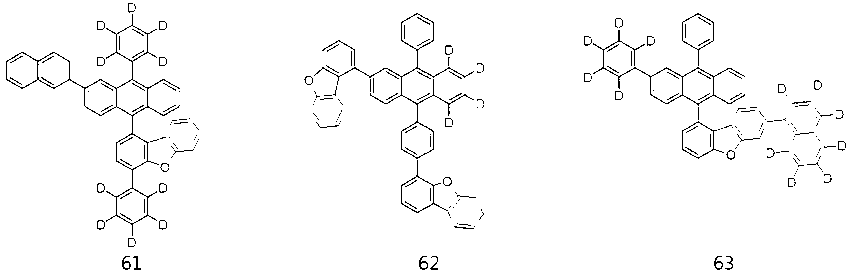

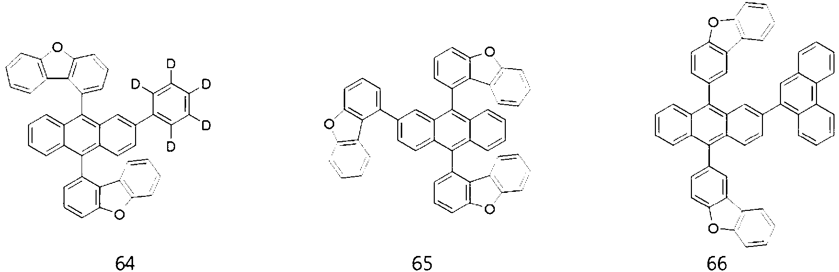

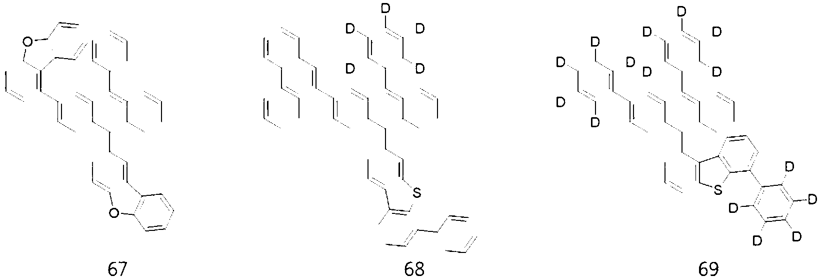

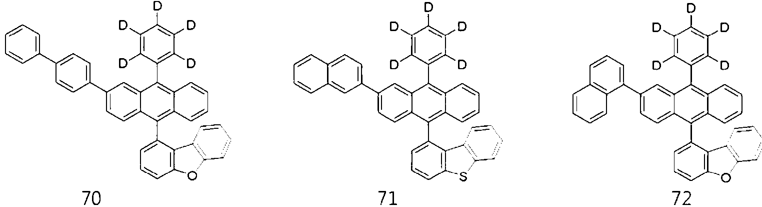

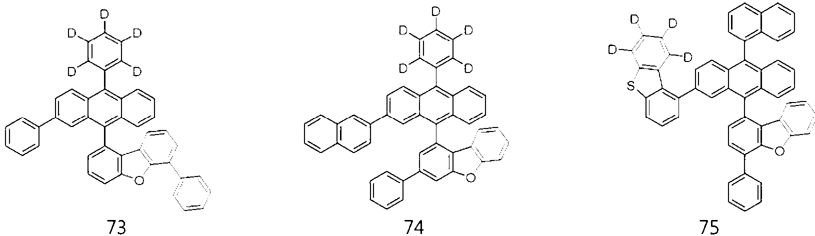

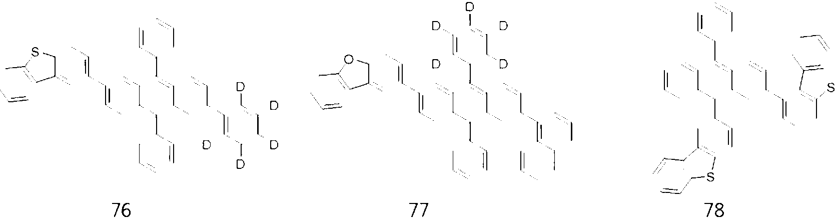

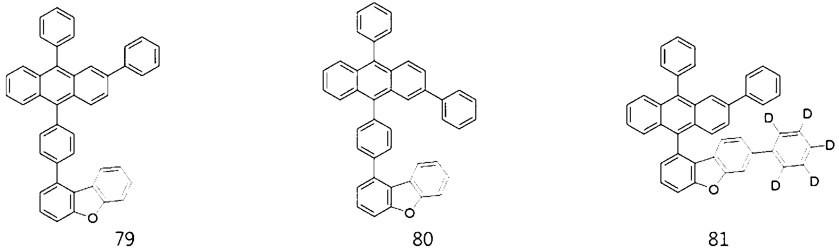

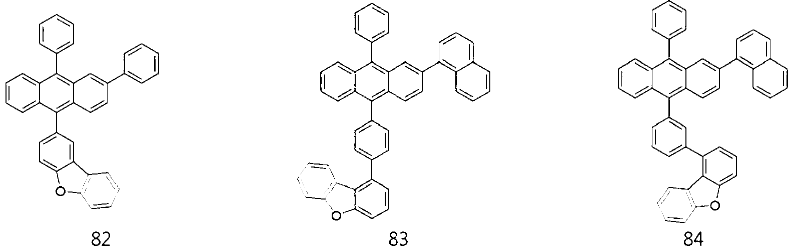

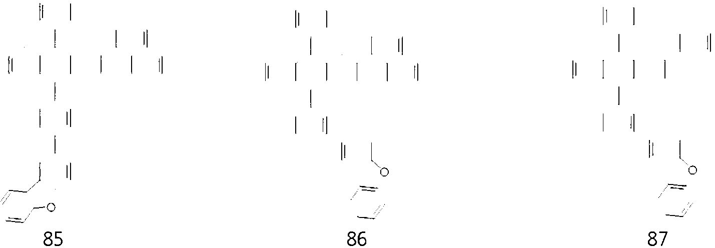

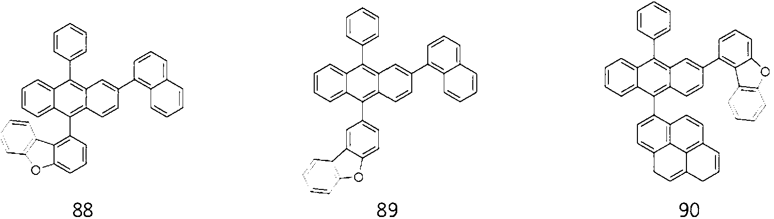

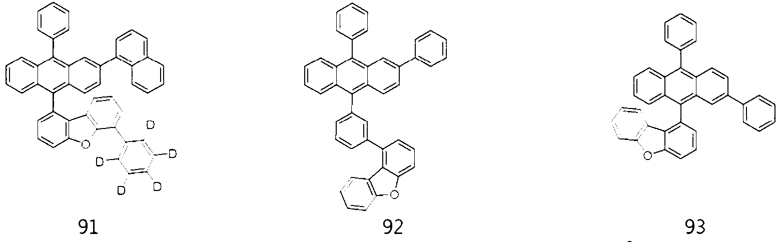

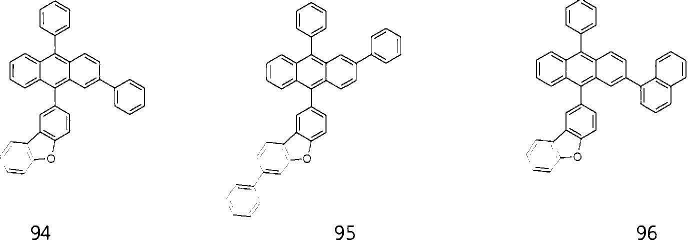

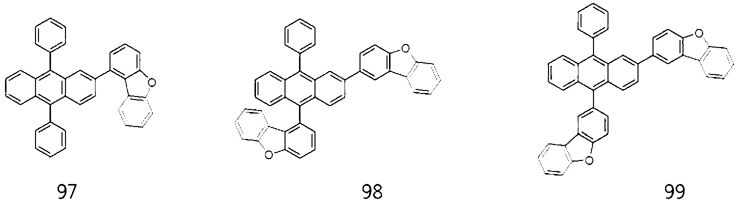

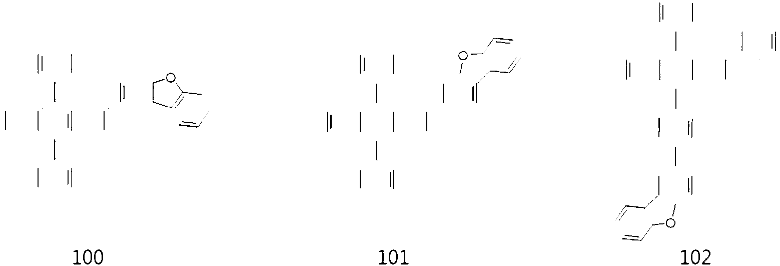

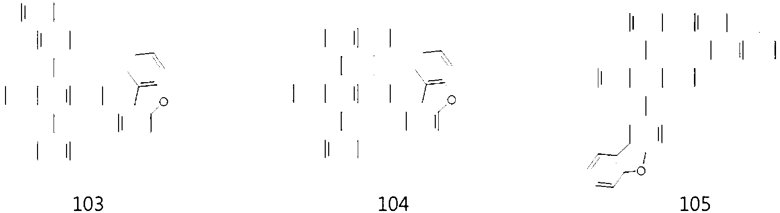

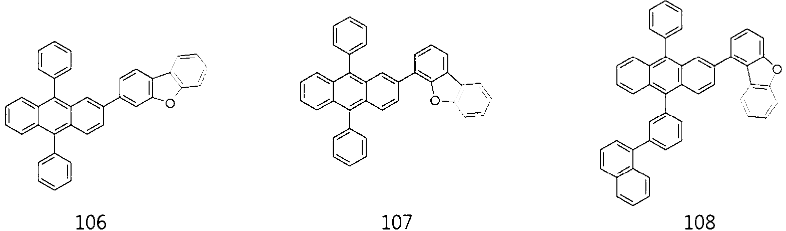

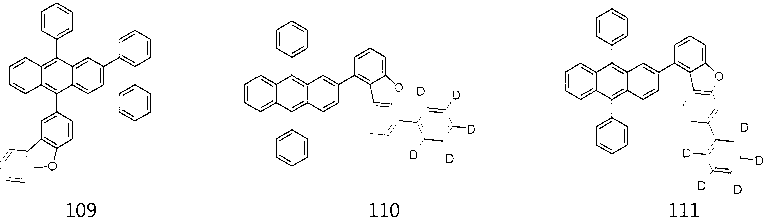

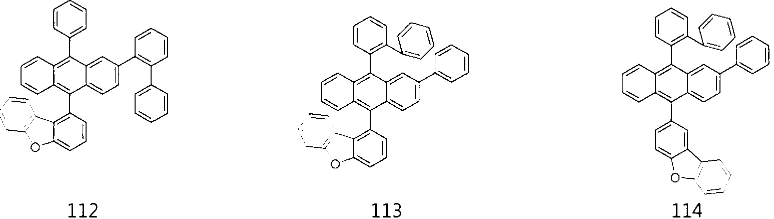

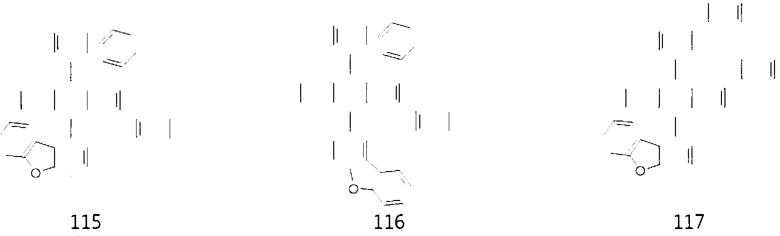

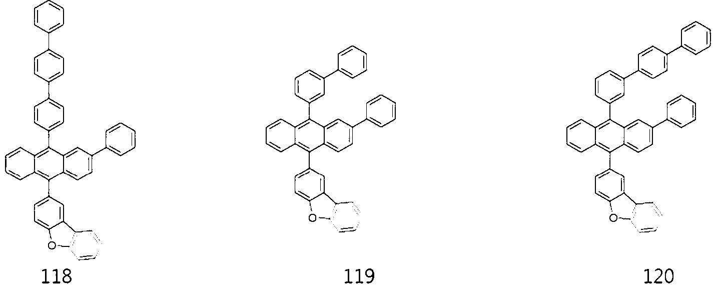

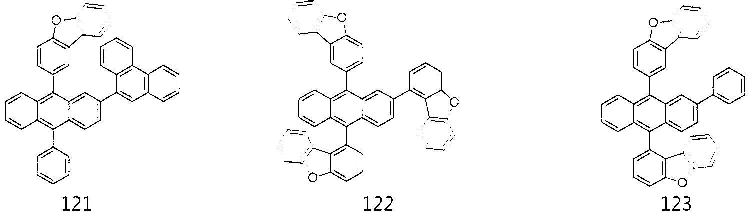

3. Dispositif électroluminescent organique selon la revendication 1, dans lequel le composé de [formule B-1] est choisi parmi les composés représentés par les [formule B91] à [formule B105], et les [formule B112] à [formule B135] suivantes :
